# EUROPEAN PATENT APPLICATION

(11) **EP 4 527 387 A1**
(43) Date of publication of application: **26.03.2025**
(21) Application number: 23806804.3
(22) Date of filing: 11.05.2023
(51) Int. Cl.: A61K 31/472, A61K 31/5377, A61K 45/06, A61K 9/08, A61P 27/02

(54) **COMPOUND PHARMACEUTICAL COMPOSITION FOR TREATING GLAUCOMA AND USE THEREOF**

(30) Priority: 18.05.2022 CN 202210550653
(71) Applicant: Shenyang Pharmaceutical University, Shenyang, Liaoning 110016 (CN)
(72) Inventor: JIANG, Qikun, Shenyang, Liaoning 110016 (CN); WANG, Tao, Shenyang, Liaoning 110016 (CN); CHI, Meiling, Shenyang, Liaoning 110016 (CN); ZHAO, Chen, Shenyang, Liaoning 110016 (CN)
(74) Representative: Sun, Yanan
(86) International application number: PCT/CN2023/093497
(87) International publication number: WO 2023/221852

(57) **Abstract**

The present invention provides ophthalmic dual and triple compound medicine compositions for treating glaucoma and ocular hypertension. Through a salt modification technology, stability of the medicine compositions is improved remarkably. The dual compound medicine composition is prepared from netarsudil free alkali or pharmaceutically acceptable salts of the same and a β-adrenergic receptor blocker. The triple compound medicine composition is prepared from the netarsudil free alkali or the pharmaceutically acceptable salts of the same, the β-adrenergic receptor blocker and a prostaglandin analog. The dual and triple compound medicine compositions have excellent stability when pH is in a range from 4.5 to 5.4, an effective dose of the medicine composition of the present invention is applied to eyes of a patient in need once a day at bedtime or close to bedtime, an intra-ocular pressure can be reduced efficiently and quickly, the intra-ocular pressure remains in a physiological range for a longer time, side effects are minor, and patient compliance is high.

## Description

### TECHNICAL FIELD

The present invention belongs to the field of medicine and particularly relates to ophthalmic dual and triple compound medicine compositions and use of the same in preparation of medicine for preventing or treating glaucoma or medicine for reducing an intra-ocular pressure.

### BACKGROUND

Glaucoma is a common ophthalmic disease, which is brought on due to primary risk factors of pathological increasing of intra-ocular pressure and insufficient blood supply to an optic nerve. Increasing of intra-ocular pressure causes pressure on the optic nerve, which makes an optic nerve fiber layer thinner, damages optic nerve cells and then leads to diminution of vision, a visual field defect, optic neuratrophy, etc., and a patient with severe glaucoma may even suffer from eye blindness, so glaucoma has become a leading killer for human blindness.

At present, there are several types of common unilateral preparations as follows: (1) an Rho kinase inhibitor: it is medicine for reducing an intra-ocular pressure which directly acts on a trabecular meshwork and changes cell morphology, cell movement, cytoplasmic division, smooth muscle contraction, etc. of the trabecular meshwork mainly by affecting cytoskeleton, so as to increase aqueous humor outflow and reduce the intra-ocular pressure; and meanwhile, the Rho kinase inhibitor plays a role in improving retina vascular perfusion, facilitating optic nerve regeneration, protecting the optic nerve, reducing filtering bleb scarring, etc. A drug commonly used in clinic is netarsudil mesylate. (2) A β-adrenergic receptor blocker: it may reduce generation of aqueous humor and facilitate aqueous humor drainage and discharge by blocking the β receptor, has a powerful and lasting effect on reducing the intra-ocular pressure and effectively controls the intra-ocular pressure. Drugs commonly used in clinic are timolol maleate, carteolol hydrochloride, betaxolol hydrochloride, metipranolol hydrochloride, etc. (3) A prostaglandin analog: it reduces the intra-ocular pressure by relaxing ciliary muscle, widening intermuscular space and increasing outflow and drainage of a uveoscleral pathway of the aqueous humor, and has a high effect on reducing the intra-ocular pressure at night, especially for a patient intolerant to or having a poor effect with other medicine for reducing the intra-ocular pressure, and drugs commonly used in clinic are latanoprost, bimatoprost, travoprost, tafluprost, etc.

Glaucoma is a chronic eye disease, and the patient needs long-term medication. However, the unilateral preparations cannot usually effectively control the intra-ocular pressure, and two types of drugs need to be combined for use. For use of two types of unilateral preparations in combination or a dual compound preparation currently on the market (such as Combigan^{®}, Cosopt^{®}, etc.), due to increase of administration frequency, an exposed quantity of a preservative may also increase remarkably, which leads to poor safety, many side effects, poor patient compliance and other problems. Based on this, in the present invention, the β-adrenergic receptor blocker (timolol free alkali or salts of the same, carteolol free alkali or salts of the same, betaxolol free alkali or salts of the same, or metipranolol free alkali or salts of the same) and netarsudil representing the Rho kinase inhibitor are combined into a fixed dual compound medicine composition, which has the following strengths: (1) the intra-ocular pressure may be reduced through different action mechanisms, and the therapeutic effect is significant and exact; and (2) drug administration is performed once a day, the administration frequency is reduced, the exposed quantity of the preservative is reduced, occurrence of the side effects on the eyes is reduced, and patient compliance is improved remarkably. Thus, the ophthalmic medicine composition of the present invention provides possibility of reducing the intra-ocular pressure safely and effectively in clinic and treating glaucoma.

It may be known, in combination with prescribing information and related literature reports of commercially available netarsudil eye drops (Rhopress^{®}), that netarsudil dimesylate is unstable and prone to generating precipitate in a boric acid-sodium borate buffer solution with pH being higher than 5.4. Meanwhile, netarsudil dimesylate with the commercially available dose is in a solution state and good in stability in the boric acid-sodium borate buffer solution with pH lower than 5.4. Thus, in combination with eyes' tolerance to pH, in vitro stability of netarsudil dimesylate and a pH value range regulated by the netarsudil eye drops (Rhopress^{®}), the ophthalmic medicine composition of the present invention is preliminarily determined as a boric acid-sodium borate buffer solution with a pH value in a range from 4.5 to 5.4. It is discovered unexpectedly by the applicant in a preliminary study that netarsudil dimesylate with the commercially available dose and the β-adrenergic receptor blocker (timolol maleate, carteolol hydrochloride, betaxolol hydrochloride and metipranolol hydrochloride) are unstable in the boric acid-sodium borate buffer solution with a pH value in a range from 4.5 to 5.4, namely, netarsudil dimesylate may be precipitated, and white precipitate appears in the solution. To verify the reason of causing precipitation of netarsudil dimesylate, maleic acid and methanesulfonic acid, hydrochloric acid and methanesulfonic acid, maleic acid and netarsudil dimesylate, hydrochloric acid and netarsudil dimesylate, timolol maleate and methanesulfonic acid, carteolol hydrochloride and methanesulfonic acid, betaxolol hydrochloride and methanesulfonic acid, metipranolol hydrochloride and methanesulfonic acid, timolol maleate and netarsudil dimesylate, carteolol hydrochloride and netarsudil dimesylate, betaxolol hydrochloride and netarsudil dimesylate, metipranolol hydrochloride and netarsudil dimesylate with the commercially available doses are respectively mixed in the boric acid-sodium borate buffer solution with a pH value in a range from 4.5 to 5.4, and a result shows that netarsudil dimesylate is incompatible with the maleic acid, the hydrochloric acid, timolol maleate, carteolol hydrochloride, betaxolol hydrochloride and metipranolol hydrochloride, and white precipitate may be generated. Thus, it is indicated that netarsudil dimesylate is incompatible with the maleic acid and the hydrochloric acid.

Based on this, in the present invention, compatibility of the β-adrenergic receptor blocker (timolol maleate, carteolol hydrochloride, betaxolol hydrochloride, or metipranolol hydrochloride) with netarsudil dimesylate is improved through a salt engineering strategy, namely, by regulating salt-forming types of the β-adrenergic receptor blocker (timolol, carteolol, betaxolol or metipranolol) and the netarsudil, the compatibility of the β-adrenergic receptor blocker with the netarsudil in a water-for-injection solution with a pH value in a range from 4.5 to 5.4 is good. Meanwhile, the present invention constructs a triple compound preparation based on the netarsudil and the β-adrenergic receptor blocker, such as the netarsudil, the β-adrenergic receptor blocker (timolol, carteolol, betaxolol or metipranolol) and the prostaglandin analog. The triple compound preparation has strengths of a high effect on reducing the intra-ocular pressure, allowing drug administration once a day, etc., and it is expected to solve problems in only one existing triple compound preparation (Krytantek Ofteno^{®}, dorzolamide 2%/timolol 0.5%/brimonidine 0.2%) on the global market that there is a serious adverse event, the effect on reducing the intra-ocular pressure is limited, etc.

### SUMMARY

For overcoming defects of a poor therapeutic effect, many side effects, poor patient compliance and the like in an existing unilateral preparation, dual compound preparation and triple compound preparation for treating glaucoma, the present invention provides a dual compound medicine composition including both β-adrenergic receptor blocker and netarsudil; and a triple compound medicine composition including the β-adrenergic receptor blocker, the netarsudil and a prostaglandin analog, the problem that the β-adrenergic receptor blocker (timolol free alkali or salts of the same, carteolol free alkali or salts of the same, betaxolol free alkali or salfts of the same, or metipranolol free alkali or salts of the same) is incompatible with some types of salts of the netarsudil is solved successfully through a salt engineering strategy, and the compound medicine composition of the present invention can reduce an intra-ocular pressure more remarkably compared with a commercially available compound ophthalmic preparation.

That is, the present invention involves the the following content.
(1) In an aspect, the present invention provides an ophthalmic dual compound composition solution, including netarsudil free alkali or pharmaceutically acceptable salts of the same and a β-adrenergic receptor blocker (timolol free alkali or salts of the same, carteolol free alkali or salts of the same, betaxolol free alkali or salts of the same, or metipranolol free alkali or salts of the same) as active medicine components as well as a certain quantity of buffering agent, tonicity agent, preservative and pH regulator; and an ophthalmic triple compound composition solution, including netarsudil free alkali or pharmaceutically acceptable salts of the same, the β-adrenergic receptor blocker (timolol free alkali or salts of the same, carteolol free alkali or salts of the same, betaxolol free alkali or salts of the same, or metipranolol free alkali or salts of the same) and a prostaglandin analog as active medicine components as well as a certain quantity of buffering agent, tonicity agent, preservative and pH regulator.
(2) According to the above composition solution described in (1), the netarsudil is in a form of free alkali or any pharmaceutically acceptable salt (except mesylate) of the same in a case that the β-adrenergic receptor blocker is selected from timolol maleate, or carteolol hydrochloride, or betaxolol hydrochloride, or metipranolol hydrochloride, preferably, netarsudil maleate, netarsudil sulfate, netarsudil dihydrobromide, netarsudil dihydrochloride, netarsudil diformate, netarsudil dinitrate, netarsudil diacetate, netarsudil dibenzoate, netarsudil diphenylacetate, netarsudil succinate, netarsudil oxalate, netarsudil dihydriodate, or netarsudil dipropionate.
(3) According to the above composition solution described in (1), the timolol is in a form of free alkali or any pharmaceutically acceptable salt (except maleate) of the same in a case that the netarsudil is dimesylate, preferably, timolol mesylate, timolol sulfate, timolol hydrobromide, timolol phosphate, timolol nitrate, timolol citrate, timolol tartrate, timolol salicylate, timolol malate, timolol lactate, timolol phenylacetate, timolol succinate, timolol hydriodate, timolol formate, timolol acetate, timolol benzoate, timolol esilate, timolol oxalate or timolol propionate; the carteolol is in a form of free alkali or any pharmaceutically acceptable salt (except hydrochloride) of the same, preferably, carteolol mesylate, carteolol hydrobromide, carteolol sulfate, carteolol esilate, carteolol nitrate, carteolol citrate, carteolol tartrate, carteolol salicylate, carteolol malate, carteolol lactate, carteolol phenylacetate, carteolol succinate, carteolol hydriodate, carteolol formate, carteolol acetate, carteolol benzoate, carteolol esilate, carteolol oxalate or carteolol propionate; the betaxolol is in a form of free alkali or any pharmaceutically acceptable salt (except hydrochloride) of the same, preferably, betaxolol mesylate, betaxolol hydrobromide, betaxolol sulfate, betaxolol esilate, betaxolol nitrate, betaxolol citrate, betaxolol tartrate, betaxolol salicylate, betaxolol malate, betaxolol lactate, betaxolol phenylacetate, betaxolol succinate, betaxolol hydriodate, betaxolol formate, betaxolol acetate, betaxolol benzoate, betaxolol esilate, betaxolol oxalate or betaxolol propionate; and the metipranolol is in a form of free alkali or any pharmaceutically acceptable salt (except hydrochloride) of the same, preferably, metipranolol mesylate, metipranolol hydrobromide, metipranolol sulfate, metipranolol esilate, metipranolol nitrate, metipranolol citrate, metipranolol tartrate, metipranolol salicylate, metipranolol malate, metipranolol lactate, metipranolol phenylacetate, metipranolol succinate, metipranolol hydriodate, metipranolol formate, metipranolol acetate, metipranolol benzoate, metipranolol esilate, metipranolol oxalate or metipranolol propionate.
(4) According to the above composition solution described in (1), the prostaglandin analog includes but is not limited to latanoprost, bimatoprost, travoprost, tafluprost, AR-102, cloprostenol isopropyl ester, 13,14-dihydrocloprostenol isopropyl ester, latanoprostene bunod, unoprostone, PGF1α isopropyl ester, PGF2α isopropyl ester, PGF3α isopropyl ester and fluprostenol isopropyl ester.
(5) According to the above composition solution described in (1), the tonicity agent includes but is not limited to glycerol, sorbitol, mannitol, propylene glycol, erythritol, arabitol, xylitol, ribitol, galactitol, polyethylene glycol, lactitol and other sugar alcohol, sodium chloride, potassium chloride and calcium chloride, or any combination of the above.
(6) According to the above composition solution described in (1), the buffering agent includes but is not limited to boric acid or salts of the same, sodium dihydrogen phosphate dihydrate, sodium dihydrogen phosphate monohydrate, sodium phosphate anhydrate, citric acid or salts of the same, gluconic acid or salts of the same, acetic acid or salts of the same, phosphoric acid or salts of the same, various amino acids such as glutamic acid and ε-aminocaproic acid, a tris(hydroxymethyl) aminomethane buffer, or any combination of the above.
(7) According to the above composition solution described in (1), the preservative includes but is not limited to benzalkonium chloride, thimerosal, chlorbutanol, methyl p-hydroxybenzoate, propyl p-hydroxybenzoate, phenethyl alcohol, edetate disodium, boric acid, sorbic acid or any combination of the above.
(8) According to the above composition solution described in (1), examples of the pH regulator include but are not limited to sodium hydroxide, potassium hydroxide, sodium carbonate, sodium hydrogen carbonate, hydrochloric acid, citric acid or salts of the same, phosphoric acid or salts of the same, acetic acid or salts of the same, and tartaric acid or salts of the same.
(9) According to the above composition solution described in (1) to (8), the composition includes 0.02%w/v to 4.0 %w/v β-adrenergic receptor blocker (timolol free alkali or salts of the same, carteolol free alkali or salts of the same, betaxolol free alkali or salts of the same, or metipranolol free alkali or salts of the same).
(10) According to the above composition solution described in (1) to (8), the composition includes 0.005 %w/v to 0.1 %w/v netarsudil free alkali or salts of the same.
(11) According to the above composition solution described in (1) to (8), the composition includes 0.0005 %w/v to 0.05 %w/v prostaglandin analog.
(12) According to the above composition solution described in (1) to (8), the composition includes 1.0%w/v to 10.0%w/v mannitol.
(13) According to the above composition solution described in (1) to (8), the composition includes 0.05%w/v boric acid.
(14) According to the above composition solution described in (1) to (8), the composition includes 0.001%w/v to 0.02 %w/v benzalkonium chloride.
(15) The above composition solution described in (1) to (14) has an osmotic pressure in a range from 280 mOsmol/kg to 320 mOsmol/kg.
(16) The above composition solution described in (1) to (15) has pH in a range from 4.5 to 5.4.
(17) According to the above composition solution described in (1) to (16), compared with zero day, a content of each main medicine component has no significant change compared with zero day after being stored at 5°Cfor 24 months, which remains consistent with a storage condition of commercially available eye drops before opening.
(18) According to the above composition solution described in (1) to (16), compared with zero day, a content of each main medicine component has no significant change compared with zero day after being stored at 25°Cfor 6 weeks, which meets a requirement for a storage condition of commercially available eye drops after opening.
(19) According to the above composition solution described in (1) to (16), compared with zero day, a content of each main medicine component has no significant change compared with zero day after being stored at 40°Cfor 14 days, which meets a requirement for departing from a storage condition of commercially available eye drops for a short time.
(20) In another aspect, the present invention provides use of the composition solution in the first aspect in preparation for medicine for preventing or treating an eye disease.
(21) According to the use described in (20), the eye disease is glaucoma or symptoms related to the same.
(22) In a third aspect, the present invention provides use of the composition solution in the first aspect in preparation of medicine for reducing an intra-ocular pressure.

Compared with the prior art, the present invention has the following beneficial effects.
(1) Through a salt engineering strategy, a problem of incompatibility of the β-adrenergic receptor blocker (timolol free alkali or salts of the same, carteolol free alkali or salts of the same, betaxolol free alkali or salts of the same, or metipranolol free alkali or salts of the same) with some types of salts of netarsudil is solved, so as to obtain the solution having the excellent stability and including the β-adrenergic receptor blocker (timolol free alkali or salts of the same, carteolol free alkali or salts of the same, betaxolol free alkali or salts of the same, or metipranolol free alkali or salts of the same) and the netarsudil.
(2) In another aspect, the medicine composition solution provided by the present invention is useful for preparing medicine for preventing or treating the eye disease (especially, glaucoma or symptoms related to the same) and medicine for reducing the intra-ocular pressure, and the medicine composition of the present invention can reduce the intra-ocular pressure more remarkably compared with commercially available compound ophthalmic preparations.

### BRIEF DESCRIPTION OF THE DRAWINGS

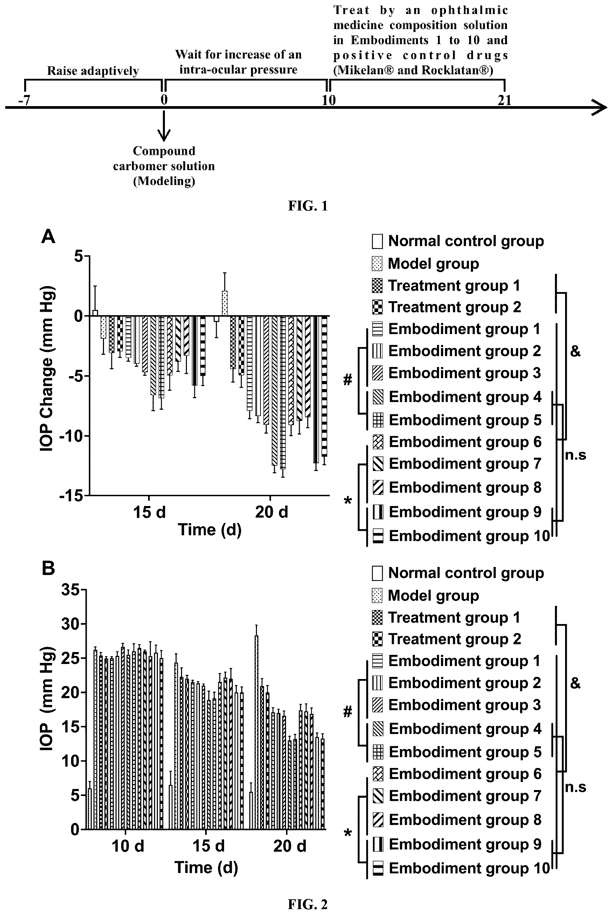

FIG. 1 represents a dosage regimen of an ophthalmic medicine composition solution in Embodiments 1 to 10, a positive control drug treatment group 1 (Rhopress^{®}, netarsudil mesylate eye drops), and a positive control drug treatment group 2 (Mikelan^{®}, carteolol hydrochloride eye drops).
FIG. 2 represents a column diagram made according to data of an intra-ocular pressure variation value (2A) and an intra-ocular pressure actual measured value (2B) of each group of rabbits measured on the fifteenth day and the twentieth day when an ophthalmic medicine composition solution of Embodiments 1 to 10 and positive control drugs (Rhopress^{®} and Mikelan^{®}) are administered once a day for 10 days on end starting with the tenth day. On the twentieth day: &: P < 0.05, a treatment group 1 and a treatment group 2 compared with Embodiment groups 1 to 10; #: P < 0.05, Embodiment groups 1, 2 and 3 compared with Embodiment groups 4 and 5; _{*}: P < 0.05, Embodiment groups 6, 7 and 8 compared with Embodiment groups 9 and 10; and n.s: P > 0.05, Embodiment groups 4 and 5 compared with Embodiment groups 9 and 10.

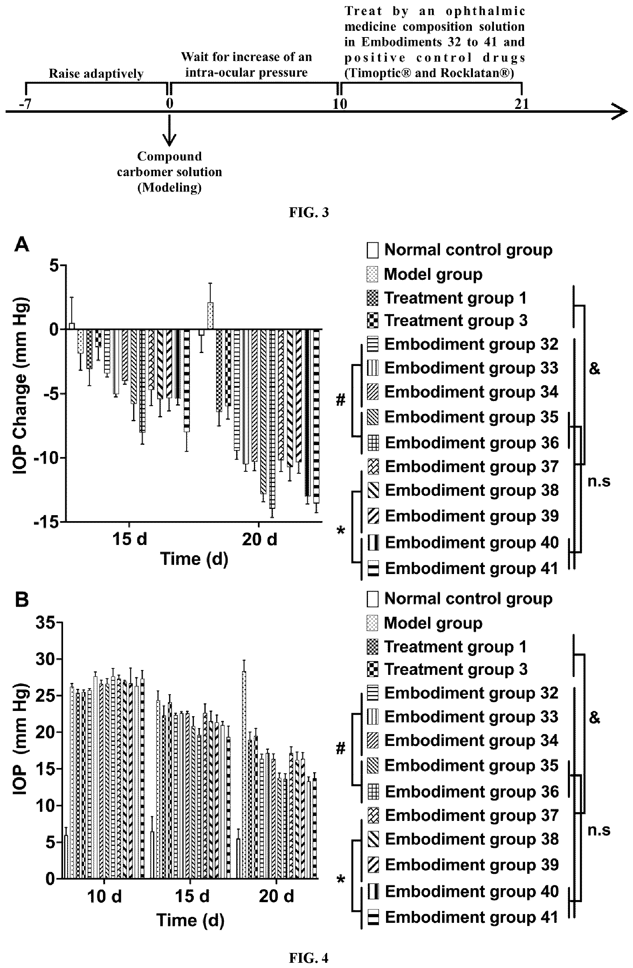

FIG. 3 represents a dosage regimen of an ophthalmic medicine composition solution in Embodiments 12 to 21, a positive control drug treatment group 1 and a positive control drug treatment group 3 (Timoptic^{®}, timolol maleate eye drops).
FIG. 4 represents a column diagram made according to data of an intra-ocular pressure variation value (4A) and an intra-ocular pressure actual measured value (4B) of each group of rabbits measured on the fifteenth day and the twentieth day when an ophthalmic medicine composition solution of Embodiments 12 to 21 and a positive control drug (Timoptic^{®} and Rhopress^{®}) are administered once a day for 10 days on end starting with the tenth day. On the twentieth day: &: P < 0.05, a treatment group 1 and a treatment group 3 compared with Embodiment groups 12 to 21; #: P < 0.05, Embodiment groups 12, 13 and 14 compared with Embodiment groups 15 and 16; _{*}: P < 0.05, Embodiment groups 17, 18 and 19 compared with Embodiment groups 20 and 21; and n.s: P > 0.05, Embodiment groups 15 and 16 compared with Embodiment groups 20 and 21.

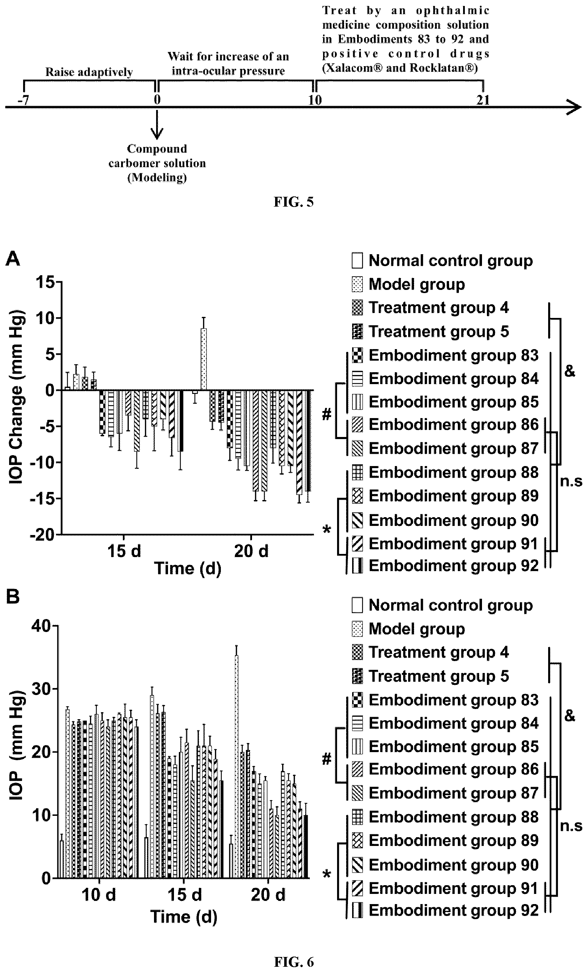

FIG. 5 represents a dosage regimen of an ophthalmic medicine composition solution in Embodiments 83 to 92, a positive control drug treatment group 4 (Xalacom^{®}, latanoprost and timolol eye drops), and a positive control drug treatment group 5 (Rocklatan^{®}, latanoprost and netarsudil eye drops).
FIG. 6 represents a column diagram made according to data of an intra-ocular pressure variation value (6A) and an intra-ocular pressure actual measured value (6B) of each group of rabbits measured on the fifteenth day and the twentieth day when an ophthalmic medicine composition solution of Embodiments 83 to 92 and positive control drugs (Xalacom^{®} and Rocklatan^{®}) are administered once a day for 10 days on end starting with the tenth day. On the twentieth day: &: P < 0.05, a treatment group 4 and a treatment group 5 compared with Embodiment groups 83 to 92; #: P < 0.05, Embodiment groups 83, 84 and 85 compared with Embodiment groups 86 and 87; _{*}: P < 0.05, Embodiment groups 88, 89 and 90 compared with Embodiment groups 91 and 92; and n.s: P > 0.05, Embodiment groups 86 and 87 compared with Embodiment groups 91 and 92.

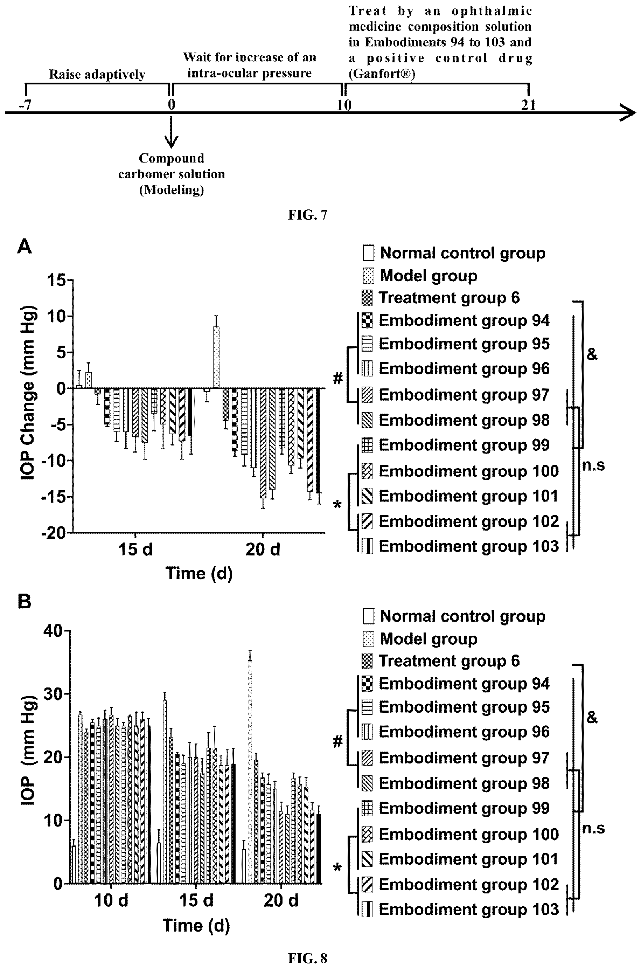

FIG. 7 represents a dosage regimen of an ophthalmic medicine composition solution in Embodiments 94 to 103 and a positive control drug treatment group 6 (Ganfort^{®}, bimatoprost and timolol maleate eye drops).
FIG. 8 represents a column diagram made according to data of an intra-ocular pressure variation value (8A) and an intra-ocular pressure actual measured value (8B) of each group of rabbits measured on the fifteenth day and the twentieth day when an ophthalmic medicine composition solution of Embodiments 94 to 103 and a positive control drug (Ganfort^{®}) are administered once a day for 10 days on end starting with the tenth day. On the twentieth day: &: P < 0.05, a treatment group 6 compared with Embodiment groups 94 to 103; #: P < 0.05, Embodiment groups 94, 95 and 96 compared with Embodiment groups 97 and 98; _{*}: P < 0.05, Embodiment groups 99, 100 and 101 compared with Embodiment groups 102 and 103; and n.s: P > 0.05, Embodiment groups 97 and 98 compared with Embodiment groups 102 and 103.

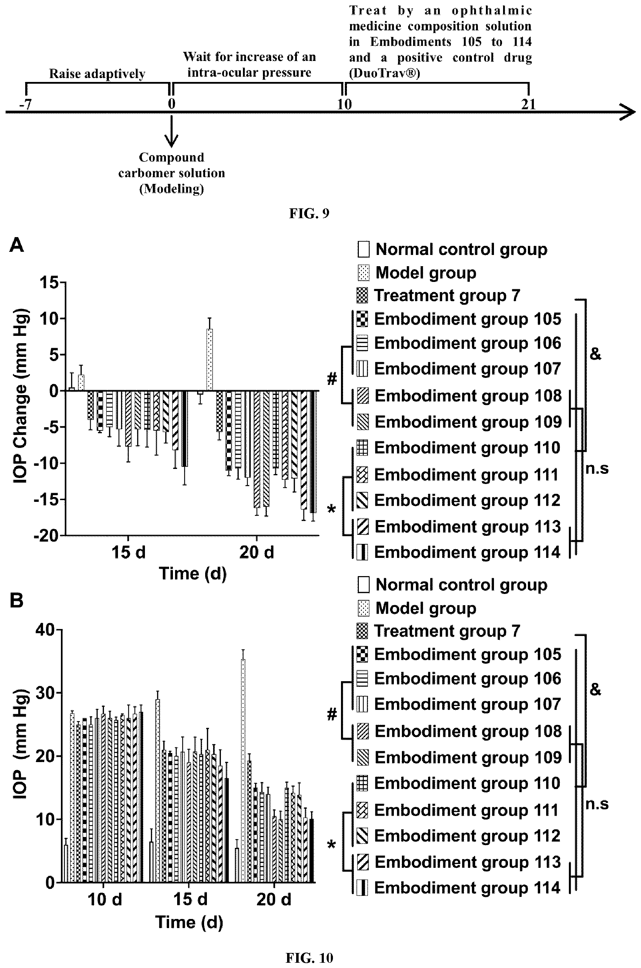

FIG. 9 represents a dosage regimen of an ophthalmic medicine composition solution in Embodiments 105 to 114 and a positive control drug treatment group 7 (DuoTrav^{®}, travoprost and timolol maleate eye drops).
FIG. 10 represents a column diagram made according to data of an intra-ocular pressure variation value (10A) and an intra-ocular pressure actual measured value (10B) of each group of rabbits measured on the fifteenth day and the twentieth day when an ophthalmic medicine composition solution of Embodiments 105 to 114 and a positive control drug (DuoTrav^{®}) are administered once a day for 10 days on end starting with the tenth day. On the twentieth day: &: P < 0.05, a treatment group 7 compared with Embodiment groups 105 to 114; #: P < 0.05, Embodiment groups 105, 106 and 107 compared with Embodiment groups 108 and 109; _{*}: P < 0.05, Embodiment groups 110, 111 and 112 compared with Embodiment groups 113 and 114; and n.s: P > 0.05, Embodiment groups 108 and 109 compared with Embodiment groups 113 and 114.

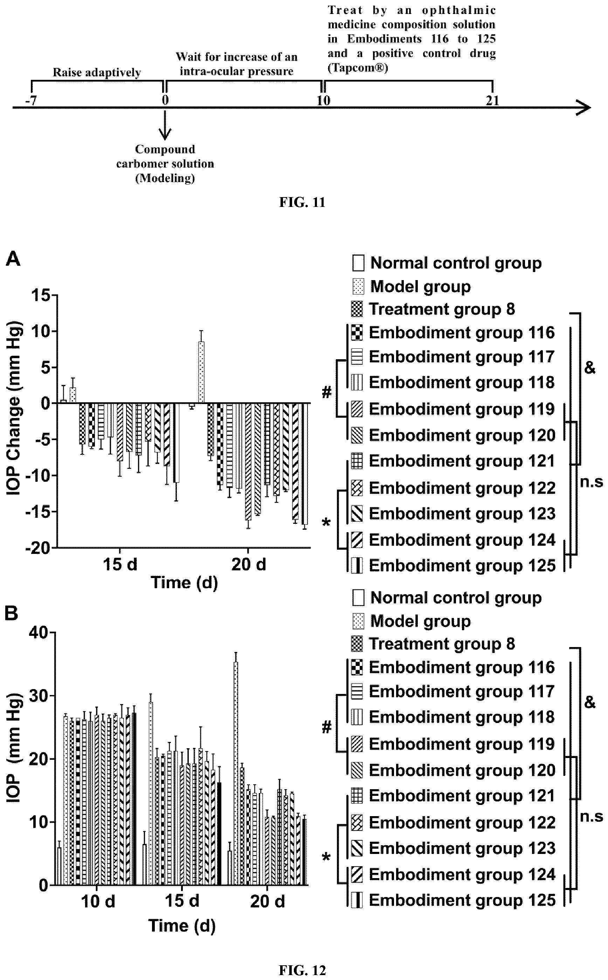

FIG. 11 represents a dosage regimen of an ophthalmic medicine composition solution in Embodiments 116 to 125 and a positive control drug treatment group 8 (Tapcom^{®}, tafluprost timolol maleate eye drops).
FIG. 12 represents a column diagram made according to data of an intra-ocular pressure variation value (12A) and an intra-ocular pressure actual measured value (12B) of each group of rabbits measured on the fifteenth day and the twentieth day when an ophthalmic medicine composition solution of Embodiments 116 to 125 and a positive control drug (Tapcom^{®}) are administered once a day for 10 days on end starting with the tenth day. On the twentieth day: &: P < 0.05, a treatment group 8 compared with Embodiment groups 116 to 125; #: P < 0.05, Embodiment groups 116, 117 and 118 compared with Embodiment groups 119 and 120; _{*}: P < 0.05, Embodiment groups 121, 122 and 123 compared with Embodiment groups 124 and 125; and n.s: P > 0.05, Embodiment groups 119 and 120 compared with Embodiment groups 124 and 125.

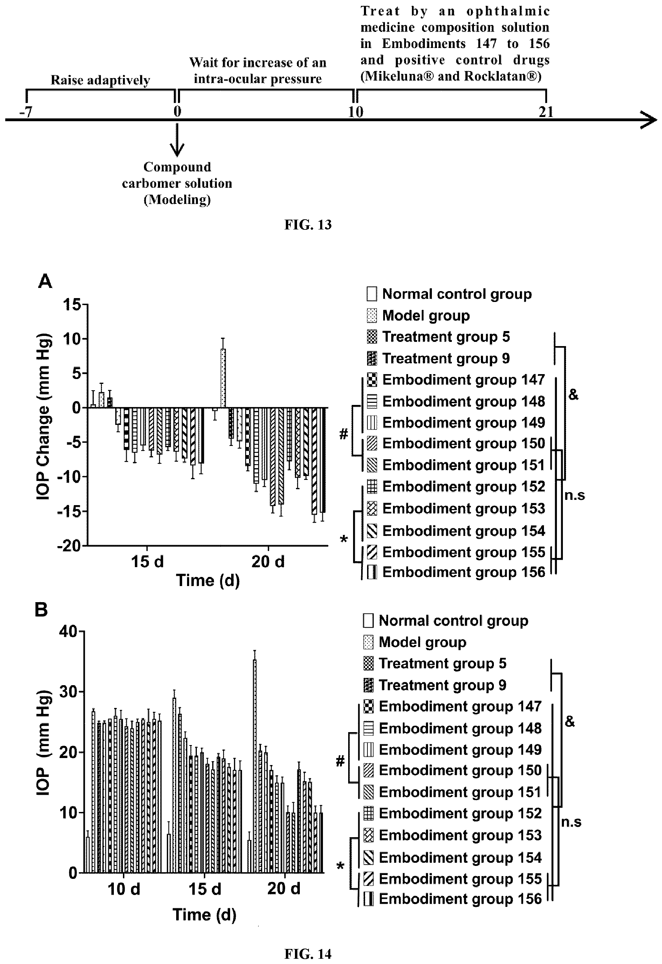

FIG. 13 represents a dosage regimen of an ophthalmic medicine composition solution in Embodiments 147 to 156, a positive control drug treatment group 5, and a positive control drug treatment group 9 (Mikeluna^{®}, carteolol hydrochloride and latanoprost eye drops).
FIG. 14 represents a column diagram made according to data of an intra-ocular pressure variation value (14A) and an intra-ocular pressure actual measured value (14B) of each group of rabbits measured on the fifteenth day and the twentieth day when an ophthalmic medicine composition solution of Embodiments 147 to 156 and positive control drugs (Rocklatan^{®} and Mikeluna^{®}) are administered once a day for 10 days on end starting with the tenth day. On the twentieth day: &: P < 0.05, a treatment group 5 and a treatment group 9 compared with Embodiment groups 147 to 156; #: P < 0.05, Embodiment groups 147, 148 and 149 compared with Embodiment groups 150 and 151; _{*}: P < 0.05, Embodiment groups 152, 153 and 154 compared with Embodiment groups 155 and 156; and n.s: P > 0.05, Embodiment groups 150 and 151 compared with Embodiment groups 155 and 156.

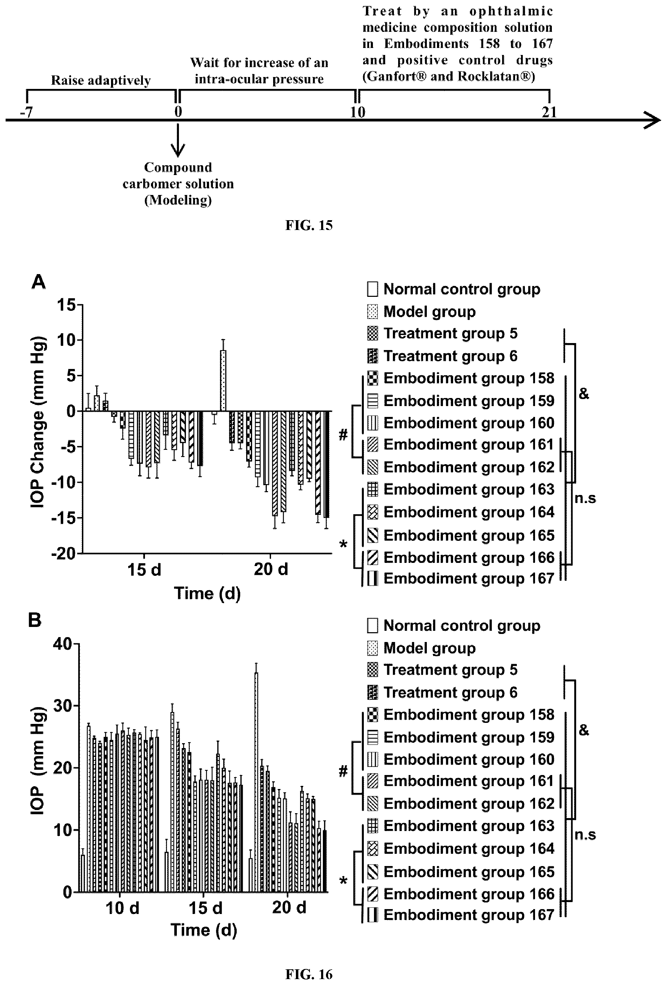

FIG. 15 represents a dosage regimen of an ophthalmic medicine composition solution in Embodiments 158 to 167, a positive control drug treatment group 5 and a positive control drug treatment group 6.
FIG. 16 represents a column diagram made according to data of an intra-ocular pressure variation value (16A) and an intra-ocular pressure actual measured value (16B) of each group of rabbits measured on the fifteenth day and the twentieth day when an ophthalmic medicine composition solution of Embodiments 158 to 167 and positive control drugs (Rocklatan^{®} and Ganfort^{®}) are administered once a day for 10 days on end starting with the tenth day. On the twentieth day: &: P < 0.05, a treatment group 5 and a treatment group 6 compared with Embodiment groups 158 to 167; #: P < 0.05, Embodiment groups 158, 159 and 160 compared with Embodiment groups 161 and 162; _{*}: P < 0.05, Embodiment groups 163, 164 and 165 compared with Embodiment groups 166 and 167; and n.s: P > 0.05, Embodiment groups 161 and 162 compared with Embodiment groups 166 and 167.

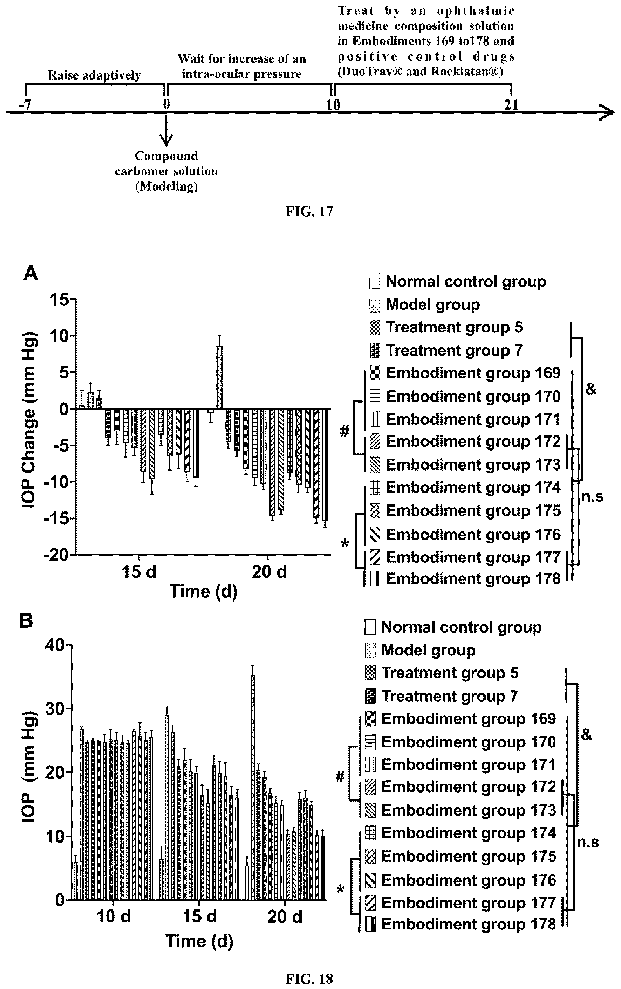

FIG. 17 represents a dosage regimen of an ophthalmic medicine composition solution in Embodiments 169 to 178, a positive control drug treatment group 5 and a positive control drug treatment group 7.
FIG. 18 represents a column diagram made according to data of an intra-ocular pressure variation value (18A) and an intra-ocular pressure actual measured value (18B) of each group of rabbits measured on the fifteenth day and the twentieth day when an ophthalmic medicine composition solution of Embodiments 169 to 178 and positive control drugs (Rocklatan^{®} and DuoTrav^{®}) are administered once a day for 10 days on end starting with the tenth day. On the twentieth day: &: P < 0.05, a treatment group 5 and a treatment group 7 compared with Embodiment groups 169 to 178; #: P < 0.05, Embodiment groups 169, 170 and 171 compared with Embodiment groups 172 and 173; _{*}: P < 0.05, Embodiment groups 174, 175 and 176 compared with Embodiment groups 177 and 178; and n.s: P > 0.05, Embodiment groups 172 and 173 compared with Embodiment groups 177 and 178.

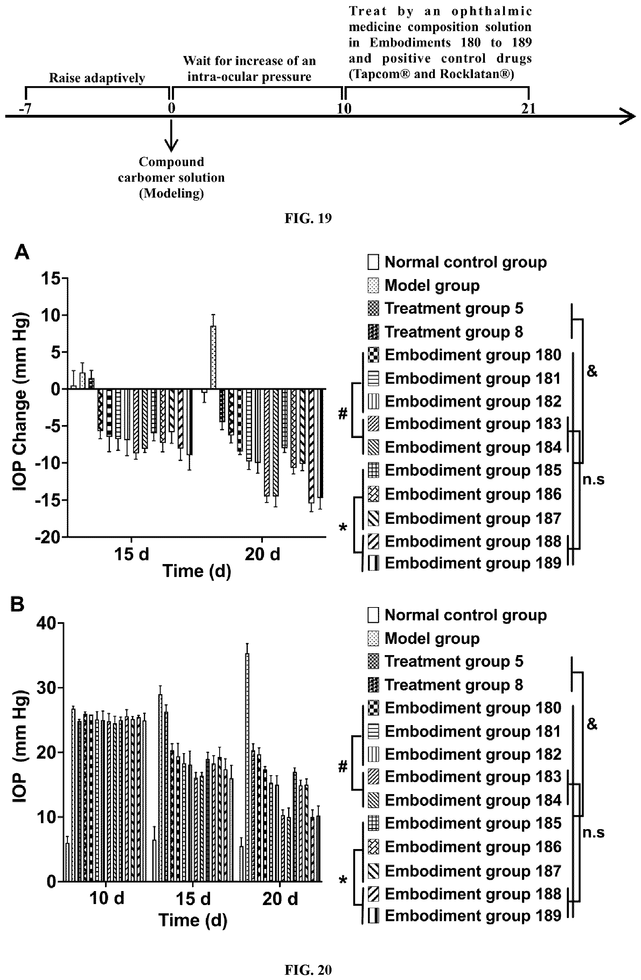

FIG. 19 represents a dosage regimen of an ophthalmic medicine composition solution in Embodiments 180 to 189, a positive control drug treatment group 5 and a positive control drug treatment group 8.
FIG. 20 represents a column diagram made according to data of an intra-ocular pressure variation value (20A) and an intra-ocular pressure actual measured value (20B) of each group of rabbits measured on the fifteenth day and the twentieth day when an ophthalmic medicine composition solution of Embodiments 180 to 189 and positive control drugs (Rocklatan^{®} and Tapcom^{®}) are administered once a day for 10 days on end starting with the tenth day. On the twentieth day: &: P < 0.05, a treatment group 5 and a treatment group 8 compared with Embodiment groups 180 to 189; #: P < 0.05, Embodiment groups 180, 181 and 182 compared with Embodiment groups 183 and 184; _{*}: P < 0.05, Embodiment groups 185, 186 and 187 compared with Embodiment groups 188 and 189; and n.s: P > 0.05, Embodiment groups 183 and 184 compared with Embodiment groups 188 and 189.

### DETAILED DESCRIPTION

In an ophthalmic medicine composition of the present invention, when netarsudil is dimesylate, timolol is preferably in a salt form formed from a pharmaceutically acceptable acid (except maleic acid). As specific acid, methanesulfonic acid, sulfuric acid, hydrobromic acid, phosphoric acid, nitric acid, citric acid, tartaric acid, salicylic acid, malic acid, ethanesulfonic acid, lactic acid, or phenylacetic acid may be listed, more preferably methanesulfonic acid.

In the ophthalmic medicine composition of the present invention, when the netarsudil is dimesylate, carteolol is preferably in a salt form formed from a pharmaceutically acceptable acid (except hydrochloric acid). As specific acid, methanesulfonic acid, sulfuric acid, hydrobromic acid, phosphoric acid, nitric acid, citric acid, tartaric acid, salicylic acid, malic acid, ethanesulfonic acid, lactic acid, or phenylacetic acid may be listed, more preferably methanesulfonic acid.

In the ophthalmic medicine composition of the present invention, when the netarsudil is dimesylate, betaxolol is preferably in a salt form formed from a pharmaceutically acceptable acid (except hydrochloric acid). As specific acid, methanesulfonic acid, sulfuric acid, hydrobromic acid, phosphoric acid, nitric acid, citric acid, tartaric acid, salicylic acid, malic acid, ethanesulfonic acid, lactic acid, or phenylacetic acid may be listed, more preferably methanesulfonic acid.

In the ophthalmic medicine composition of the present invention, when the netarsudil is dimesylate, metipranolol is preferably in a salt form formed from a pharmaceutically acceptable acid (except hydrochloric acid). As specific acid, methanesulfonic acid, sulfuric acid, hydrobromic acid, phosphoric acid, nitric acid, citric acid, tartaric acid, salicylic acid, malic acid, ethanesulfonic acid, lactic acid, or phenylacetic acid may be listed, more preferably methanesulfonic acid.

In an ophthalmic medicine composition solution of the present invention, when the timolol is maleate, the carteolol is hydrochloride, the betaxolol is hydrochloride, or the metipranolol is hydrochloride, the netarsudil is preferably in a salt form formed from a pharmaceutically acceptable acid (except methanesulfonic acid). As specific acid, maleic acid, sulfuric acid, hydrobromic acid, hydrochloric acid, formic acid, nitric acid, acetic acid, phenylacetic acid, butanedioic acid, oxalic acid, hydroiodic acid, or propionic acid may be listed, more preferably maleic acid and hydrochloric acid.

In the medicine composition of the present invention, there is no particular limit as long as salts of the timolol, the carteolol, the betaxolol, the metipranolol and the netarsudil are pharmacologically permissible salts. Specifically, examples of appropriate inorganic anions include but are not limited to inorganic anions derived from the following inorganic acid: hydrochloric acid, hydrobromic acid, hydroiodic acid, sulfuric acid, sulphurous acid, nitric acid, nitrous acid, phosphoric acid and phosphorous acid. Examples of appropriate organic anions include but are not limited to organic anions derived from the following organic acid: 2-(acetoxy)benzoic acid, acetic acid, ascorbic acid, aspartic acid, benzoic acid, camphorsulfonic acid, cinnamic acid, citric acid, edetic acid, ethanedisulfonic acid, ethanesulfonic acid, fumaric acid, glucoheptonic acid, gluconic acid, glutamic acid, glycolic acid hydroxymaleic acid, hydroxynaphthalene carboxylic acid, isethionic acid, lactic acid, lactobionic acid, lauric acid, maleic acid, malic acid, ethanesulfonic acid, methanesulfonic acid, galactaric acid, oleic acid, oxalic acid, palmitic acid, pamoic acid, pantothenic acid, phenylacetic acid, sulfonic acid, propionic acid, pyruvic acid, salicylic acid, stearic acid, succinic acid, sulfanilic acid, tartaric acid, p-toluenesulfonic acid, and valeric acid. Examples of appropriate polymer organic anions include but are not limited to polymer organic anions derived from the following polyacid: tannic acid and carboxymethylcellulose.

The medicine composition of the present invention further includes at least one type of prostaglandin analog as an active compound. Specifically, examples of the prostaglandin analog include but are not limited to prostaglandin of latanoprost, bimatoprost, travoprost, tafluprost, AR-102, cloprostenol isopropyl ester, 13,14-dihydrocloprostenol isopropyl ester, latanoprostene bunod, unoprostone, PGF1α isopropyl ester, PGF2α isopropyl ester, PGF3α isopropyl ester and fluprostenol isopropyl ester.

In the ophthalmic medicine composition solution of the present invention, the β-adrenergic receptor blocker (timolol free alkali or salts of the same, carteolol free alkali or salts of the same, betaxolol free alkali or salts of the same, or metipranolol free alkali or salts of the same) has a concentration in a range from 0.02%w/v to 4.0%w/v, more preferably 0.3%w/v to 2.0%w/v.

In the ophthalmic medicine composition solution of the present invention, the netarsudil free alkali or salts of the same has/have a concentration in a range from 0.005%w/v to 0.1%w/v, more preferably 0.02%w/v to 0.04%w/v, most preferably 0.02%w/v.

In the ophthalmic medicine composition solution of the present invention, the prostaglandin analog has a concentration in a range from 0.0005%w/v to 0.05%w/v, more preferably 0.0015%w/v to 0.03%w/v.

The ophthalmic medicine composition solution of the present invention may include a tonicity agent. Examples of the tonicity agent include but are not limited to sodium chloride, potassium chloride, mannitol, dextrose, glycerol and propylene glycol. Appropriately, the tonicity agent may exist in the ophthalmic composition in an amount of about 0.01%w/v to about 10%w/v, or about 1%w/v to about 10%w/v, or about 2.5%w/v to about 7.5%w/v, or about 4%w/v to about 6 %w/v. Appropriately, the tonicity agent may exist in an amount of at least 0.01%w/v, at least 0.05 %w/v, at least 1 %w/v, at least 2.5 %w/v, at least 4 %w/v, or at least 5 %w/v. Appropriately, the tonicity agent may exist in an amount not exceeding 10%w/v, not exceeding 8%w/v, or not exceeding 6%w/v. In an implementation, the tonicity agent may exist in the ophthalmic composition in an amount of about 3.0%w/v, about 3.1%w/v, about 3.2%w/v, about 3.3%w/v, about 3.4%w/v, about 3.5%w/v, about 3.6%w/v, about 3.7%w/v, about 3.8%w/v, about 3.9%w/v, about 4.0%w/v, about 4.1%w/v, about 4.2%w/v, about 4.3%w/v, about 4.4%w/v, about 4.5%w/v, about 4.6%w/v, about 4.7%w/v, about 4.8%w/v, about 4.9%w/v, about 5.0%w/v, about 5.1%w/v, about 5.2 %w/v, about 5.3 %w/v, about 5.4 %w/v or about 5.5 % w/v. The tonicity agent may be appropriately mannitol.

The ophthalmic medicine composition solution of the present invention may include a buffering agent. The appropriate buffering agent includes but is not limited to acetic acid, citric acid, carbonic acid, phosphoric acid, boric acid, pharmaceutically acceptable salts of them, tromethamine, and a combination of them. The buffering agent may exist in the ophthalmic composition in an amount of about 0.01 %w/v to about 1 %w/v, or about 0.1 %w/v to about 0.9 %w/v, or about 0.3 %w/v to about 0.8 %w/v, or about 0.4 %w/v to about 0.6%w/v. Appropriately, the buffering agent may exist in an amount of at least 0.01%w/v, at least 0.05 %w/v, at least 0.1 %w/v, at least 0.3 %w/v, or at least 0.5%w/v. Appropriately, the buffering agent may exist in an amount not exceeding 1.0%w/v, not exceeding 0.8%w/v, or not exceeding 0.6%w/v. In an implementation, the buffering agent may exist in the ophthalmic composition in an amount of about 0.01%w/v, about 0.02%w/v, about 0.03%w/v, about 0.04%w/v, about 0.05%w/v, about 0.06%w/v, about 0.07%w/v, about 0.08%w/v, about 0.09% w/v, or about 0.1% w/v. The buffering agent may be appropriately boric acid.

The ophthalmic medicine composition solution of the present invention may include a preservative. The appropriate preservative includes but is not limited to benzalkonium chloride, thimerosal, chlorbutanol, methyl p-hydroxybenzoate, propyl p-hydroxybenzoate, phenethyl alcohol, edetate disodium, boric acid, sorbic acid or another reagent known to those skilled in the art. The preservative may exist in the ophthalmic composition in an amount of about 0.001 %w/v to about 0.02 %w/v. The preservative may be appropriately benzalkonium chloride. The ophthalmic medicine composition solution of the present invention may include a pH regulator, and an amount of the pH regulator is enough to regulate pH of the composition to a range from about 4 to about 9. Appropriately, the ophthalmic composition may have pH in a range from about 4.5 to about 5.4. The appropriate pH regulator includes but is not limited to sodium hydroxide.

### Embodiments

The present disclosure has a plurality of aspects described through the following non-restrictive embodiments. In various embodiments, the following materials and characterization technique are used.

### Embodiments 1 to 5: a preparation including netarsudil dimesylate and carteolol mesylate

**Table 1. Preparation of a local ophthalmic medicine composition solution for reducing an intra-ocular pressure as follows:**

| No. | Component | Embodiment 1 | Embodiment 2 | Embodiment 3 | Embodiment 4 | Embodiment 5 |
|---|---|---|---|---|---|---|
| 1. | Netarsudil dimesylate | 14.2 mg | 14.2 mg | 28.5 mg | 28.5 mg | 57.0 mg |
| 2. | Carteolol mesylate | 1329 mg | 2658 mg | 1329 mg | 2658 mg | 5316 mg |
| 3. | Boric acid | 50 mg | 50 mg | 50 mg | 50 mg | 50 mg |
| 4. | Mannitol | 3700 mg | 3700 mg | 3700 mg | 3700 mg | 3700 mg |
| 5. | Benzalkonium chloride | 5 mg | 5 mg | 5 mg | 5 mg | 5 mg |
| 6. | Sodium hydroxide | q.s. | q.s. | q.s. | q.s. | q.s. |
| 7. | Water for injection added by | 100 ml | 100 ml | 100 ml | 100 ml | 100 |

A preparation process is as follows: 1) 95% of a prescription dosage of water for injection is weighed, prescription dosages of active components and adjuvants are added and stirred to be dissolved completely, and pH is regulated with a sodium hydroxide solution (10%) to a range from 4.5 to 5.4; 2) water for injection is supplemented to a full prescription dosage, stirring is performed to be uniform, and a volume reaches 100%; and 3) the solution in step 2 is put in a low-density polyethylene medicinal eye drop bottle, and its stability is studied under different storage temperature conditions.

**Table 2. Contents of various main medicine components in Embodiments 1 to 5 after being stored at 5°Cfor 24 months**

| | | | | | | |
|---|---|---|---|---|---|---|
| No. | Component | Embodiment 1 | Embodiment 2 | Embodiment 3 | Embodiment 4 | Embodiment 5 |

| | | Content % | | | | |
|---|---|---|---|---|---|---|
| 1. | Netarsudil dimesylate | 99.3 | 98.6 | 97.4 | 99.1 | 99.0 |
| 2. | Carteolol mesylate | 99.7 | 99.2 | 99.3 | 99.0 | 99.1 |

**Table 3. Contents of various main medicine components in commercially available eye drops (Mikelan^{®} and Rhopress^{®}) after being stored at 5°Cfor 24 months**

| No | Component | Mikelan^{®} | Rhopress^{®} |
|---|---|---|---|
| | | Content % | |
| 1. | Netarsudil dimesylate | - | 100.0 |
| 2. | Carteolol hydrochloride | 99.9 | - |

According to Embodiments 1 to 5, the preparation including netarsudil dimesylate and carteolol mesylate has good stability after being stored under a storage condition of 5°Cfor 24 months, which meets a requirement for a storage condition of the commercially available eye drops (Mikelan^{®} and Rhopress^{®}) before opening.

**Table 4. Contents of various main medicine components in Embodiments 1 to 5 after being stored at 25°Cfor 6 weeks**

| No. | Component | Embodiment 1 | Embodiment 2 | Embodiment 3 | Embodiment 4 | Embodiment 5 |
|---|---|---|---|---|---|---|
| | | Content % | | | | |
| 1. | Netarsudil dimesylate | 99.1 | 99.0 | 99.3 | 99.1 | 98.7 |
| 2. | Carteolol mesylate | 100.1 | 101.2 | 100.6 | 100.4 | 100.6 |

**Table 5. Contents of various main medicine components in commercially available eye drops (Mikelan^{®} and Rhopress^{®}) after being stored at 25°Cfor 6 weeks**

| No. | Component | Mikelan^{®} | Rhopress^{®} |
|---|---|---|---|
| | | Content % | |
| 1. | Netarsudil dimesylate | - | 99.9 |
| 2. | Carteolol hydrochloride | 99.9 | - |

According to Embodiments 1 to 5, the preparation including netarsudil dimesylate and carteolol mesylate has good stability after being stored under a storage condition of 25°Cfor 6 weeks, which meets a requirement for a storage condition of the commercially available eye drops (Mikelan^{®} and Rhopress^{®}) after opening.

**Table 6. Contents of various main medicine components in Embodiments 1 to 5 after being stored at 40°Cfor 14 days**

| No. | Component | Embodiment 1 | Embodiment 2 | Embodiment 3 | Embodiment 4 | Embodiment 5 |
|---|---|---|---|---|---|---|
| | | Content % | | | | |
| 1. | Netarsudil dimesylate | 98.9 | 99.5 | 99.0 | 99.1 | 99.9 |
| 2. | Carteolol mesylate | 99.9 | 100.0 | 101.3 | 100.5 | 100.3 |

**Table 7. Contents of various main medicine components in commercially available eye drops (Mikelan^{®} and Rhopress^{®}) after being stored at 40°Cfor 14 days**

| No. | Component | Mikelan^{®} | Rhopress^{®} |
|---|---|---|---|
| | | Content % | |
| 1. | Netarsudil dimesylate | - | 99.3 |
| 2. | Carteolol hydrochloride | 99.5 | - |

According to Embodiments 1 to 5, the preparation including netarsudil dimesylate and carteolol mesylate has good stability after being stored under a storage condition of 40°Cfor 14 days, which meets a requirement for departing from a storage condition of the commercially available eye drops (Mikelan^{®} and Rhopress^{®}) for a short time.

### Embodiments 6 to 10: a preparation including netarsudil dihydrochloride and carteolol hydrochloride

**Table 8. Preparation of a local ophthalmic medicine composition solution for reducing an intra-ocular pressure as follows:**

| No. | Component | Embodiment 6 | Embodiment 7 | Embodiment 8 | Embodiment 9 | Embodiment 10 |
|---|---|---|---|---|---|---|
| 1. | Netarsudil dihydrochloride | 11.6 mg | 11.6 mg | 23.3 mg | 23.3 mg | 46.6 mg |
| 2. | Carteolol hydrochloride | 1000 mg | 2000 mg | 1000 mg | 2000 mg | 4000 mg |
| 3. | Boric acid | 50 mg | 50 mg | 50 mg | 50 mg | 50 mg |
| 4. | Mannitol | 3700 mg | 3700 mg | 3700 mg | 3700 mg | 3700 mg |
| 5. | Benzalkonium chloride | 5 mg | 5 mg | 5 mg | 5 mg | 5 mg |
| 6. | Sodium hydroxide | q.s. | q.s. | q.s. | q.s. | q.s. |
| 7. | Water for injection added by | 100 mL | 100 mL | 100 mL | 100 mL | 100 |

A preparation process may refer to the preparation process in Embodiments 1 to 5.

**Table 9. Contents of various main medicine components in Embodiments 6 to 10 after being stored at 5°Cfor 24 months**

| No. | Component | Embodiment 6 | Embodiment 7 | Embodiment 8 | Embodiment 9 | Embodiment 10 |
|---|---|---|---|---|---|---|
| | | Content % | | | | |
| 1. | Netarsudil dihydrochloride | 100.2 | 100.1 | 99.5 | 99.3 | 99.1 |
| 2. | Carteolol hydrochloride | 101.3 | 100.8 | 100.3 | 102.3 | 100.2 |

According to Embodiments 6 to 10, the preparation including netarsudil dihydrochloride and carteolol hydrochloride has good stability after being stored under a storage condition of 5°Cfor 24 months, which meets a requirement for a storage condition of the commercially available eye drops (Mikelan^{®} and Rhopress^{®}) before opening.

**Table 10. Contents of various main medicine components in Embodiments 6 to 10 after being stored at 25°Cfor 6 weeks**

| No. | Component | Embodiment 6 | Embodiment 7 | Embodiment 8 | Embodiment 9 | Embodiment 10 |
|---|---|---|---|---|---|---|
| | | Content % | | | | |
| 1. | Netarsudil dihydrochloride | 97.9 | 98.4 | 99.9 | 99.1 | 99.0 |
| 2. | Carteolol hydrochloride | 99.7 | 99.4 | 99.5 | 99.6 | 99.8 |

According to Embodiments 6 to 10, the preparation including netarsudil dihydrochloride and carteolol hydrochloride has good stability after being stored under a storage condition of 25°Cfor 6 weeks, which meets a requirement for a storage condition of the commercially available eye drops (Mikelan^{®} and Rhopress^{®}) after opening.

**Table 11. Contents of various main medicine components in Embodiments 6 to 10 after being stored at 40°Cfor 14 days**

| No. | Component | Embodiment 6 | Embodiment 7 | Embodiment 8 | Embodiment 9 | Embodiment 10 |
|---|---|---|---|---|---|---|
| | | Content % | | | | |
| 1. | Netarsudil dihydrochloride | 99.9 | 99.7 | 99.8 | 99.9 | 99.8 |
| 2. | Carteolol hydrochloride | 100.3 | 99.9 | 98.6 | 99.5 | 99.5 |

According to Embodiments 6 to 10, the preparation including netarsudil dihydrochloride and carteolol hydrochloride has good stability after being stored under a storage condition of 40°Cfor 14 days, which meets a requirement for departing from a storage condition of the commercially available eye drops (Mikelan^{®} and Rhopress^{®}) for a short time.

### Embodiment 11: evaluation of pharmaceutical effects of the ophthalmic medicine composition solution in Embodiments 1 to 10 by using a a Japanese white rabbit glaucoma model

Experimental animal: healthy male Japanese white rabbit (Shenyang Pharmaceutical University, License Number: SYXK (Liaoning) 2018-0009), original weight 2.5 kg to 3.0 kg, 3 rabbits/group.

### Experimental grouping:

Normal control group: no processing.
Model group: an intraocular hypertension model is established, and no eye drop is administered.
Treatment group 1: an intraocular hypertension model is established, and commercially available netarsudil eye drops (Rhopress^{®}) are administered.
Treatment group 2: an intraocular hypertension model is established, and commercially available carteolol hydrochloride eye drops (Mikelan^{®}) are administered.
Embodiment group 1: an intraocular hypertension model is established, and an ophthalmic medicine composition solution in Embodiment 1 is administered.
Embodiment group 2: an intraocular hypertension model is established, and an ophthalmic medicine composition solution in Embodiment 2 is administered.
Embodiment group 3: an intraocular hypertension model is established, and an ophthalmic medicine composition solution in Embodiment 3 is administered.
Embodiment group 4: an intraocular hypertension model is established, and an ophthalmic medicine composition solution in Embodiment 4 is administered.
Embodiment group 5: an intraocular hypertension model is established, and an ophthalmic medicine composition solution in Embodiment 5 is administered.
Embodiment group 6: an intraocular hypertension model is established, and an ophthalmic medicine composition solution in Embodiment 6 is administered.
Embodiment group 7: an intraocular hypertension model is established, and an ophthalmic medicine composition solution in Embodiment 7 is administered.
Embodiment group 8: an intraocular hypertension model is established, and an ophthalmic medicine composition solution in Embodiment 8 is administered.
Embodiment group 9: an intraocular hypertension model is established, and an ophthalmic medicine composition solution in Embodiment 9 is administered.
Embodiment group 10: an intraocular hypertension model is established, and an ophthalmic medicine composition solution in Embodiment 10 is administered.

Model establishment: no processing is made to rabbits in the normal control group, urethane 0.8 - 1g/kg is injected into ear veins of the other 13 groups of white rabbits on the zero day for general anesthesia, 10 µL of aqueous humor is extracted from an anterior chamber of each eye by using a 1 mL injection syringe, 10 µL compound carbomer solution (including, percentage by weight, 0.3% carbomer as a main drug and 0.025% dexamethasone) is injected, an intra-ocular pressure is measured every two days, and clinical manifestation is observed. A successful model has turbidity in the anterior chamber and significant increase of the intra-ocular pressure. An ocular hypertension rabbit model is established successfully after 10 days.

A dosage regimen: according to a current clinical dosage regimen of commercially available netarsudil eye drops (Rhopress^{®}) and commercially available carteolol hydrochloride eye drops (Mikelan^{®}), this experiment is mainly divided into the following groups: (1) the treatment group 1: 50 µL of the commercially available netarsudil eye drops (Rhopress^{®}) is administered to each eye each time once a day (9:00 PM); (2) the treatment group 2: 50 µL of the commercially available carteolol hydrochloride eye drops (Mikelan^{®}) is administered to each eye each time twice a day (9:00 AM and 9:00 PM); and (3) Embodiment groups 1 to 10: 50 µL eye drops in Embodiments 1 to 10 are administered to each eye each time once a day (9:00 PM). Drug administration is started in all the groups on the tenth day when the models are established successfully and lasts for 10 days. Eye drops are not administered to the normal control group and the model group. The dosage regimen is shown in FIG. 1.

A test index and method:
intra-ocular pressure: the intra-ocular pressure is tested once a day (9:00 PM) on the zeroth, second, fourth, sixth, eighth, tenth, eleventh, twelfth, thirteenth, fourteenth, fifteenth, sixteenth, seventeenth, eighteenth, nineteenth and twentieth days of the experiment. Upper and lower eyelids of the rabbits are opened gently, an ophthalmotonometer approaches to eyes of the rabbits, a distance is adjusted well, a measurement button is pressed gently, the eyelids are not touched during a measurement process, 6 times of measurement are completed in sequence, and then a single measurement value and an average value (mmHg) may be obtained.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 1, the treatment group 2 and Embodiment groups 1, 2, 3, 6, 7 and 8, the IOP can be reduced significantly by the ophthalmic composition solutions of Embodiment groups 4, 5, 9 and 10, however, IOP actual measured values and IOP variation values in Embodiment groups 4, 5, 9 and 10 have no significant difference.

**Table 12. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| | IOP variation value (mmHg) | | | | | | | | | | | | | |
|---|---|---|---|---|---|---|---|---|---|---|---|---|---|---|
| Ti m e (d ) | No rm al co ntr ol gro up | M od el gr ou p | Trea tme nt grou p1 | Trea tme nt grou p2 | Embo dime nt group 1 | Embo dime nt group 2 | Embo dime nt group 3 | Embo dime nt group 4 | Embo dime nt group 5 | Embo dime nt group 6 | Embo dime nt group 7 | Embo dime nt group 8 | Embo dime nt group 9 | Embo dime nt group 10 |
| 1 0 d | - | - | - | - | - | - | - | - | - | - | - | - | - | - |
| 1 5 d | 0.5 0 | -1. 88 | -3.0 9 | -2.9 5 | -3.50 | -3.98 | -4.69 | -6.60 | -6.86 | -4.96 | -3.82 | -3.30 | -5.80 | -5.00 |
| 2 0 d | -0. 50 | 2. 10 | -4.4 3 | -4.9 5 | -7.90 | -8.34 | -9.07 | -12.5 0 | -12.8 0 | -9.10 | -8.75 | -8.44 | -12.3 0 | -11.7 3 |

**Table 13. Intra-ocular pressure actual measured values of each group of rabbits measured on the tenth day, the fifteenth day and the twentieth day when drug administration is performed for 10 days on end starting with the tenth day**

| IOP actual measured value (mmHg) | | | | | | | | | | | | | | |
|---|---|---|---|---|---|---|---|---|---|---|---|---|---|---|
| Ti m e (d ) | No rm al co ntrol gro up | M od el gr ou p | Trea tme nt grou p 1 | Trea tme nt grou p2 | Embo dime nt group 1 | Embo dime nt group 2 | Embo dime nt group 3 | Embo dime nt group 4 | Embo dime nt group 5 | Embo dime nt group 6 | Embo dime nt group 7 | Embo dime nt group 8 | Embo dime nt group 9 | Embo dime nt group 10 |
| 1 0 d | 6.0 0 | 26 .2 3 | 25.3 7 | 24.9 5 | 25.00 | 25.34 | 25.67 | 25.50 | 26.00 | 26.50 | 26.00 | 25.30 | 25.80 | 25.00 |
| 1 5 d | 6.5 0 | 24 .3 5 | 22.2 8 | 22.0 0 | 21.50 | 21.36 | 20.98 | 18.90 | 19.14 | 21.54 | 22.18 | 22.00 | 20.00 | 20.00 |
| 2 0 d | 5.5 0 | 28 .3 3 | 20.9 4 | 20.0 0 | 17.10 | 17.00 | 16.60 | 13.00 | 13.20 | 17.40 | 17.25 | 16.86 | 13.50 | 13.27 |

### Embodiments 12: a preparation including netarsudil dimesylate and carteolol hydrobromide

**Table 14. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol hydrobromide | 2550 mg |
| 3. | Boric acid | 50 mg |
| 4. | Mannitol | 4300 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 15. Contents of various main medicine components in Embodiment 12 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.3 | 100.5 |
| 2. | Carteolol hydrobromide | 101.4 | 100.7 |

According to Embodiment 12, the preparation including netarsudil dimesylate and carteolol hydrobromide has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiments 13: a preparation including netarsudil dimesylate and carteolol sulfate

**Table 16. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol sulfate | 2680 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 17. Contents of various main medicine components in Embodiment 13 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.3 | 98.9 |
| 2. | Carteolol sulfate | 100.3 | 100.5 |

According to Embodiment 13, the preparation including netarsudil dimesylate and carteolol sulfate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 14: a preparation including netarsudil dimesylate and carteolol esilate

**Table 18. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol esilate | 3340 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 19. Contents of various main medicine components in Embodiment 14 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.5 | 101.1 |
| 2. | Carteolol esilate | 100.8 | 100.4 |

According to Embodiment 14, the preparation including netarsudil dimesylate and carteolol esilate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiments 15: a preparation including netarsudil dimesylate and carteolol nitrate

**Table 20. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol nitrate | 2430 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 21. Contents of various main medicine components in Embodiment 15 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.7 | 100.2 |
| 2. | Carteolol nitrate | 99.9 | 99.4 |

According to Embodiment 15, the preparation including netarsudil dimesylate and carteolol nitrate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiments 16: a preparation including netarsudil dimesylate and carteolol citrate

**Table 22. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol citrate | 3770 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 23. Contents of various main medicine components in Embodiment 16 after being stored at 5°Cfor 24 months and at 25°Cfor 6 weeks**

| No. | Component | 5°C | 25°C |
|---|---|---|---|
| | | Content % | |
| 1. | Netarsudil dimesylate | 98.5 | 99.2 |
| 2. | Carteolol citrate | 100.5 | 101.4 |

According to Embodiment 16, the preparation including netarsudil dimesylate and carteolol citrate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 17: a preparation including netarsudil dimesylate and carteolol tartrate

**Table 24. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol tartrate | 3030 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 25. Contents of various main medicine components in Embodiment 17 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.4 | 99.7 |
| 2. | Carteolol tartrate | 101.4 | 100.3 |

According to Embodiment 17, the preparation including netarsudil dimesylate and carteolol tartrate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiments 18: a preparation including netarsudil dimesylate and carteolol salicylate

**Table 26. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol salicylate | 2940 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 27. Contents of various main medicine components in Embodiment 18 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.9 | 100.5 |
| 2. | Carteolol salicylate | 99.3 | 99.1 |

According to Embodiment 18, the preparation including netarsudil dimesylate and carteolol salicylate has good stability after being stored under a storage condition of 5°Cfor 24 months, which meets a requirement for a storage condition of the commercially available eye drops (Mikelan^{®}and Rhopress^{®}) before opening; and has good stability after being stored under a storage condition of 25°Cfor 6 weeks, which meets a requirement for a storage condition of the commercially available eye drops (Mikelan^{®}and Rhopress^{®}) after opening.

### Embodiment 19: a preparation including netarsudil dimesylate and carteolol malate

**Table 28. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol malate | 3690 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 29. Contents of various main medicine components in Embodiment 19 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.8 | 99.7 |
| 2. | Carteolol malate | 100.4 | 99.6 |

According to Embodiment 19, the preparation including netarsudil dimesylate and carteolol malate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®}and Rhopress^{®}) after opening.

### Embodiment 20: a preparation including netarsudil dimesylate and carteolol lactate

**Table 30. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol lactate | 2620 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 31. Contents of various main medicine components in Embodiment 20 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.4 | 100.1 |
| 2. | Carteolol lactate | 101.2 | 100.7 |

According to Embodiment 20, the preparation including netarsudil dimesylate and carteolol lactate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 21: a preparation including netarsudil dimesylate and carteolol phenylacetate

**Table 32. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Carteolol phenylacetate | 3410 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 33. Contents of various main medicine components in Embodiment 21 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.4 | 100.1 |
| 2. | Carteolol phenylacetate | 99.9 | 99.9 |

According to Embodiment 21, the preparation including netarsudil dimesylate and carteolol phenylacetate has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 22: a preparation including netarsudil dihydrobromide and carteolol hydrochloride

**Table 34. Preparation of a local ophthalmic medicine composition solution for reducing an intra-ocular pressure as follows:**

| No. | Component | Weight |
|---|---|---|
| 1. | Netarsudil dihydrobromide | 27.1 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 35. Contents of various main medicine components in Embodiment 22 after being stored at 5°Cfor 24 months and at 25°Cfor 6 weeks**

| No. | Component | 5°C | 25°C |
|---|---|---|---|
| | | Content % | |
| 1. | Netarsudil dihydrobromide | 99.2 | 100.1 |
| 2. | Carteolol hydrochloride | 100.4 | 99.9 |

According to Embodiment 22, the preparation including netarsudil dihydrobromide and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 23: a preparation including netarsudil sulfate and carteolol hydrochloride

**Table 36. Preparation of a local ophthalmic medicine composition solution for reducing an intra-ocular pressure as follows:**

| No. | Component | Weight |
|---|---|---|
| 1. | Netarsudil sulfate | 24.3 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 37. Contents of various main medicine components in Embodiment 23 after being stored at 5°Cfor 24 months and at 25°Cfor 6 weeks**

| No. | Component | 5°C | 25°C |
|---|---|---|---|
| | | Content % | |
| 1. | Netarsudil sulfate | 100.2 | 100.1 |
| 2. | Carteolol hydrochloride | 99.7 | 100.1 |

According to Embodiment 23, the preparation including netarsudil sulfate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 24: a preparation including netarsudil diformate and carteolol hydrochloride

**Table 38. Preparation of a local ophthalmic medicine composition solution for reducing an intra-ocular pressure as follows:**

| No. | Component | Weight |
|---|---|---|
| 1. | Netarsudil diformate | 24.1 mg |
| 2. | Carteolol hydrochloride | 2000 mg |
| 3. | Boric acid | 50 mg |
| 4. | Mannitol | q.s. |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 39. Contents of various main medicine components in Embodiment 24 after being stored at 5°Cfor 24 months and at 25°Cfor 6 weeks**

| No. | Component | 5°C | 25°C |
|---|---|---|---|
| | | Content % | |
| 1. | Netarsudil diformate | 99.5 | 99.4 |
| 2. | Carteolol hydrochloride | 100.2 | 100.5 |

According to Embodiment 24, the preparation including netarsudil diformate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 25: a preparation including netarsudil nitrate and carteolol hydrochloride

**Table 40. Preparation of a local ophthalmic medicine composition solution for reducing an intra-ocular pressure as follows:**

| No. | Component | Weight |
|---|---|---|
| 1. | Netarsudil nitrate | 25.6 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 41. Contents of various main medicine components in Embodiment 25 after being stored at 5°Cfor 24 months and at 25°Cfor 6 weeks**

| No. | Component | 5°C | 25°C |
|---|---|---|---|
| | | Content % | |
| 1. | Netarsudil nitrate | 99.7 | 99.7 |
| 2. | Carteolol hydrochloride | 100.2 | 101.7 |

According to Embodiment 25, the preparation including netarsudil nitrate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 26: a preparation including netarsudil diacetate and carteolol hydrochloride

**Table 42. Preparation of a local ophthalmic medicine composition solution for reducing an intra-ocular pressure as follows:**

| No. | Component | Weight |
|---|---|---|
| 1. | Netarsudil diacetate | 27.8 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 43. Contents of various main medicine components in Embodiment 26 after being stored at 5°Cfor 24 months and at 25°Cfor 6 weeks**

| No. | Component | 5°C | 25°C |
|---|---|---|---|
| | | Content % | |
| 1. | Netarsudil diacetate | 99.1 | 98.5 |
| 2. | Carteolol hydrochloride | 99.8 | 99.5 |

According to Embodiment 26, the preparation including netarsudil diacetate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 27: a preparation including netarsudil dibenzoate and carteolol hydrochloride

**Table 44. Preparation of a local ophthalmic medicine composition solution for reducing an intra-ocular pressure as follows:**

| No. | Component | Weight |
|---|---|---|
| 1. | Netarsudil dibenzoate | 30.8 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Pure water | q.s. |

A preparation process may refer to the preparation process in Embodiments 1 to 5.

**Table 45. Contents of various main medicine components in Embodiment 27 after being stored at 5°Cfor 24 months and at 25°Cfor 6 weeks**

| No. | Component | 5°C | 25°C |
|---|---|---|---|
| | | Content % | |
| 1. | Netarsudil dibenzoate | 100.2 | 101.2 |
| 2. | Carteolol hydrochloride | 100.4 | 100.7 |

According to Embodiment 27, the preparation including netarsudil dibenzoate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 28: a preparation including netarsudil oxalate and carteolol hydrochloride

**Table 46. Preparation of a local ophthalmic medicine composition solution for reducing an intra-ocular pressure as follows:**

| No. | Component | Weight |
|---|---|---|
| 1. | Netarsudil oxalate | 27.9 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 47. Contents of various main medicine components in Embodiment 28 after being stored at 5°Cfor 24 months and at 25°Cfor 6 weeks**

| No. | Component | 5°C | 25°C |
|---|---|---|---|
| | | Content % | |
| 1. | Netarsudil oxalate | 99.9 | 100.3 |
| 2. | Carteolol hydrochloride | 100.9 | 99.8 |

According to Embodiment 28, the preparation including netarsudil oxalate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 29: a preparation including netarsudil succinate and carteolol hydrochloride

**Table 48. Preparation of a local ophthalmic medicine composition solution for reducing an intra-ocular pressure as follows:**

| No. | Component | Weight |
|---|---|---|
| 1. | Netarsudil succinate | 27.9 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 49. Contents of various main medicine components in Embodiment 29 after being stored at 5°Cfor 24 months and at 25°Cfor 6 weeks**

| No. | Component | 5°C | 25°C |
|---|---|---|---|
| | | Content % | |
| 1. | Netarsudil succinate | 98.1 | 99.0 |
| 2. | Carteolol hydrochloride | 100.1 | 100.7 |

According to Embodiment 29, the preparation including netarsudil succinate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 30: a preparation including netarsudil diphenylacetate and carteolol hydrochloride

**Table 50. Preparation of a local ophthalmic medicine composition solution for reducing an intra-ocular pressure as follows:**

| No. | Component | Weight |
|---|---|---|
| 1. | Netarsudil diphenylacetate | 32 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 51. Contents of various main medicine components in Embodiment 30 after being stored at 5°Cfor 24 months and at 25°Cfor 6 weeks**

| No. | Component | 5°C | 25°C |
|---|---|---|---|
| | | Content % | |
| 1. | Netarsudil diphenylacetate | 99.4 | 99.8 |
| 2. | Carteolol hydrochloride | 99.1 | 99.0 |

According to Embodiment 30, the preparation including netarsudil diphenylacetate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Embodiment 31: a preparation including netarsudil maleate and carteolol hydrochloride

**Table 52. Preparation of a local ophthalmic medicine composition solution for reducing an intra-ocular pressure as follows:**

| No. | Component | Weight |
|---|---|---|
| 1. | Netarsudil maleate | 25.1 mg |
| 2. | Carteolol hydrochloride | 2000 mg |
| 3. | Boric acid | 50 mg |
| 4. | Mannitol | 3700 mg |
| 5. | Benzalkonium chloride | 5 mg |
| 6. | Sodium hydroxide | q.s. |
| 7. | Water for injection added by | 100 |

A preparation process may refer to the preparation process in Embodiments 1 to 5.

**Table 53. Contents of various main medicine components in Embodiment 31 after being stored at 5°Cfor 24 months and at 25°Cfor 6 weeks**

| No. | Component | 5°C | 25°C |
|---|---|---|---|
| | | Content % | |
| 1. | Netarsudil maleate | 99.4 | 99.8 |
| 2. | Carteolol hydrochloride | 99.1 | 99.0 |

According to Embodiment 31, the preparation including netarsudil maleate and carteolol hydrochloride has good stability after being stored under a storage condition of 5°Cfor 24 months, which meets a requirement for a storage condition of the commercially available eye drops (Mikelan^{®} and Rhopress^{®}) before opening; and has good stability after being stored under a storage condition of 25°Cfor 6 weeks, which meets a requirement for a storage condition of the commercially available eye drops (Mikelan^{®} and Rhopress^{®}) after opening.

### Comparative examples 1 to 4: a preparation including netarsudil dimesylate and carteolol hydrochloride

**Table 54. Preparation of a local ophthalmic medicine composition solution for reducing an intra-ocular pressure as follows:**

| No. | Component | Comparative example 1 | Comparative example 2 | Comparative example 3 | Comparative example 4 |
|---|---|---|---|---|---|
| 1. | Netarsudil dimesylate | 14.25 mg | 14.25 mg | 28.5 mg | 28.5 mg |
| 2. | Carteolol hydrochloride | 1000 mg | 2000 mg | 1000 mg | 2000 mg |
| 3. | Boric acid | 50 mg | 50 mg | 50 mg | 50 mg |
| 4. | Mannitol | 3700 mg | 3700 mg | 3700 mg | 3700 mg |
| 5. | Benzalkonium chloride | 5 mg | 5 mg | 5 mg | 5 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 1 to 5.

**Table 55. Contents of various main medicine components in Comparative examples 1 to 4 after being stored at 5°Cfor 7 days**

| No. | Component | Comparative example 1 | Comparative example 2 | Comparative example 3 | Comparative example 4 |
|---|---|---|---|---|---|
| | | Content % | | | |
| 1. | Netarsudil dimesylate | <30 | <30 | <30 | <30 |
| 2. | Carteolol hydrochloride | 101.2 | 100.8 | 100.3 | 100.2 |

According to Comparative examples 1 to 4: the preparation including netarsudil dimesylate and carteolol hydrochloride has incompatibility under the storage condition of 5°C, and netarsudil dimesylate is precipitated.

### Comparative examples 5 to 8: a preparation including netarsudil dimesylate and hydrochloric acid

**Table 56. Preparation of a local ophthalmic medicine composition solution for reducing an intra-ocular pressure as follows:**

| No. | Component | Comparative example 5 | Comparative example 6 | Comparative example 7 | Comparative example 8 |
|---|---|---|---|---|---|
| 1. | Netarsudil dimesylate | 14.25 mg | 14.25 mg | 28.5 mg | 28.5 mg |
| 2. | Hydrochloric acid | 0.094 mL | 0.188 mL | 0.094 mL | 0.188 mL |
| 3. | Boric acid | 50 mg | 50 mg | 50 mg | 50 mg |
| 4. | Mannitol | 3700 mg | 3700 mg | 3700 mg | 3700 mg |
| 5. | Benzalkonium chloride | 5 mg | 5 mg | 5 mg | 5 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 1 to 5.

**Table 57. Contents of various main medicine components in Comparative examples 5 to 8 after being stored at 5°Cfor 24 h**

| No. | Component | Comparative example 5 | Comparative example 6 | Comparative example 7 | Comparative example 8 |
|---|---|---|---|---|---|
| | | Content % | | | |
| 1. | Netarsudil dimesylate | <30 | <30 | <30 | <30 |

According to Comparative examples 5 to 8: the preparation including netarsudil dimesylate and hydrochloric acid has incompatibility under the storage condition of 5°C, and netarsudil dimesylate is precipitated.

### Embodiments 32 to 36: a preparation including netarsudil dimesylate and timolol mesylate

**Table 58. Preparation of a local ophthalmic medicine composition solution for reducing an intra-ocular pressure as follows:**

| No . | Component | Embodimen t 32 | Embodimen t33 | Embodimen t 34 | Embodimen t 35 | Embodimen t 36 |
|---|---|---|---|---|---|---|
| 1. | Netarsudil dimesylate | 14.25 mg | 14.25 mg | 28.5 mg | 28.5 mg | 0.057 g |
| 2. | Timolol mesylate | 325 mg | 650 mg | 325 mg | 650 mg | 1300 mg |
| 3. | Boric acid | 50 mg | 50 mg | 50 mg | 50 mg | 50 mg |
| 4. | Mannitol | 4300 mg | 4300 mg | 4300 mg | 4300 mg | 4300 mg |
| 5. | Benzalkonium chloride | 12 mg | 12 mg | 12 mg | 12 mg | 12 mg |
| 6. | Sodium hydroxide | q.s. | q.s. | q.s. | q.s. | q.s. |
| 7. | Water for injection added by | 100 mL | 100 mL | 100 mL | 100 mL | 100 |

A preparation process may refer to the preparation process in Embodiments 1 to 5.

**Table 59. Contents of various main medicine components in Embodiments 32 to 36 after being stored at 5°Cfor 24 months**

| No. | Component | Embodiment 32 | Embodiment 33 | Embodiment 34 | Embodiment 35 | Embodiment 36 |
|---|---|---|---|---|---|---|
| | | Content % | | | | |
| 1. | Netarsudil dimesylate | 99.8 | 100.7 | 100.3 | 100.2 | 99.8 |
| 2. | Timolol mesylate | 100.3 | 99.2 | 99.4 | 99.3 | 99.9 |

**Table 60. Contents of various main medicine components in commercially available eye drops (Timoptic^{®} and Rhopress^{®}) after being stored at 5°Cfor 24 months**

| No. | Component | Timoptic^{®} | Rhopress^{®} |
|---|---|---|---|
| | | Content % | |
| 1. | Netarsudil dimesylate | - | 99.7 |
| 2. | Timolol maleate | 99.8 | - |

According to Embodiments 32 to 36, the preparation including netarsudil dimesylate and timolol mesylate has good stability after being stored under a storage condition of 5°Cfor 24 months, which meets a requirement for a storage condition of the commercially available eye drops (Timoptic^{®} and Rhopress^{®}) before opening.

**Table 61. Contents of various main medicine components in Embodiments 32 to 36 after being stored at 25°Cfor 6 weeks**

| No. | Component | Embodiment 32 | Embodiment 33 | Embodiment 34 | Embodiment 35 | Embodiment 36 |
|---|---|---|---|---|---|---|
| | | Content % | | | | |
| 1. | Netarsudil dimesylate | 100.8 | 99.9 | 100.4 | 100.1 | 99.2 |
| 2. | Timolol mesylate | 100.2 | 100.4 | 100.2 | 99.9 | 100.4 |

**Table 62. Contents of various main medicine components in commercially available eye drops (Timoptic^{®} and Rhopress^{®}) after being stored at 25°Cfor 6 weeks**

| No. | Component | Timoptic^{®} | Rhopress^{®} |
|---|---|---|---|
| | | Content % | |
| 1. | Netarsudil dimesylate | - | 99.7 |
| 2. | Timolol maleate | 99.8 | - |

According to Embodiments 32 to 36, the preparation including netarsudil dimesylate and timolol mesylate has good stability after being stored under a storage condition of 25°Cfor 6 weeks, which meets a requirement for a storage condition of the commercially available eye drops (Timoptic^{®} and Rhopress^{®}) after opening.

**Table 63. Contents of various main medicine components in Embodiments 32 to 36 after being stored at 40°Cfor 14 days**

| No. | Component | Embodiment 32 | Embodiment 33 | Embodiment 34 | Embodiment 35 | Embodiment 36 |
|---|---|---|---|---|---|---|
| | | | | Content % | | |
| 1. | Netarsudil dimesylate | 99.2 | 100.4 | 100.6 | 100.6 | 100.2 |
| 2. | Timolol mesylate | 99.1 | 100.2 | 100.5 | 99.4 | 99.6 |

**Table 64. Contents of various main medicine components in commercially available eye drops (Timoptic^{®} and Rhopress^{®}) after being stored at 40°Cfor 14 days**

| No. | Component | Timoptic^{®} | Rhopress^{®} |
|---|---|---|---|
| | | Content % | |
| 1. | Netarsudil dimesylate | - | 99.7 |
| 2. | Timolol maleate | 99.8 | - |

According to Embodiments 32 to 36, the preparation including netarsudil dimesylate and timolol mesylate has good stability after being stored under a storage condition of 40°Cfor 14 days, which meets a requirement for departing from a storage condition of the commercially available eye drops (Timoptic^{®} and Rhopress^{®}) for a short time.

### Embodiments 37 to 41: a preparation including netarsudil maleate and timolol maleate

**Table 65. Preparation of a local ophthalmic medicine composition solution for reducing an intra-ocular pressure as follows:**

| No. | Component | Embodiment 37 | Embodiment 38 | Embodiment 39 | Embodiment 40 | Embodiment 41 |
|---|---|---|---|---|---|---|
| 1. | Netarsudil maleate | 12.6 mg | 12.6 mg | 25.1 mg | 25.1 mg | 50.2 mg |
| 2. | Timolol maleate | 341.7 mg | 683.5 mg | 341.7 mg | 683.5 mg | 1367 mg |
| 3. | Boric acid | 50 mg | 50 mg | 50 mg | 50 mg | 50 mg |
| 4. | Mannitol | 4300 mg | 4300 mg | 4300 mg | 4300 mg | 4300 mg |
| 5. | Benzalkonium chloride | 12 mg | 12 mg | 12 mg | 12 mg | 12 mg |
| 6. | Sodium hydroxide | q.s. | q.s. | q.s. | q.s. | q.s. |
| 7. | Water for injection added by | 100 mL | 100 mL | 100 mL | 100 mL | 100 |

A preparation process may refer to the preparation process in Embodiments 1 to 5.

**Table 66. Contents of various main medicine components in Embodiments 6 to 10 after being stored at 5°Cfor 24 months**

| No. | Component | Embodiment 37 | Embodiment 38 | Embodiment 39 | Embodiment 40 | Embodiment 41 |
|---|---|---|---|---|---|---|
| | | Content % | | | | |
| 1. | Netarsudil maleate | 100.2 | 100.2 | 100.1 | 99.8 | 100.2 |
| 2. | Timolol maleate | 100.3 | 100.3 | 99.7 | 100.2 | 100.1 |

According to Embodiments 37 to 41, the preparation including netarsudil maleate and timolol maleate has good stability after being stored under a storage condition of 5°Cfor 24 months, which meets a requirement for a storage condition of the commercially available eye drops (Timoptic^{®} and Rhopress^{®}) before opening.

**Table 67. Contents of various main medicine components in Embodiments 37 to 41 after being stored at 25°Cfor 6 weeks**

| No. | Component | Embodiment 37 | Embodiment 38 | Embodiment 39 | Embodiment 40 | Embodiment 41 |
|---|---|---|---|---|---|---|
| | | | | Content % | | |
| 1. | Netarsudil maleate | 99.1 | 100.2 | 100.5 | 99.6 | 100.7 |
| 2. | Timolol maleate | 99.2 | 99.6 | 100.4 | 100.1 | 100.8 |

According to Embodiments 37 to 41, the preparation including netarsudil maleate and timolol maleate has good stability after being stored under a storage condition of 25°Cfor 6 weeks, which meets a requirement for a storage condition of the commercially available eye drops (Timoptic^{®} and Rhopress^{®}) after opening.

**Table 68. Contents of various main medicine components in Embodiments 37 to 41 after being stored at 40°Cfor 14 days**

| No. | Component | Embodiment 37 | Embodiment 38 | Embodiment 39 | Embodiment 40 | Embodiment 41 |
|---|---|---|---|---|---|---|
| | | Content % | | | | |
| 1. | Netarsudil maleate | 100.2 | 99.9 | 100.4 | 100.6 | 99.8 |
| 2. | Timolol maleate | 100.1 | 99.8 | 100.2 | 99.7 | 100.2 |

According to Embodiments 37 to 41, the preparation including netarsudil maleate and timolol maleate has good stability after being stored under a storage condition of 40°Cfor 14 days, which meets a requirement for departing from a storage condition of the commercially available eye drops (Timoptic^{®} and Rhopress^{®}) for a short time.

### Embodiment 42: evaluation of pharmaceutical effects of the ophthalmic medicine composition solution in Embodiments 32 to 41 by using a Japanese white rabbit glaucoma model

### Experimental grouping:

Normal control group: no processing.

Model group: an intraocular hypertension model is established, and no eye drop is administered.

Treatment group 1: an intraocular hypertension model is established, and commercially available netarsudil eye drops (Rhopress^{®}) are administered.

Treatment group 3: an intraocular hypertension model is established, and commercially available timolol eye drops (Timoptic^{®}) are administered.

Embodiment group 32: an intraocular hypertension model is established, and an ophthalmic medicine composition solution in Embodiment 32 is administered.

Embodiment group 33: an intraocular hypertension model is established, and an ophthalmic medicine composition solution in Embodiment 33 is administered.

Embodiment group 34: an intraocular hypertension model is established, and an ophthalmic medicine composition solution in Embodiment 34 is administered.

Embodiment group 35: an intraocular hypertension model is established, and an ophthalmic medicine composition solution in Embodiment 35 is administered.

Embodiment group 36: an intraocular hypertension model is established, and an ophthalmic medicine composition solution in Embodiment 36 is administered.

Embodiment group 37: an intraocular hypertension model is established, and an ophthalmic medicine composition solution in Embodiment 37 is administered.

Embodiment group 38: an intraocular hypertension model is established, and an ophthalmic medicine composition solution in Embodiment 38 is administered.

Embodiment group 39: an intraocular hypertension model is established, and an ophthalmic medicine composition solution in Embodiment 39 is administered.

Embodiment group 40: an intraocular hypertension model is established, and an ophthalmic medicine composition solution in Embodiment 40 is administered.

Embodiment group 41: an intraocular hypertension model is established, and an ophthalmic medicine composition solution in Embodiment 41 is administered.

A dosage regimen: according to a current clinical dosage regimen of commercially available netarsudil eye drops (Rhopress^{®}) and commercially available timolol eye drops (Timoptic^{®}), this experiment is mainly divided into the following groups: (1) the treatment group 1: 50 µL the commercially available netarsudil eye drops (Rhopress^{®}) are administered to each eye each time once a day (9:00 PM); (2) the treatment group 3: 50 µL the commercially available timolol eye drops (Timoptic^{®}) are administered to each eye each time twice a day (9:00 AM and 9:00 PM); and (3) Embodiment groups 32 to 41: 50 µL eye drops in Embodiments 32 to 41 are administered to each eye each time once a day (9:00 PM). Drug administration is started in all the groups on the tenth day when the models are established successfully and lasts for 10 days. No processing is made to the normal control group and the model group. The dosage regimen is shown in FIG. 3.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 1, the treatment group 3 and Embodiment groups 32, 33, 34, 37, 38 and 39, the IOP can be reduced significantly by the ophthalmic composition solutions of Embodiment groups 35, 36, 40 and 41, however, IOP actual measured values and IOP variation values in Embodiment groups 35, 36, 40 and 41 have no significant difference.

**Table 69. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| | IOP variation value (mmHg) | | | | | | | | | | | | | |
|---|---|---|---|---|---|---|---|---|---|---|---|---|---|---|
| Ti m e (d ) | No rm al co ntr ol gro up | M od el gr ou p | Trea tme nt grou p 1 | Trea tme nt grou p 3 | Embo dime nt group 32 | Embo dime nt group 33 | Embo dime nt group 34 | Embo dime nt group 35 | Embo dime nt group 36 | Embo dime nt group 37 | Embo dime nt group 38 | Embo dime nt group 39 | Embo dime nt group 40 | Embo dime nt group 41 |
| 1 0 d | - | | | | | | | | | | | | | |
| 1 5 d | 0.5 0 | -1. 88 | -3.0 9 | -1.3 9 | -3.44 | -5.03 | -4.03 | -5.80 | -8.03 | -4.70 | -5.42 | -5.34 | -5.38 | -8.00 |
| 2 0 d | -0. 50 | 2. 10 | -6.4 3 | -5.9 8 | -9.44 | -10.5 0 | -10.3 1 | -12.8 2 | -13.9 8 | -10.2 0 | -10.7 2 | -10.3 4 | -13.0 0 | -13.5 8 |

**Table 70. Intra-ocular pressure actual measured values of each group of rabbits measured on the tenth day, the fifteenth day and the twentieth day when drug administration is performed for 10 days on end starting with the tenth day**

| Ti m e (d ) | IOP actual measured value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 1 | Trea tme nt grou p 3 | Embo dime nt group 32 | Embo dime nt group 33 | Embo dime nt group 34 | Embo dime nt group 35 | Embo dime nt group 36 | Embo dime nt group 37 | Embo dime nt group 38 | Embo dime nt group 39 | Embo dime nt group 40 | Embo dime nt group 41 |
| 1 0 d | 6.0 0 | 26 .2 3 | 25.3 7 | 25.5 0 | 25.77 | 27.63 | 26.63 | 26.63 | 27.63 | 27.33 | 26.93 | 26.67 | 26.33 | 27.33 |
| 1 5 d | 6.5 0 | 24 .3 5 | 22.2 8 | 24.1 1 | 22.33 | 22.60 | 22.60 | 20.83 | 19.60 | 22.63 | 21.51 | 21.33 | 20.95 | 19.33 |
| 2 0 d | 5.5 0 | 28 .3 3 | 18.9 4 | 19.5 2 | 16.33 | 17.13 | 16.32 | 13.81 | 13.65 | 17.13 | 16.21 | 16.33 | 13.33 | 13.75 |

### Embodiment 43: a preparation including netarsudil dimesylate and timolol hydrobromide

**Table 71. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol hydrobromide | 628 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 72. Contents of various main medicine components in Embodiment 43 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.3 | 100.1 |
| 2. | Timolol hydrobromide | 100.2 | 100.6 |

According to Embodiment 43, the preparation including netarsudil dimesylate and timolol hydrobromide has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 44: a preparation including netarsudil dimesylate and timolol sulfate

**Table 73. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol sulfate | 655 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 74. Contents of various main medicine components in Embodiment 44 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.3 | 100.1 |
| 2. | Timolol sulfate | 100.2 | 99.1 |

According to Embodiment 44, the preparation including netarsudil dimesylate and timolol sulfate and having pH in a range from 4.5 to 5.4 has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 45: a preparation including netarsudil dimesylate and timolol esilate

**Table 75. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol esilate | 674 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 76. Contents of various main medicine components in Embodiment 45 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.8 | 100.1 |
| 2. | Timolol esilate | 100.2 | 100.4 |

According to Embodiment 45, the preparation including netarsudil dimesylate and timolol esilate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 46: a preparation including netarsudil dimesylate and timolol nitrate

**Table 77. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol nitrate | 600 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 78. Contents of various main medicine components in Embodiment 46 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.2 | 100.2 |
| 2. | Timolol nitrate | 100.4 | 100.8 |

According to Embodiment 46, the preparation including netarsudil dimesylate and timolol nitrate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 47: a preparation including netarsudil dimesylate and timolol citrate

**Table 79. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol citrate | 804 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 80. Contents of various main medicine components in Embodiment 47 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.2 | 100.2 |
| 2. | Timolol citrate | 100.3 | 99.3 |

According to Embodiment 47, the preparation including netarsudil dimesylate and timolol citrate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 48: a preparation including netarsudil dimesylate and timolol tartrate

**Table 81. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol tartrate | 737 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 82. Contents of various main medicine components in Embodiment 48 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.6 | 100.3 |
| 2. | Timolol tartrate | 100.3 | 99.2 |

According to Embodiment 48, the preparation including netarsudil dimesylate and timolol tartrate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 49: a preparation including netarsudil dimesylate and timolol salicylate

**Table 83. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol salicylate | 718 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 84. Contents of various main medicine components in Embodiment 49 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.5 | 100.5 |
| 2. | Timolol salicylate | 99.6 | 99.3 |

According to Embodiment 49, the preparation including netarsudil dimesylate and timolol salicylate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 50: a preparation including netarsudil dimesylate and timolol malate

**Table 85. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol malate | 712 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 86. Contents of various main medicine components in Embodiment 50 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.3 | 99.1 |
| 2. | Timolol malate | 100.6 | 100.2 |

According to Embodiment 50, the preparation including netarsudil dimesylate and timolol malate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 51: a preparation including netarsudil dimesylate and timolol lactate

**Table 87. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol lactate | 642 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 88. Contents of various main medicine components in Embodiment 51 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.8 | 100.9 |
| 2. | Timolol lactate | 99.6 | 100.2 |

According to Embodiment 51, the preparation including netarsudil dimesylate and timolol lactate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 52: a preparation including netarsudil dimesylate and timolol phenylacetate

**Table 89. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol phenylacetate | 715 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 90. Contents of various main medicine components in Embodiment 52 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.3 | 100.9 |
| 2. | Timolol phenylacetate | 100.2 | 100.3 |

According to Embodiment 52, the preparation including netarsudil dimesylate and timolol phenylacetate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 53: a preparation including netarsudil dihydrobromide and timolol maleate

**Table 91. Preparation of a local ophthalmic medicine composition solution for reducing an intra-ocular pressure as follows:**

| No. | Component | Weight |
|---|---|---|
| 1. | Netarsudil dihydrobromide | 27.1 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 92. Contents of various main medicine components in Embodiment 53 after being stored at 5°Cfor 24 months and at 25°Cfor 6 weeks**

| No. | Component | 5°C | 25°C |
|---|---|---|---|
| | | Content % | |
| 1. | Netarsudil dihydrobromide | 99.4 | 100.3 |
| 2. | Timolol maleate | 99.7 | 100.9 |

According to Embodiment 53, the preparation including netarsudil dihydrobromide and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 54: a preparation including netarsudil dihydrochloride and timolol maleate

**Table 93. Preparation of a local ophthalmic medicine composition solution for reducing an intra-ocular pressure as follows:**

| No. | Component | Weight |
|---|---|---|
| 1. | Netarsudil dihydrochloride | 23.3 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 94. Contents of various main medicine components in Embodiment 54 after being stored at 5°Cfor 24 months and at 25°Cfor 6 weeks**

| No. | Component | 5°C | 25°C |
|---|---|---|---|
| | | Content % | |
| 1. | Netarsudil dihydrochloride | 99.5 | 100.3 |
| 2. | Timolol maleate | 100.5 | 99.6 |

According to Embodiment 54, the preparation including netarsudil dihydrochloride and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 55: a preparation including netarsudil sulfate and timolol maleate

**Table 95. Preparation of a local ophthalmic medicine composition solution for reducing an intra-ocular pressure as follows:**

| No. | Component | Weight |
|---|---|---|
| 1. | Netarsudil sulfate | 24.3 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 96. Contents of various main medicine components in Embodiment 55 after being stored at 5°Cfor 24 months and at 25°Cfor 6 weeks**

| No. | Component | 5°C | 25°C |
|---|---|---|---|
| | | Content % | |
| 1. | Netarsudil sulfate | 100.5 | 100.2 |
| 2. | Timolol maleate | 100.3 | 100.6 |

According to Embodiment 55, the preparation including netarsudil sulfate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 56: a preparation including netarsudil diformate and timolol maleate

**Table 97. Preparation of a local ophthalmic medicine composition solution for reducing an intra-ocular pressure as follows:**

| No. | Component | Weight |
|---|---|---|
| 1. | Netarsudil diformate | 24.1 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 98. Contents of various main medicine components in Embodiment 56 after being stored at 5°Cfor 24 months and at 25°Cfor 6 weeks**

| No. | Component | 5°C | 25°C |
|---|---|---|---|
| | | Content % | |
| 1. | Netarsudil diformate | 100.3 | 100.5 |
| 2. | Timolol maleate | 99.6 | 99.7 |

According to Embodiment 56, the preparation including netarsudil diformate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 57: a preparation including netarsudil dinitrate and timolol maleate

**Table 99. Preparation of a local ophthalmic medicine composition solution for reducing an intra-ocular pressure as follows:**

| No. | Component | Weight |
|---|---|---|
| 1. | Netarsudil dinitrate | 25.6 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 100. Contents of various main medicine components in Embodiment 57 after being stored at 5°Cfor 24 months and at 25°Cfor 6 weeks**

| No. | Component | 5°C | 25°C |
|---|---|---|---|
| | | Content % | |
| 1. | Netarsudil dinitrate | 99.3 | 100.8 |
| 2. | Timolol maleate | 100.2 | 100.5 |

According to Embodiment 57, the preparation including netarsudil dinitrate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 58: a preparation including netarsudil diacetate and timolol maleate

**Table 101. Preparation of a local ophthalmic medicine composition solution for reducing an intra-ocular pressure as follows:**

| No. | Component | Weight |
|---|---|---|
| 1. | Netarsudil diacetate | 27.8 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 102. Contents of various main medicine components in Embodiment 58 after being stored at 5°Cfor 24 months and at 25°Cfor 6 weeks**

| No. | Component | 5°C | 25°C |
|---|---|---|---|
| | | Content % | |
| 1. | Netarsudil diacetate | 100.2 | 100.6 |
| 2. | Timolol maleate | 100.1 | 99.1 |

According to Embodiment 58, the preparation including netarsudil diacetate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 59: a preparation including netarsudil dibenzoate and timolol maleate

**Table 103. Preparation of a local ophthalmic medicine composition solution for reducing an intra-ocular pressure as follows:**

| No. | Component | Weight |
|---|---|---|
| 1. | Netarsudil dibenzoate | 30.8 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 104. Contents of various main medicine components in Embodiment 59 after being stored at 5°Cfor 24 months and at 25°Cfor 6 weeks**

| No. | Component | 5°C | 25°C |
|---|---|---|---|
| | | Content % | |
| 1. | Netarsudil dibenzoate | 99.5 | 99.3 |
| 2. | Timolol maleate | 100.3 | 100.8 |

According to Embodiment 59, the preparation including netarsudil dibenzoate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 60: a preparation including netarsudil oxalate and timolol maleate

**Table 105. Preparation of a local ophthalmic medicine composition solution for reducing an intra-ocular pressure as follows:**

| No. | Component | Weight |
|---|---|---|
| 1. | Netarsudil oxalate | 27.9 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 106. Contents of various main medicine components in Embodiment 60 after being stored at 5°Cfor 24 months and at 25°Cfor 6 weeks**

| No. | Component | 5°C | 25°C |
|---|---|---|---|
| | | Content % | |
| 1. | Netarsudil oxalate | 99.4 | 100.7 |
| 2. | Timolol maleate | 99.6 | 100.8 |

According to Embodiment 60, the preparation including netarsudil oxalate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 61: a preparation including netarsudil succinate and timolol maleate

**Table 107. Preparation of a local ophthalmic medicine composition solution for reducing an intra-ocular pressure as follows:**

| No. | Component | Weight |
|---|---|---|
| 1. | Netarsudil succinate | 25.2 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 108. Contents of various main medicine components in Embodiment 61 after being stored at 5°Cfor 24 months and at 25°Cfor 6 weeks**

| No. | Component | 5°C | 25°C |
|---|---|---|---|
| | | Content % | |
| 1. | Netarsudil succinate | 100.3 | 99.2 |
| 2. | Timolol maleate | 100.6 | 100.8 |

According to Embodiment 61, the preparation including netarsudil succinate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Embodiment 62: a preparation including netarsudil diphenylacetate and timolol maleate

**Table 109. Preparation of a local ophthalmic medicine composition solution for reducing an intra-ocular pressure as follows:**

| No. | Component | Weight |
|---|---|---|
| 1. | Netarsudil diphenylacetate | 32 mg |
| 2. | Timolol maleate | 683.5 mg |
| 3. | Boric acid | 50 mg |
| 4. | Mannitol | 4300 mg |
| 5. | Benzalkonium chloride | 12 mg |
| 6. | Sodium hydroxide | q.s. |
| 7. | Water for injection added by | 100 |

A preparation process may refer to the preparation process in Embodiments 1 to 5.

**Table 110. Contents of various main medicine components in Embodiment 62 after being stored at 5°Cfor 24 months and at 25°Cfor 6 weeks**

| No. | Component | 5°C | 25°C |
|---|---|---|---|
| | | Content % | |
| 1. | Netarsudil diphenylacetate | 99.1 | 100.6 |
| 2. | Timolol maleate | 99.3 | 100.5 |

According to Embodiment 62, the preparation including netarsudil diphenylacetate and timolol maleate has good stability after being stored under a storage condition of 5°Cfor 24 months, which meets a requirement for a storage condition of the commercially available eye drops (Timoptic^{®} and Rhopress^{®}) before opening; and has good stability after being stored under a storage condition of 25°Cfor 6 weeks, which meets a requirement for a storage condition of the commercially available eye drops (Timoptic^{®} and Rhopress^{®}) after opening.

### Comparative examples 9 to 12: a preparation including netarsudil dimesylate and timolol maleate

**Table 111. Preparation of a local ophthalmic medicine composition solution for reducing an intra-ocular pressure as follows:**

| No. | Component | Comparative example 9 | Comparative example 10 | Comparative example 11 | Comparative example 12 |
|---|---|---|---|---|---|
| 1. | Netarsudil dimesylate | 14.25 mg | 14.25 mg | 28.5 mg | 28.5 mg |
| 2. | Timolol maleate | 341.7 mg | 683.5 mg | 341.7 mg | 683.5 mg |
| 3. | Boric acid | 50 mg | 50 mg | 50 mg | 50 mg |
| 4. | Mannitol | 4300 mg | 4300 mg | 4300 mg | 4300 mg |
| 5. | Benzalkonium chloride | 12 mg | 12 mg | 12 mg | 12 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 1 to 5.

**Table 112. Contents of various main medicine components in Comparative examples 9 to 12 after being stored at 5°Cfor 7 days**

| No. | Component | Comparative example 9 | Comparative example 10 | Comparative example 11 | Comparative example 12 |
|---|---|---|---|---|---|
| | | Content % | | | |
| 1. | Netarsudil dimesylate | <30 | <30 | <30 | <30 |
| 2. | Timolol maleate | 99.3 | 99.5 | 100.3 | 100.1 |

According to Comparative examples 9 to 12, the preparation including netarsudil dimesylate and timolol maleate has incompatibility under the storage condition of 5°C, and netarsudil dimesylate is precipitated.

### Comparative examples 13 to 16: a preparation including netarsudil dimesylate and maleic acid

**Table 113. Preparation of a local ophthalmic medicine composition solution for reducing an intra-ocular pressure as follows:**

| No. | Component | Comparative example 13 | Comparative example 14 | Comparative example 15 | Comparative example 16 |
|---|---|---|---|---|---|
| 1. | Netarsudil dimesylate | 14.25 mg | 14.25 mg | 28.5 mg | 28.5 mg |
| 2. | Maleic acid | 91.75 mg | 183.5 mg | 91.75 mg | 183.5 mg |
| 3. | Boric acid | 50 mg | 50 mg | 50 mg | 50 mg |
| 4. | Mannitol | 4300 mg | 4300 mg | 4300 mg | 4300 mg |
| 5. | Benzalkonium chloride | 12 mg | 12 mg | 12 mg | 12 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 1 to 5.

**Table 114. Contents of various main medicine components in Comparative examples 13 to 16 after being stored at 5°Cfor 7 days**

| No. | Component | Comparative example 13 | Comparative example 14 | Comparative example 15 | Comparative example 16 |
|---|---|---|---|---|---|
| | | Content % | | | |
| 1. | Netarsudil dimesylate | <30 | <30 | <30 | <30 |

According to Comparative examples 13 to 16, the preparation including netarsudil dimesylate and maleic acid has incompatibility under the storage condition of 5°C, and netarsudil dimesylate is precipitated.

### Embodiments 63 to 67: a preparation including netarsudil dimesylate and betaxolol mesylate

**Table 115. Preparation of a local ophthalmic medicine composition solution for reducing an intra-ocular pressure as follows:**

| No. | Component | Embodiment 63 | Embodiment 64 | Embodiment 65 | Embodiment 66 | Embodiment 67 |
|---|---|---|---|---|---|---|
| 1. | Netarsudil dimesylate | 14.2 mg | 14.2 mg | 28.5 mg | 28.5 mg | 57.0 mg |
| 2. | Betaxolol mesylate | 164.1 mg | 328.2 mg | 164.1 mg | 328.2 mg | 656.3 mg |
| 3. | Boric acid | 50 mg | 50 mg | 50 mg | 50 mg | 50 mg |
| 4. | Mannitol | 4400 mg | 4400 mg | 4400 mg | 4400 mg | 4400 mg |
| 5. | Benzalkonium chloride | 10 mg | 10 mg | 10 mg | 10 mg | 10 mg |
| 6. | Sodium hydroxide | q.s. | q.s. | q.s. | q.s. | q.s. |
| 7. | Water for injection added by | 100 ml | 100 ml | 100 ml | 100 ml | 100 |

A preparation process may refer to the preparation process in Embodiments 1 to 5.

**Table 116. Contents of various main medicine components in Embodiments 63 to 67 after being stored at 5°Cfor 24 months**

| No. | Component | Embodiment 63 | Embodiment 64 | Embodiment 65 | Embodiment 66 | Embodiment 67 |
|---|---|---|---|---|---|---|
| | | Content % | | | | |
| 1. | Netarsudil dimesylate | 99.8 | 99.9 | 100.2 | 100.5 | 101.1 |
| 2. | Betaxolol mesylate | 99.6 | 100.2 | 99.8 | 100.1 | 100.3 |

**Table 117. Contents of various main medicine components in commercially available eye drops (Betoptic^{®} and Rhopress^{®}) after being stored at 5°Cfor 24 months**

| No. | Component | Betoptic^{®} | Rhopress^{®} |
|---|---|---|---|
| | | Content % | |
| 1. | Netarsudil dimesylate | - | 100.4 |
| 2. | Betaxolol hydrochloride | 99.5 | - |

According to Embodiments 63 to 67, the preparation including netarsudil dimesylate and betaxolol mesylate has good stability after being stored under a storage condition of 5°Cfor 24 months, which meets a requirement for a storage condition of the commercially available eye drops (Betoptic^{®} and Rhopress^{®}) before opening.

**Table 118. Contents of various main medicine components in Embodiments 63 to 67 after being stored at 25°Cfor 6 weeks**

| No. | Component | Embodiment 63 | Embodiment 64 | Embodiment 65 | Embodiment 66 | Embodiment 67 |
|---|---|---|---|---|---|---|
| | | Content % | | | | |
| 1. | Netarsudil dimesylate | 99.7 | 99.4 | 100.5 | 100.2 | 100.2 |
| 2. | Betaxolol mesylate | 98.5 | 99.1 | 99.6 | 99.4 | 99.9 |

**Table 119. Contents of various main medicine components in commercially available eye drops (Betoptic^{®} and Rhopress^{®}) after being stored at 25°Cfor 6 weeks**

| No. | Component | Betoptic^{®} | Rhopress^{®} |
|---|---|---|---|
| | | Content % | |
| 1. | Netarsudil dimesylate | - | 99.9 |
| 2. | Betaxolol hydrochloride | 99.9 | - |

According to Embodiments 63 to 67, the preparation including netarsudil dimesylate and betaxolol mesylate has good stability after being stored under a storage condition of 25°Cfor 6 weeks, which meets a requirement for a storage condition of the commercially available eye drops (Betoptic^{®} and Rhopress^{®}) after opening.

**Table 120. Contents of various main medicine components in Embodiments 63 to 67 after being stored at 40°Cfor 14 days**

| No. | Component | Embodiment 63 | Embodiment 64 | Embodiment 65 | Embodiment 66 | Embodiment 67 |
|---|---|---|---|---|---|---|
| | | Content % | | | | |
| 1. | Netarsudil dimesylate | 99.6 | 99.0 | 100.0 | 99.8 | 99.8 |
| 2. | Betaxolol mesylate | 99.8 | 100.7 | 100.4 | 99.6 | 99.4 |

**Table 121. Contents of various main medicine components in commercially available eye drops (Betoptic^{®} and Rhopress^{®}) after being stored at 40°Cfor 14 days**

| No. | Component | Betoptic^{®} | Rhopress^{®} |
|---|---|---|---|
| | | Content % | |
| 1. | Netarsudil dimesylate | - | 101.3 |
| 2. | Betaxolol hydrochloride | 100.9 | - |

According to Embodiments 63 to 67, the preparation including netarsudil dimesylate and betaxolol mesylate has good stability after being stored under a storage condition of 40°Cfor 14 days, which meets a requirement for departing from a storage condition of the commercially available eye drops (Betoptic^{®} and Rhopress^{®}) for a short time.

### Embodiments 68 to 72: a preparation including netarsudil dihydrochloride and betaxolol hydrochloride

**Table 122. Preparation of a local ophthalmic medicine composition solution for reducing an intra-ocular pressure as follows:**

| No. | Component | Embodiment 68 | Embodiment 69 | Embodiment 70 | Embodiment 71 | Embodiment 72 |
|---|---|---|---|---|---|---|
| 1. | Netarsudil dihydrochloride | 11.6 mg | 11.6 mg | 23.3 mg | 23.3 mg | 46.6 mg |
| 2. | Betaxolol hydrochloride | 139.8 mg | 279.6 mg | 139.8 mg | 279.6 mg | 559.3 mg |
| 3. | Boric acid | 50 mg | 50 mg | 50 mg | 50 mg | 50 mg |
| 4. | Mannitol | 4400 mg | 4400 mg | 4400 mg | 4400 mg | 4400 mg |
| 5. | Benzalkonium chloride | 10 mg | 10 mg | 10 mg | 10 mg | 10 mg |
| 6. | Sodium hydroxide | q.s. | q.s. | q.s. | q.s. | q.s. |
| 7. | Water for injection added by | 100 mL | 100 mL | 100 mL | 100 mL | 100 |

A preparation process may refer to the preparation process in Embodiments 1 to 5.

**Table 123. Contents of various main medicine components in Embodiments 68 to 72 after being stored at 5°Cfor 24 months**

| No. | Component | Embodiment 68 | Embodiment 69 | Embodiment 70 | Embodiment 71 | Embodiment 72 |
|---|---|---|---|---|---|---|
| | | Content % | | | | |
| 1. | Netarsudil dihydrochloride | 100.5 | 100.1 | 99.8 | 99.5 | 100.3 |
| 2. | Betaxolol hydrochloride | 98.9 | 99.5 | 99.3 | 99.9 | 100.1 |

According to Embodiments 68 to 72, the preparation including netarsudil dihydrochloride and betaxolol hydrochloride has good stability after being stored under a storage condition of 5°Cfor 24 months, which meets a requirement for a storage condition of the commercially available eye drops (Betoptic^{®} and Rhopress^{®}) before opening.

**Table 124. Contents of various main medicine components in Embodiments 68 to 72 after being stored at 25°Cfor 6 weeks**

| No. | Component | Embodiment 68 | Embodiment 69 | Embodiment 70 | Embodiment 71 | Embodiment 72 |
|---|---|---|---|---|---|---|
| | | Content % | | | | |
| 1. | Netarsudil dihydrochloride | 99.4 | 99.7 | 100.5 | 100.2 | 99.9 |
| 2. | Betaxolol hydrochloride | 100.2 | 99.1 | 100.5 | 98.7 | 99.5 |

According to Embodiments 68 to 72, the preparation including netarsudil dihydrochloride and betaxolol hydrochloride has good stability after being stored under a storage condition of 25°Cfor 6 weeks, which meets a requirement for a storage condition of the commercially available eye drops (Betoptic^{®} and Rhopress^{®}) after opening.

**Table 125. Contents of various main medicine components in Embodiments 68 to 72 after being stored at 40°Cfor 14 days**

| No. | Component | Embodiment 68 | Embodiment 69 | Embodiment 70 | Embodiment 71 | Embodiment 72 |
|---|---|---|---|---|---|---|
| | | Content % | | | | |
| 1. | Netarsudil dihydrochloride | 99.8 | 99.4 | 101.2 | 100.7 | 100.3 |
| 2. | Betaxolol hydrochloride | 100.5 | 100.6 | 99.8 | 100.1 | 99.9 |

According to Embodiments 68 to 72, the preparation including netarsudil dihydrochloride and betaxolol hydrochloride has good stability after being stored under a storage condition of 40°Cfor 14 days, which meets a requirement for departing from a storage condition of the commercially available eye drops (Betoptic^{®} and Rhopress^{®}) for a short time.

### Comparative examples 17 to 20: a preparation including netarsudil dimesylate and betaxolol hydrochloride

**Table 126. Preparation of a local ophthalmic medicine composition solution for reducing an intra-ocular pressure as follows:**

| No. | Component | Comparative example 17 | Comparative example 18 | Comparative example 19 | Comparative example 20 |
|---|---|---|---|---|---|
| 1. | Netarsudil dimesylate | 14.25 mg | 14.25 mg | 28.5 mg | 28.5 mg |
| 2. | Betaxolol hydrochloride | 139.8 mg | 279.6 mg | 139.8 mg | 279.6 mg |
| 3. | Boric acid | 50 mg | 50 mg | 50 mg | 50 mg |
| 4. | Mannitol | 4400 mg | 4400 mg | 4400 mg | 4400 mg |
| 5. | Benzalkonium chloride | 10 mg | 10 mg | 10 mg | 10 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 1 to 5.

**Table 127. Contents of various main medicine components in Comparative examples 17 to 20 after being stored at 5°Cfor 7 days**

| No. | Component | Comparative example 17 | Comparative example 18 | Comparative example 19 | Comparative example 20 |
|---|---|---|---|---|---|
| | | Content % | | | |
| 1. | Netarsudil dimesylate | <30 | <30 | <30 | <30 |
| 2. | Betaxolol hydrochloride | 99.9 | 100.2 | 99.8 | 99.8 |

According to Comparative examples 17 to 20, the preparation including netarsudil dimesylate and carteolol hydrochloride has incompatibility under the storage condition of 5°C, and netarsudil dimesylate is precipitated.

### Embodiments 73 to 77: a preparation including netarsudil dimesylate and metipranolol mesylate

**Table 128. Preparation of a local ophthalmic medicine composition solution for reducing an intra-ocular pressure as follows:**

| No. | Component | Embodiment 73 | Embodiment 74 | Embodiment 75 | Embodiment 76 | Embodiment 77 |
|---|---|---|---|---|---|---|
| 1. | Netarsudil dimesylate | 14.2 mg | 14.2 mg | 28.5 mg | 28.5 mg | 57.0 mg |
| 2. | Metipranolol mesylate | 131.1 mg | 393.2 mg | 131.1 mg | 393.2 mg | 786.4 mg |
| 3. | Boric acid | 50 mg | 50 mg | 50 mg | 50 mg | 50 mg |
| 4. | Mannitol | 4600 mg | 4600 mg | 4600 mg | 4600 mg | 4600 mg |
| 5. | Benzalkonium chloride | 10 mg | 10 mg | 10 mg | 10 mg | 10 mg |
| 6. | Sodium hydroxide | q.s. | q.s. | q.s. | q.s. | q.s. |
| 7. | Water for injection added by | 100 ml | 100 ml | 100 ml | 100 ml | 100 |

A preparation process may refer to the preparation process in Embodiments 1 to 5.

**Table 129. Contents of various main medicine components in Embodiments 73 to 77 after being stored at 5°Cfor 24 months**

| No. | Component | Embodiment 73 | Embodiment 74 | Embodiment 75 | Embodiment 76 | Embodiment 77 |
|---|---|---|---|---|---|---|
| | | Content % | | | | |
| 1. | Netarsudil dimesylate | 99.8 | 100.3 | 100.4 | 101.1 | 100.2 |
| 2. | Metipranolol mesylate | 99.8 | 99.7 | 99.5 | 99.9 | 100.0 |

**Table 130. Contents of various main medicine components in commercially available eye drops (Optipranolol^{®} and Rhopress^{®}) after being stored at 5°Cfor 24 months**

| No. | Component | Optipranolol^{®} | Rhopress^{®} |
|---|---|---|---|
| | | Content % | |
| 1. | Netarsudil dimesylate | - | 99.9 |
| 2. | Metipranolol hydrochloride | 99.8 | - |

According to Embodiments 73 to 77, the preparation including netarsudil dimesylate and metipranolol mesylate has good stability after being stored under a storage condition of 5°Cfor 24 months, which meets a requirement for a storage condition of the commercially available eye drops (Optipranolol^{®} and Rhopress^{®}) before opening.

**Table 131. Contents of various main medicine components in Embodiments 73 to 77 after being stored at 25°Cfor 6 weeks**

| No. | Component | Embodiment 73 | Embodiment 74 | Embodiment 75 | Embodiment 76 | Embodiment 77 |
|---|---|---|---|---|---|---|
| | | Content % | | | | |
| 1. | Netarsudil dimesylate | 100.2 | 99.8 | 100.3 | 99.6 | 99.9 |
| 2. | Metipranolol mesylate | 99.8 | 99.9 | 99.6 | 99.8 | 100.3 |

**Table 132. Contents of various main medicine components in commercially available eye drops (Optipranolol^{®} and Rhopress^{®}) after being stored at 25°Cfor 6 weeks**

| No. | Component | Optipranolol^{®} | Rhopress^{®} |
|---|---|---|---|
| | | Content % | |
| 1. | Netarsudil dimesylate | - | 99.6 |
| 2. | Metipranolol hydrochloride | 100.4 | - |

According to Embodiments 73 to 77, the preparation including netarsudil dimesylate and metipranolol mesylate has good stability after being stored under a storage condition of 25°Cfor 6 weeks, which meets a requirement for a storage condition of the commercially available eye drops (Optipranolol^{®} and Rhopress^{®}) after opening.

**Table 133. Contents of various main medicine components in Embodiments 73 to 77 after being stored at 40°Cfor 14 days**

| No. | Component | Embodiment 73 | Embodiment 74 | Embodiment 75 | Embodiment 76 | Embodiment 77 |
|---|---|---|---|---|---|---|
| | | Content % | | | | |
| 1. | Netarsudil dimesylate | 99.8 | 100.3 | 99.9 | 99.7 | 99.8 |
| 2. | Metipranolol mesylate | 99.9 | 100.1 | 100.7 | 99.9 | 99.7 |

**Table 134. Contents of various main medicine components in commercially available eye drops (Optipranolol^{®} and Rhopress^{®}) after being stored at 40°Cfor 14 days**

| No. | Component | Optipranolol^{®} | Rhopress^{®} |
|---|---|---|---|
| | | Content % | |
| 1. | Netarsudil dimesylate | - | 99.5 |
| 2. | Metipranolol hydrochloride | 99.8 | - |

According to Embodiments 73 to 77, the preparation including netarsudil dimesylate and metipranolol mesylate has good stability after being stored under a storage condition of 40°Cfor 14 days, which meets a requirement for departing from a storage condition of the commercially available eye drops (Optipranolol^{®} and Rhopress^{®}) for a short time.

### Embodiments 78 to 82: a preparation including netarsudil dihydrochloride and metipranolol hydrochloride

**Table 135. Preparation of a local ophthalmic medicine composition solution for reducing an intra-ocular pressure as follows:**

| No. | Component | Embodiment 78 | Embodiment 79 | Embodiment 80 | Embodiment 81 | Embodiment 82 |
|---|---|---|---|---|---|---|
| 1. | Netarsudil dihydrochloride | 11.6 mg | 11.6 mg | 23.3 mg | 23.3 mg | 46.6 mg |
| 2. | Metipranolol hydrochloride | 111.8 mg | 335.4 mg | 111.8 mg | 335.4 mg | 670.8 mg |
| 3. | Boric acid | 50 mg | 50 mg | 50 mg | 50 mg | 50 mg |
| 4. | Mannitol | 4600 mg | 4600 mg | 4600 mg | 4600 mg | 4600 mg |
| 5. | Benzalkonium chloride | 10 mg | 10 mg | 10 mg | 10 mg | 10 mg |
| 6. | Sodium hydroxide | q.s. | q.s. | q.s. | q.s. | q.s. |
| 7. | Water for injection added by | 100 mL | 100 mL | 100 mL | 100 mL | 100 |

A preparation process may refer to the preparation process in Embodiments 1 to 5.

**Table 136. Contents of various main medicine components in Embodiments 78 to 82 after being stored at 5°Cfor 24 months**

| No. | Component | Embodiment 78 | Embodiment 79 | Embodiment 80 | Embodiment 81 | Embodiment 82 |
|---|---|---|---|---|---|---|
| | | Content % | | | | |
| 1. | Netarsudil dihydrochloride | 100.2 | 100.1 | 99.5 | 99.3 | 99.1 |
| 2. | Metipranolol hydrochloride | 101.3 | 100.8 | 100.3 | 102.3 | 100.2 |

According to Embodiments 78 to 82, the preparation including netarsudil dihydrochloride and metipranolol hydrochloride has good stability after being stored under a storage condition of 5°Cfor 24 months, which meets a requirement for a storage condition of the commercially available eye drops (Optipranolol^{®} and Rhopress^{®}) before opening.

**Table 137. Contents of various main medicine components in Embodiments 78 to 82 after being stored at 25°Cfor 6 weeks**

| No. | Component | Embodiment 78 | Embodiment 79 | Embodiment 80 | Embodiment 81 | Embodiment 82 |
|---|---|---|---|---|---|---|
| | | Content % | | | | |
| 1. | Netarsudil dihydrochloride | 97.9 | 98.4 | 99.9 | 99.1 | 99.0 |
| 2. | Metipranolol hydrochloride | 99.7 | 99.4 | 99.5 | 99.6 | 99.8 |

According to Embodiments 78 to 82, the preparation including netarsudil dihydrochloride and metipranolol hydrochloride has good stability after being stored under a storage condition of 25°Cfor 6 weeks, which meets a requirement for a storage condition of the commercially available eye drops (Optipranolol^{®} and Rhopress^{®}) after opening.

**Table 138. Contents of various main medicine components in Embodiments 78 to 82 after being stored at 40°Cfor 14 days**

| No. | Component | Embodiment 78 | Embodiment 79 | Embodiment 80 | Embodiment 81 | Embodiment 82 |
|---|---|---|---|---|---|---|
| | | Content % | | | | |
| 1. | Netarsudil dihydrochloride | 99.9 | 99.7 | 99.8 | 99.9 | 99.8 |
| 2. | Metipranolol hydrochloride | 100.3 | 99.9 | 98.6 | 99.5 | 99.5 |

According to Embodiments 78 to 82, the preparation including netarsudil dihydrochloride and metipranolol hydrochloride has good stability after being stored under a storage condition of 40°Cfor 14 days, which meets a requirement for departing from a storage condition of the commercially available eye drops (Optipranolol^{®} and Rhopress^{®}) for a short time.

### Comparative examples 21 to 24: a preparation including netarsudil dimesylate and metipranolol hydrochloride

**Table 139. Preparation of a local ophthalmic medicine composition solution for reducing an intra-ocular pressure as follows:**

| No. | Component | Comparative example 21 | Comparative example 22 | Comparative example 23 | Comparative example 24 |
|---|---|---|---|---|---|
| 1. | Netarsudil dimesylate | 14.25 mg | 14.25 mg | 28.5 mg | 28.5 mg |
| 2. | Metipranolol hydrochloride | 111.8 mg | 335.4 mg | 111.8 mg | 335.4 mg |
| 3. | Boric acid | 50 mg | 50 mg | 50 mg | 50 mg |
| 4. | Mannitol | 4600 mg | 4600 mg | 4600 mg | 4600 mg |
| 5. | Benzalkonium chloride | 10 mg | 10 mg | 10 mg | 10 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 1 to 5.

**Table 140. Contents of various main medicine components in Comparative examples 21 to 24 after being stored at 5°Cfor 7 days**

| No. | Component | Comparative example 21 | Comparative example 22 | Comparative example 23 | Comparative example 24 |
|---|---|---|---|---|---|
| | | Content % | | | |
| 1. | Netarsudil dimesylate | <30 | <30 | <30 | <30 |
| 2. | Metipranolol hydrochloride | 101.2 | 100.8 | 100.3 | 100.2 |

According to Comparative examples 21 to 24, the preparation including netarsudil dimesylate and metipranolol hydrochloride has incompatibility under the storage condition of 5°C, and netarsudil dimesylate is precipitated.

### Embodiments 83 to 87: a preparation including netarsudil dimesylate, timolol mesylate and latanoprost

**Table 141. Preparation of a local ophthalmic medicine composition solution for reducing an intra-ocular pressure as follows:**

| No. | Component | Embodiment 83 | Embodiment 84 | Embodiment 85 | Embodiment 86 | Embodiment 87 |
|---|---|---|---|---|---|---|
| 1. | Netarsudil dimesylate | 14.2 mg | 14.2 mg | 28.5 mg | 28.5 mg | 57.0 mg |
| 2. | Timolol mesylate | 325 mg | 650 mg | 325 mg | 650 mg | 1300 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process is as follows: 1) 50% of a prescription dosage of water for injection is weighed, prescription dosages of prostaglandins active components and benzalkonium chloride are added and heating in water bath and stirring are performed to be dissolved completely; 2) 45% of the prescription dosage of water for injection is weighed, prescription dosages of other active components and adjuvants are added, and stirring is performed to be dissolved completely; 3) the solution in step 2 is added into the solution in step 1, stirring is performed to be uniform, and pH is regulated with a sodium hydroxide solution (10%) to a range from 4.5 to 5.4; 4) water for injection is supplemented to a full prescription dosage, stirring is performed to be uniform, and a volume reaches 100%; and 5) the solution in step 4 is put in a low-density polyethylene medicinal eye drop bottle, and its stability is studied under different storage temperature conditions.

**Table 142. Contents of various main medicine components in Embodiments 83 to 87 after being stored at 5°Cfor 24 months**

| No. | Component | Embodiment 83 | Embodiment 84 | Embodiment 85 | Embodiment 86 | Embodiment 87 |
|---|---|---|---|---|---|---|
| | | Content % | | | | |
| 1. | Netarsudil dimesylate | 99.9 | 99.7 | 100.2 | 100.1 | 99.6 |
| 2. | Timolol mesylate | 101.3 | 100.2 | 100.4 | 99.8 | 99.7 |
| 3. | Latanoprost | 100.2 | 99.8 | 99.6 | 99.2 | 100.5 |

**Table 143. Contents of various main medicine components in commercially available eye drops (Timoptic^{®}, Rhopress^{®} and Xalatan^{®}) after being stored at 5°Cfor 24 months**

| No. | Component | Timoptic^{®} | Rhopress^{®} | Xalatan^{®} |
|---|---|---|---|---|
| | | Content % | | |
| 1. | Timolol maleate | 99.7 | - | - |
| 2. | Netarsudil dimesylate | - | 99.9 | - |
| 3. | Latanoprost | - | - | 99.8 |

According to Embodiments 83 to 87, the preparation including netarsudil dimesylate, timolol mesylate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening.

**Table 144. Contents of various main medicine components in Embodiments 83 to 87 after being stored at 25°Cfor 6 weeks**

| No. | Component | Embodiment 83 | Embodiment 84 | Embodiment 85 | Embodiment 86 | Embodiment 87 |
|---|---|---|---|---|---|---|
| | | Content % | | | | |
| 1. | Netarsudil dimesylate | 99.8 | 98.9 | 101.4 | 100.4 | 99.7 |
| 2. | Timolol mesylate | 100.3 | 99.4 | 100.6 | 98.9 | 100.6 |
| 3. | Latanoprost | 100.9 | 100.1 | 101.4 | 99.6 | 100.1 |

**Table 145. Contents of various main medicine components in commercially available eye drops (Timoptic^{®}, Rhopress^{®} and Xalatan^{®}) after being stored at 25°Cfor 6 weeks**

| No. | Component | Timoptic^{®} | Rhopress^{®} | Xalatan^{®} |
|---|---|---|---|---|
| | | Content % | | |
| 1. | Timolol maleate | 99.5 | - | - |
| 2. | Netarsudil dimesylate | - | 99.8 | - |
| 3. | Latanoprost | - | - | 99.6 |

According to Embodiments 83 to 87, the preparation including netarsudil dimesylate, timolol mesylate and latanoprost has good stability after being stored under a storage condition of 25°Cfor 6 weeks, which meets a requirement for a storage condition of the commercially available eye drops (Timoptic^{®}, Rhopress^{®} and Xalatan^{®}) after opening.

**Table 146. Contents of various main medicine components in Embodiments 83 to 87 after being stored at 40°Cfor 14 days**

| No. | Component | Embodiment 83 | Embodiment 84 | Embodiment 85 | Embodiment 86 | Embodiment 87 |
|---|---|---|---|---|---|---|
| | | Content % | | | | |
| 1. | Netarsudil dimesylate | 100.2 | 101.4 | 100.8 | 99.6 | 100.3 |
| 2. | Timolol mesylate | 100.1 | 100.5 | 99.5 | 99.6 | 99.1 |
| 3. | Latanoprost | 99.1 | 99.2 | 100.1 | 100.8 | 100.1 |

**Table 147. Contents of various main medicine components in commercially available eye drops (Timoptic^{®}, Rhopress^{®} and Xalatan^{®}) after being stored at 40°Cfor 14 days**

| No. | Component | Timoptic^{®} | Rhopress^{®} | Xalatan^{®} |
|---|---|---|---|---|
| | | Content % | | |
| 1. | Timolol maleate | 99.8 | - | - |
| 2. | Netarsudil dimesylate | - | 99.5 | - |
| 3. | Latanoprost | - | - | 99.7 |

According to Embodiments 83 to 87, the preparation including netarsudil dimesylate, timolol mesylate and latanoprost has good stability after being stored under a storage condition of 40°Cfor 14 days, which meets a requirement for departing from a storage condition of the commercially available eye drops (Timoptic^{®}, Rhopress^{®} and Xalatan^{®}) for a short time.

### Embodiments 88 to 92: a preparation including netarsudil maleate, timolol maleate and latanoprost

**Table 148. Preparation of a local ophthalmic medicine composition solution for reducing an intra-ocular pressure as follows:**

| No. | Component | Embodiment 88 | Embodiment 89 | Embodiment 90 | Embodiment 91 | Embodiment 92 |
|---|---|---|---|---|---|---|
| 1. | Netarsudil maleate | 12.6 mg | 12.6 mg | 25.1 mg | 25.1 mg | 50.2 mg |
| 2. | Timolol maleate | 341.7 mg | 683.5 mg | 341.7 mg | 683.5 mg | 1367 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 149. Contents of various main medicine components in Embodiments 88 to 92 after being stored at 5°Cfor 24 months**

| No. | Component | Embodiment 88 | Embodiment 89 | Embodiment 90 | Embodiment 91 | Embodiment 92 |
|---|---|---|---|---|---|---|
| | | Content % | | | | |
| 1. | Netarsudil maleate | 100.1 | 100.4 | 100.5 | 99.5 | 99.2 |
| 2. | Timolol maleate | 100.8 | 99.3 | 99.4 | 100.1 | 100.3 |
| 3. | Latanoprost | 101.2 | 100.1 | 99.3 | 99.4 | 100.1 |

According to Embodiments 88 to 92, the preparation including netarsudil maleate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening.

**Table 150. Contents of various main medicine components in Embodiments 88 to 92 after being stored at 25°Cfor 6 weeks**

| No. | Component | Embodiment 88 | Embodiment 89 | Embodiment 90 | Embodiment 91 | Embodiment 92 |
|---|---|---|---|---|---|---|
| | | Content % | | | | |
| 1. | Netarsudil maleate | 100.1 | 100.8 | 99.5 | 99.8 | 100.5 |
| 2. | Timolol maleate | 99.3 | 99.3 | 100.5 | 100.4 | 100.1 |
| 3. | Latanoprost | 99.2 | 100.1 | 100.7 | 99.1 | 98.9 |

According to Embodiments 88 to 92, the preparation including netarsudil maleate, timolol maleate and latanoprost has good stability after being stored under a storage condition of 25°Cfor 6 weeks, which meets a requirement for a storage condition of the commercially available eye drops (Timoptic^{®}, Rhopress^{®} and Xalatan^{®}) after opening.

**Table 151. Contents of various main medicine components in Embodiments 88 to 92 after being stored at 40°Cfor 14 days**

| No. | Component | Embodiment 88 | Embodiment 89 | Embodiment 90 | Embodiment 91 | Embodiment 92 |
|---|---|---|---|---|---|---|
| | | Content % | | | | |
| 1. | Netarsudil maleate | 100.1 | 98.9 | 100.9 | 100.1 | 98.8 |
| 2. | Timolol maleate | 100.9 | 100.8 | 100.5 | 99.9 | 100.1 |
| 3. | Latanoprost | 99.3 | 99.1 | 100.1 | 99.5 | 100.9 |

According to Embodiments 88 to 92, the preparation including netarsudil maleate, timolol maleate and latanoprost has good stability after being stored under a storage condition of 40°Cfor 14 days, which meets a requirement for departing from a storage condition of the commercially available eye drops (Timoptic^{®}, Rhopress^{®} and Xalatan^{®}) for a short time.

### Embodiment 93: evaluation of pharmaceutical effects of the ophthalmic medicine composition solution in Embodiments 83 to 92 by using a a Japanese white rabbit glaucoma model

### Experimental grouping:

Normal control group: no processing.

Model group: an intraocular hypertension model is established, and no eye drop is administered.

Treatment group 4: an intraocular hypertension model is established, and commercially available latanoprost and timolol eye drops (Xalacom^{®}) are administered.

Treatment group 5: an intraocular hypertension model is established, and commercially available latanoprost and netarsudil eye drops (Rocklatan^{®}) are administered.

Embodiment group 83: an intraocular hypertension model is established, and an ophthalmic medicine composition solution in Embodiment 83 is administered.

Embodiment group 84: an intraocular hypertension model is established, and an ophthalmic medicine composition solution in Embodiment 84 is administered.

Embodiment group 85: an intraocular hypertension model is established, and an ophthalmic medicine composition solution in Embodiment 85 is administered.

Embodiment group 86: an intraocular hypertension model is established, and an ophthalmic medicine composition solution in Embodiment 86 is administered.

Embodiment group 87: an intraocular hypertension model is established, and an ophthalmic medicine composition solution in Embodiment 87 is administered.

Embodiment group 88: an intraocular hypertension model is established, and an ophthalmic medicine composition solution in Embodiment 88 is administered.

Embodiment group 89: an intraocular hypertension model is established, and an ophthalmic medicine composition solution in Embodiment 89 is administered.

Embodiment group 90: an intraocular hypertension model is established, and an ophthalmic medicine composition solution in Embodiment 90 is administered.

Embodiment group 91: an intraocular hypertension model is established, and an ophthalmic medicine composition solution in Embodiment 91 is administered.

Embodiment group 92: an intraocular hypertension model is established, and an ophthalmic medicine composition solution in Embodiment 92 is administered.

A dosage regimen: according to a current clinical dosage regimen of commercially available latanoprost and timolol eye drops (Xalacom^{®}) and commercially available latanoprost and netarsudil eye drops (Rocklatan^{®}), this experiment is mainly divided into the following groups: (1) the treatment group 4: 50 µL the commercially available latanoprost and timolol eye drops (Xalacom^{®}) is administered to each eye each time once a day (9:00 PM); (2) the treatment group 5: 50 µL the commercially available latanoprost and netarsudil eye drops (Rocklatan^{®}) is administered to each eye each time once a day (9:00 PM); and (3) Embodiment groups 83 to 92: 50 µL eye drops in Embodiments 83 to 92 are administered to each eye each time once a day (9:00 PM). Drug administration is started in all the groups on the tenth day when the models are established successfully and lasts for 10 days. No processing is made to the normal control group and the model group. The dosage regimen is shown in FIG. 5.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 4, the treatment group 5 and Embodiment groups 83, 84, 85, 88, 89 and 90, the IOP can be reduced significantly by the ophthalmic composition solutions of Embodiment groups 86, 87, 91 and 92, however, IOP actual measured values and IOP variation values in Embodiment groups 86, 87, 91 and 92 have no significant difference.

**Table 152. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| Tim e (d) | IOP variation value (mmHg) | | | | | | | | | | | | | |
|---|---|---|---|---|---|---|---|---|---|---|---|---|---|---|
| | Nor mal contr ol grou p | Mod el grou p | Treat ment grou p 4 | Treat ment grou p 5 | Emb odim ent grou p 83 | Emb odim ent grou p 84 | Emb odim ent grou p 85 | Emb odim ent grou p 86 | Emb odim ent grou p 87 | Emb odim ent grou p 88 | Emb odim ent grou p 89 | Emb odim ent grou p 90 | Emb odim ent grou p 91 | Emb odim ent grou p92 |
| 10d | - | - | - | - | - | - | - | - | - | - | - | - | - | - |
| 15 d | 0.50 | 2.25 | 1.84 | 1.50 | -6.00 | -6.50 | -6.00 | -3.50 | -8.50 | -4.00 | -5.00 | -4.50 | -6.60 | -8.50 |
| 20 d | -0.50 | 8.58 | -4.33 | -4.50 | -8.00 | -9.50 | -10.5 0 | -14.0 0 | -14.0 0 | -8.00 | -10.5 0 | -10.5 0 | -14.5 0 | -14.0 0 |

**Table 153. Intra-ocular pressure actual measured values of each group of rabbits measured on the tenth day, the fifteenth day and the twentieth day when drug administration is performed for 10 days on end starting with the tenth day**

| Ti m e (d ) | IOP actual measured value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 4 | Trea tme nt grou p 5 | Embo dime nt group 83 | Embo dime nt group 84 | Embo dime nt group 85 | Embo dime nt group 86 | Embo dime nt group 87 | Embo dime nt group 88 | Embo dime nt group 89 | Embo dime nt group 90 | Embo dime nt group 91 | Embo dime nt group 92 |
| 1 0 d | 6.0 0 | 26 .7 5 | 24.3 3 | 24.8 3 | 25.00 | 24.50 | 26.00 | 25.00 | 24.00 | 25.00 | 26.00 | 25.50 | 25.50 | 24.00 |
| 1 5 d | 6.5 0 | 29 .0 0 | 26.1 7 | 26.3 3 | 19.00 | 18.00 | 20.00 | 21.50 | 15.50 | 21.00 | 21.00 | 21.00 | 18.90 | 15.50 |
| 2 0 d | 5.5 0 | 35 .3 3 | 20.0 0 | 20.3 3 | 17.00 | 15.00 | 15.50 | 11.00 | 10.00 | 17.00 | 15.50 | 15.00 | 11.00 | 10.00 |

### Embodiments 94 to 98: a preparation including netarsudil dimesylate, timolol mesylate and bimatoprost

**Table 154. Preparation of a local ophthalmic medicine composition solution for reducing an intra-ocular pressure as follows:**

| No. | Component | Embodiment 94 | Embodiment 95 | Embodiment 96 | Embodiment 97 | Embodiment 98 |
|---|---|---|---|---|---|---|
| 1. | Netarsudil dimesylate | 14.2 mg | 14.2 mg | 28.5 mg | 28.5 mg | 57.0 mg |
| 2. | Timolol mesylate | 325 mg | 650 mg | 325 mg | 650 mg | 1300 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 155. Contents of various main medicine components in Embodiments 94 to 98 after being stored at 5°Cfor 24 months**

| No. | Component | Embodiment 94 | Embodiment 95 | Embodiment 96 | Embodiment 97 | Embodiment 98 |
|---|---|---|---|---|---|---|
| | | Content % | | | | |
| 1. | Netarsudil dimesylate | 99.6 | 100.7 | 100.4 | 101.1 | 100.6 |
| 2. | Timolol mesylate | 100.3 | 101.0 | 99.4 | 99.9 | 100.7 |
| 3. | Bimatoprost | 99.2 | 99.6 | 100.6 | 99.9 | 100.2 |

**Table 156. Contents of various main medicine components in commercially available eye drops (Timoptic^{®}, Rhopress^{®} and Lumigan^{®}) after being stored at 5°Cfor 24 months**

| No. | Component | Timoptic^{®} | Rhopress^{®} | Lumigan^{®} |
|---|---|---|---|---|
| | | Content % | | |
| 1. | Timolol maleate | 99.7 | - | - |
| 2. | Netarsudil dimesylate | - | 99.9 | - |
| 3. | Bimatoprost | - | - | 99.4 |

According to Embodiments 94 to 98, the preparation including netarsudil dimesylate, timolol mesylate and bimatoprost has good stability after being stored under a storage condition of 5°Cfor 24 months, which meets a requirement for a storage condition of the commercially available eye drops (Timoptic^{®}, Rhopress^{®} and Lumigan^{®}) before opening.

**Table 157. Contents of various main medicine components in Embodiments 94 to 98 after being stored at 25°Cfor 6 weeks**

| No. | Component | Embodiment 94 | Embodiment 95 | Embodiment 96 | Embodiment 97 | Embodiment 98 |
|---|---|---|---|---|---|---|
| | | Content % | | | | |
| 1. | Netarsudil dimesylate | 99.2 | 99.9 | 101.2 | 100.1 | 99.1 |
| 2. | Timolol mesylate | 99.3 | 99.8 | 101.6 | 99.9 | 99.6 |
| 3. | Bimatoprost | 100.3 | 100.3 | 101.4 | 99.2 | 100.6 |

**Table 158. Contents of various main medicine components in commercially available eye drops (Timoptic^{®}, Rhopress^{®} and Lumigan^{®}) after being stored at 25°Cfor 6 weeks**

| No. | Component | Timoptic^{®} | Rhopress^{®} | Lumigan^{®} |
|---|---|---|---|---|
| | | Content % | | |
| 1. | Timolol maleate | 99.5 | - | - |
| 2. | Netarsudil dimesylate | - | 99.8 | - |
| 3. | Bimatoprost | - | - | 99.5 |

According to Embodiments 94 to 98, the preparation including netarsudil dimesylate, timolol mesylate and bimatoprost has good stability after being stored under a storage condition of 25°Cfor 6 weeks, which meets a requirement for a storage condition of the commercially available eye drops (Timoptic^{®}, Rhopress^{®} and Lumigan^{®}) after opening.

**Table 159. Contents of various main medicine components in Embodiments 94 to 98 after being stored at 40°Cfor 14 days**

| No. | Component | Embodiment 94 | Embodiment 95 | Embodiment 96 | Embodiment 97 | Embodiment 98 |
|---|---|---|---|---|---|---|
| | | Content % | | | | |
| 1. | Netarsudil dimesylate | 101.2 | 101.1 | 99.8 | 99.1 | 99.3 |
| 2. | Timolol mesylate | 101.1 | 101.5 | 101.2 | 99.4 | 99.9 |
| 3. | Bimatoprost | 99.4 | 99.9 | 100.9 | 100.1 | 100.2 |

**Table 160. Contents of various main medicine components in commercially available eye drops (Timoptic^{®}, Rhopress^{®} and Lumigan^{®}) after being stored at 40°Cfor 14 days**

| No. | Component | Timoptic^{®} | Rhopress^{®} | Lumigan^{®} |
|---|---|---|---|---|
| | | Content % | | |
| 1. | Timolol maleate | 99.8 | - | - |
| 2. | Netarsudil dimesylate | - | 99.5 | - |
| 3. | Bimatoprost | - | - | 99.2 |

According to Embodiments 94 to 98, the preparation including netarsudil dimesylate, timolol mesylate and bimatoprost has good stability after being stored under a storage condition of 40°Cfor 14 days, which meets a requirement for departing from a storage condition of the commercially available eye drops (Timoptic^{®}, Rhopress^{®} and Lumigan^{®}) for a short time.

### Embodiments 99 to 103: a preparation including netarsudil maleate, timolol maleate and bimatoprost

**Table 161. Preparation of a local ophthalmic medicine composition solution for reducing an intra-ocular pressure as follows:**

| No. | Component | Embodiment 99 | Embodiment 100 | Embodiment 101 | Embodiment 102 | Embodiment 103 |
|---|---|---|---|---|---|---|
| 1. | Netarsudil maleate | 12.6 mg | 12.6 mg | 25.1 mg | 25.1 mg | 50.2 mg |
| 2. | Timolol maleate | 341.7 mg | 683.5 mg | 341.7 mg | 683.5 mg | 1367 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 162. Contents of various main medicine components in Embodiments 99 to 103 after being stored at 5°Cfor 24 months**

| No. | Component | Embodiment 99 | Embodiment 100 | Embodiment 101 | Embodiment 102 | Embodiment 103 |
|---|---|---|---|---|---|---|
| | | Content % | | | | |
| 1. | Netarsudil maleate | 99.1 | 100.4 | 100.8 | 99.3 | 98.2 |
| 2. | Timolol maleate | 99.8 | 99.4 | 99.4 | 100.2 | 102.1 |
| 3. | Bimatoprost | 101.1 | 99.1 | 99.5 | 99.3 | 100.1 |

According to Embodiments 99 to 103, the preparation including netarsudil maleate, timolol maleate and bimatoprost has good stability after being stored under a storage condition of 5°Cfor 24 months, which meets a requirement for a storage condition of the commercially available eye drops (Timoptic^{®}, Rhopress^{®} and Lumigan^{®}) before opening.

**Table 163. Contents of various main medicine components in Embodiments 99 to 103 after being stored at 25°Cfor 6 weeks**

| No. | Component | Embodiment 99 | Embodiment 100 | Embodiment 101 | Embodiment 102 | Embodiment 103 |
|---|---|---|---|---|---|---|
| | | Content % | | | | |
| 1. | Netarsudil maleate | 99.1 | 100.1 | 99.2 | 99.1 | 100.6 |
| 2. | Timolol maleate | 99.9 | 98.3 | 98.5 | 98.4 | 101.1 |
| 3. | Bimatoprost | 98.5 | 100.3 | 100.4 | 101.1 | 99.1 |

According to Embodiments 99 to 103, the preparation including netarsudil maleate, timolol maleate and bimatoprost has good stability after being stored under a storage condition of 25°Cfor 6 weeks, which meets a requirement for a storage condition of the commercially available eye drops (Timoptic^{®}, Rhopress^{®} and Lumigan^{®}) after opening.

**Table 164. Contents of various main medicine components after being stored at 40°Cfor 14 days**

| No. | Component | Embodiment 99 | Embodiment 100 | Embodiment 101 | Embodiment 102 | Embodiment 103 |
|---|---|---|---|---|---|---|
| | | | | Content % | | |
| 1. | Netarsudil maleate | 99.1 | 99.9 | 100.1 | 101.1 | 98.3 |
| 2. | Timolol maleate | 100.1 | 101.8 | 100.3 | 98.1 | 101.1 |
| 3. | Bimatoprost | 98.3 | 99.5 | 100.9 | 99.9 | 100.2 |

According to Embodiments 99 to 103, the preparation including netarsudil maleate, timolol maleate and bimatoprost has good stability after being stored under a storage condition of 40°Cfor 14 days, which meets a requirement for departing from a storage condition of the commercially available eye drops (Timoptic^{®}, Rhopress^{®} and Lumigan^{®}) for a short time.

### Embodiment 104: evaluation of pharmaceutical effects of the ophthalmic medicine composition solution in Embodiments 94 to 103 by using a Japanese white rabbit glaucoma model

### Experimental grouping:

Normal control group: no processing.

Model group: an intraocular hypertension model is established, and no eye drop is administered.

Treatment group 6: an intraocular hypertension model is established, and commercially available bimatoprost and timolol maleate eye drops (Ganfort^{®}) are administered.

Embodiment group 94: an intraocular hypertension model is established, and an ophthalmic medicine composition solution in Embodiment 94 is administered.

Embodiment group 95: an intraocular hypertension model is established, and an ophthalmic medicine composition solution in Embodiment 95 is administered.

Embodiment group 96: an intraocular hypertension model is established, and an ophthalmic medicine composition solution in Embodiment 96 is administered.

Embodiment group 97: an intraocular hypertension model is established, and an ophthalmic medicine composition solution in Embodiment 97 is administered.

Embodiment group 98: an intraocular hypertension model is established, and an ophthalmic medicine composition solution in Embodiment 98 is administered.

Embodiment group 99: an intraocular hypertension model is established, and an ophthalmic medicine composition solution in Embodiment 99 is administered.

Embodiment group 100: an intraocular hypertension model is established, and an ophthalmic medicine composition solution in Embodiment 100 is administered.

Embodiment group 101: an intraocular hypertension model is established, and an ophthalmic medicine composition solution in Embodiment 101 is administered.

Embodiment group 102: an intraocular hypertension model is established, and an ophthalmic medicine composition solution in Embodiment 102 is administered.

Embodiment group 103: an intraocular hypertension model is established, and an ophthalmic medicine composition solution in Embodiment 103 is administered.

A dosage regimen: according to a current clinical dosage regimen of commercially available bimaprost and timolol maleate eye drops (Ganfort^{®}), this experiment is mainly divided into the following groups: (1) the treatment group 6: 50 µL the commercially available bimatoprost and timolol maleate eye drops (Ganfort^{®}) are administered to each eye each time once a day (9:00 PM); and (2) Embodiment groups 94 to 103: 50 µL eye drops in Embodiments 94 to 103 are administered to each eye each time once a day (9:00 PM). Drug administration is started in all the groups on the tenth day when the models are established successfully and lasts for 10 days. No processing is made to the normal control group and the model group. The dosage regimen is shown in FIG. 7.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 6 and Embodiment groups 94, 95, 96, 99, 100 and 101, the IOP can be reduced significantly by the ophthalmic composition solutions of Embodiment groups 97, 98, 102 and 103, however, IOP actual measured values and IOP variation values in Embodiment groups 97, 98, 102 and 103 have no significant difference.

**Table 165. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| | IOP variation value (mmHg) | | | | | | | | | | | | |
|---|---|---|---|---|---|---|---|---|---|---|---|---|---|
| Ti m e (d ) | Nor mal con trol gro up | M od el gro up | Treat ment grou p 6 | Embo dimen t group 94 | Embo dimen t group 95 | Embo dimen t group 96 | Embo dimen t group 97 | Embo dimen t group 98 | Embo dimen t group 99 | Embo dimen t group 100 | Embo dimen t group 101 | Embo dimen t group 102 | Embo dimen t group 103 |
| 10 d | - | - | - | - | - | - | - | - | - | - | - | - | - |
| 15 d | 0.5 0 | 2.2 5 | -0.83 | -5.00 | -6.00 | -6.00 | -6.70 | -7.50 | -3.50 | -5.00 | -6.30 | -7.30 | -6.60 |
| 20 d | -0. 50 | 8.5 8 | -4.50 | -8.70 | -9.20 | -11.00 | -15.20 | -14.00 | -8.30 | -10.70 | -9.70 | -14.30 | -14.50 |

**Table 166. Intra-ocular pressure actual measured values of each group of rabbits measured on the tenth day, the fifteenth day and the twentieth day when drug administration is performed for 10 days on end starting with the tenth day**

| | IOP actual measured value (mmHg) | | | | | | | | | | | | |
|---|---|---|---|---|---|---|---|---|---|---|---|---|---|
| Ti m e (d ) | Nor mal con trol gro up | M od el gro up | Treat ment grou p 6 | Embo dimen t group 94 | Embo dimen t group 95 | Embo dimen t group 96 | Embo dimen t group 97 | Embo dimen t group 98 | Embo dimen t group 99 | Embo dimen t group 100 | Embo dimen t group 101 | Embo dimen t group 102 | Embo dimen t group 103 |
| 10 d | 6.0 0 | 26. 75 | 24.0 0 | 25.50 | 25.00 | 26.00 | 26.70 | 25.00 | 25.00 | 26.50 | 25.00 | 26.00 | 25.50 |
| 15 d | 6.5 0 | 29. 00 | 23.1 7 | 20.50 | 19.00 | 20.00 | 20.00 | 17.50 | 21.50 | 21.50 | 18.70 | 18.70 | 18.90 |
| 20 d | 5.5 0 | 35. 33 | 19.5 0 | 16.80 | 15.80 | 15.00 | 11.50 | 11.00 | 16.70 | 15.80 | 15.30 | 11.70 | 11.00 |

### Embodiments 105 to 109: a preparation including netarsudil dimesylate, timolol mesylate and travoprost

**Table 167. Preparation of a local ophthalmic medicine composition solution for reducing an intra-ocular pressure as follows:**

| No. | Component | Embodiment 105 | Embodiment 106 | Embodiment 107 | Embodiment 108 | Embodiment 109 |
|---|---|---|---|---|---|---|
| 1. | Netarsudil dimesylate | 14.2 mg | 14.2 mg | 28.5 mg | 28.5 mg | 57.0 mg |
| 2. | Timolol mesylate | 325 mg | 650 mg | 325 mg | 650 mg | 1300 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100mL | 100mL | 100mL | 100mL | 100mL |

A preparation process may refer to the preparation process in Embodiments 83 to 87.

**Table 168. Contents of various main medicine components in Embodiments 105 to 109 after being stored at 5°Cfor 24 months**

| No. | Component | Embodiment 105 | Embodiment 106 | Embodiment 107 | Embodiment 108 | Embodiment 109 |
|---|---|---|---|---|---|---|
| | | Content % | | | | |
| 1. | Netarsudil dimesylate | 99.9 | 99.7 | 100.2 | 100.1 | 99.6 |
| 2. | Timolol mesylate | 101.3 | 100.2 | 100.4 | 99.8 | 99.7 |
| 3. | Travoprost | 100.2 | 99.8 | 99.6 | 99.2 | 100.5 |

**Table 169. Contents of various main medicine components in commercially available eye drops (Timoptic^{®}, Rhopress^{®} and Travatan^{®}) after being stored at 5°Cfor 24 months**

| No. | Component | Timoptic^{®} | Rhopress^{®} | Travatan^{®} |
|---|---|---|---|---|
| | | Content % | | |
| 1. | Timolol maleate | 99.7 | - | - |
| 2. | Netarsudil dimesylate | - | 99.9 | - |
| 3. | Travoprost | - | - | 99.8 |

According to Embodiments 105 to 109, the preparation including netarsudil dimesylate, timolol mesylate and travoprost has good stability after being stored under a storage condition of 5°Cfor 24 months, which meets a requirement for a storage condition of the commercially available eye drops (Timoptic^{®}, Rhopress^{®} and Travatan^{®}) before opening.

**Table 170. Contents of various main medicine components in Embodiments 105 to 109 after being stored at 25°Cfor 6 weeks**

| No. | Component | Embodiment 105 | Embodiment 106 | Embodiment 107 | Embodiment 108 | Embodiment 109 |
|---|---|---|---|---|---|---|
| | | Content % | | | | |
| 1. | Netarsudil dimesylate | 99.8 | 98.9 | 101.4 | 100.4 | 99.7 |
| 2. | Timolol mesylate | 100.3 | 99.4 | 100.6 | 98.9 | 100.6 |
| 3. | Travoprost | 100.9 | 100.1 | 102.4 | 99.6 | 100.1 |

**Table 171. Contents of various main medicine components in commercially available eye drops (Timoptic^{®}, Rhopress^{®} and Travatan^{®}) after being stored at 25°Cfor 6 weeks**

| No. | Component | Timoptic^{®} | Rhopress^{®} | Travatan^{®} |
|---|---|---|---|---|
| | | Content % | | |
| 1. | Timolol maleate | 99.5 | - | - |
| 2. | Netarsudil dimesylate | - | 99.8 | - |
| 3. | Travoprost | - | - | 99.6 |

According to Embodiments 105 to 109, the preparation including netarsudil dimesylate, timolol mesylate and travoprost has good stability after being stored under a storage condition of 25°Cfor 6 weeks, which meets a requirement for a storage condition of the commercially available eye drops (Timoptic^{®}, Rhopress^{®} and Travatan^{®}) after opening.

**Table 172. Contents of various main medicine components in Embodiments 105 to 109 after being stored at 40°Cfor 14 days**

| No. | Component | Embodiment 105 | Embodiment 106 | Embodiment 107 | Embodiment 108 | Embodiment 109 |
|---|---|---|---|---|---|---|
| | | Content % | | | | |
| 1. | Netarsudil dimesylate | 100.2 | 101.4 | 100.8 | 99.6 | 100.3 |
| 2. | Timolol mesylate | 100.1 | 100.5 | 99.5 | 99.6 | 99.1 |
| 3. | Travoprost | 99.1 | 99.2 | 100.1 | 100.8 | 100.1 |

**Table 173. Contents of various main medicine components in commercially available eye drops (Timoptic^{®}, Rhopress^{®} and Travatan^{®}) after being stored at 40°Cfor 14 days**

| No. | Component | Timoptic^{®} | Rhopress^{®} | Travatan^{®} |
|---|---|---|---|---|
| | | Content % | | |
| 1. | Timolol maleate | 99.8 | - | - |
| 2. | Netarsudil dimesylate | - | 99.5 | - |
| 3. | Travoprost | - | - | 99.7 |

According to Embodiments 105 to 109, the preparation including netarsudil dimesylate, timolol mesylate and travoprost has good stability after being stored under a storage condition of 40°Cfor 14 days, which meets a requirement for departing from a storage condition of the commercially available eye drops (Timoptic^{®}, Rhopress^{®} andTravatan^{®}) for a short time.

### Embodiments 110 to 114: a preparation including netarsudil maleate, timolol maleate and travoprost

**Table 174. Preparation of a local ophthalmic medicine composition solution for reducing an intra-ocular pressure as follows:**

| No. | Component | Embodiment 110 | Embodiment 111 | Embodiment 112 | Embodiment 113 | Embodiment 114 |
|---|---|---|---|---|---|---|
| 1. | Netarsudil maleate | 12.6 mg | 12.6 mg | 25.1 mg | 25.1 mg | 50.2 mg |
| 2. | Timolol maleate | 341.7 mg | 683.5 mg | 341.7 mg | 683.5 mg | 1367 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 175. Contents of various main medicine components in Embodiments 110 to 114 after being stored at 5°Cfor 24 months**

| No. | Component | Embodiment 110 | Embodiment 111 | Embodiment 112 | Embodiment 113 | Embodiment 114 |
|---|---|---|---|---|---|---|
| | | Content % | | | | |
| 1. | Netarsudil maleate | 100.3 | 100.3 | 99.6 | 99.7 | 99.6 |
| 2. | Timolol maleate | 99.8 | 100.7 | 99.4 | 100.3 | 100.1 |
| 3. | Travoprost | 100.2 | 100.1 | 99.3 | 99.9 | 99.1 |

According to Embodiments 110 to 114, the preparation including netarsudil maleate, timolol maleate and travoprost has good stability after being stored under a storage condition of 5°Cfor 24 months, which meets a requirement for a storage condition of the commercially available eye drops (Timoptic^{®}, Rhopress^{®} and Travatan^{®}) before opening.

**Table 176. Contents of various main medicine components in Embodiments 110 to 114 after being stored at 25°Cfor 6 weeks**

| No. | Component | Embodiment 110 | Embodiment 111 | Embodiment 112 | Embodiment 113 | Embodiment 114 |
|---|---|---|---|---|---|---|
| | | | | Content % | | |
| 1. | Netarsudil maleate | 100.1 | 100.8 | 99.5 | 99.8 | 100.5 |
| 2. | Timolol maleate | 99.3 | 99.3 | 100.5 | 100.4 | 100.1 |
| 3. | Travoprost | 99.2 | 100.1 | 100.7 | 99.1 | 98.9 |

According to Embodiments 110 to 114, the preparation including netarsudil maleate, timolol maleate and travoprost has good stability after being stored under a storage condition of 25°Cfor 6 weeks, which meets a requirement for a storage condition of the commercially available eye drops (Timoptic^{®}, Rhopress^{®} and Travatan^{®}) after opening.

**Table 177. Contents of various main medicine components in Embodiments 110 to 114 after being stored at 40°Cfor 14 days**

| No. | Component | Embodiment 110 | Embodiment 111 | Embodiment 112 | Embodiment 113 | Embodiment 114 |
|---|---|---|---|---|---|---|
| | | Content % | | | | |
| 1. | Netarsudil maleate | 100.1 | 98.9 | 100.9 | 100.1 | 98.8 |
| 2. | Timolol maleate | 100.9 | 100.8 | 100.5 | 99.9 | 100.1 |
| 3. | Travoprost | 99.3 | 99.1 | 100.1 | 99.5 | 100.9 |

According to Embodiments 110 to 114, the preparation including netarsudil maleate, timolol maleate and travoprost has good stability after being stored under a storage condition of 40°Cfor 14 days, which meets a requirement for departing from a storage condition of the commercially available eye drops (Timoptic^{®}, Rhopress^{®} and Travatan^{®}) for a short time.

### Embodiment 115: evaluation of pharmaceutical effects of the ophthalmic medicine composition solution in Embodiments 105 to 114 by using a Japanese white rabbit glaucoma model

### Experimental grouping:

Normal control group: no processing.

Model group: an intraocular hypertension model is established, and no eye drop is administered.

Treatment group 7: an intraocular hypertension model is established, and commercially available travoprost and timolol maleate eye drops (DuoTrav^{®}) are administered.

Embodiment group 105: an intraocular hypertension model is established, and an ophthalmic medicine composition solution in Embodiment 105 is administered.

Embodiment group 106: an intraocular hypertension model is established, and an ophthalmic medicine composition solution in Embodiment 106 is administered.

Embodiment group 107: an intraocular hypertension model is established, and an ophthalmic medicine composition solution in Embodiment 107 is administered.

Embodiment group 108: an intraocular hypertension model is established, and an ophthalmic medicine composition solution in Embodiment 108 is administered.

Embodiment group 109: an intraocular hypertension model is established, and an ophthalmic medicine composition solution in Embodiment 109 is administered.

Embodiment group 110: an intraocular hypertension model is established, and an ophthalmic medicine composition solution in Embodiment 110 is administered.

Embodiment group 111: an intraocular hypertension model is established, and an ophthalmic medicine composition solution in Embodiment 111 is administered.

Embodiment group 112: an intraocular hypertension model is established, and an ophthalmic medicine composition solution in Embodiment 112 is administered.

Embodiment group 113: an intraocular hypertension model is established, and an ophthalmic medicine composition solution in Embodiment 113 is administered.

Embodiment group 114: an intraocular hypertension model is established, and an ophthalmic medicine composition solution in Embodiment 114 is administered.

A dosage regimen: according to a current clinical dosage regimen of commercially available travoprost and timolol maleate eye drops (DuoTrav^{®}), this experiment is mainly divided into the following groups: (1) the treatment group 7: 50 µL the commercially available travoprost and timolol maleate eye drops (DuoTrav^{®}) are administered to each eye each time once a day (9:00 PM); and (2) Embodiment groups 105 to 114: 50 µL eye drops in Embodiments 105 to 114 are administered to each eye each time once a day (9:00 PM). Drug administration is started in all the groups on the tenth day when the models are established successfully and lasts for 10 days. No processing is made to the normal control group and the model group. The dosage regimen is shown in FIG. 9.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 7 and Embodiment groups 105, 106, 107, 110, 111 and 112, the IOP can be reduced significantly by the ophthalmic composition solutions of Embodiment groups 108, 109, 113 and 114, however, IOP actual measured values and IOP variation values in Embodiment groups 108, 109, 113 and 114 have no significant difference.

**Table 178. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| | IOP variation value (mmHg) | | | | | | | | | | | | |
|---|---|---|---|---|---|---|---|---|---|---|---|---|---|
| Ti m e (d ) | Nor mal con trol gro up | M od el gro up | Treat ment grou p 7 | Embo dimen t group 105 | Embo dimen t group 106 | Embo dimen t group 107 | Embo dimen t group 108 | Embo dimen t group 109 | Embo dimen t group 110 | Embo dimen t group 111 | Embo dimen t group 112 | Embo dimen t group 113 | Embo dimen t group 114 |
| 10 d | - | - | - | - | - | - | - | - | - | - | - | - | |
| 15 d | 0.5 0 | 2.2 5 | -4.00 | -5.50 | -5.00 | -5.30 | -7.70 | -5.30 | -5.40 | -5.50 | -5.70 | -8.20 | -10.50 |
| 20 d | -0. 50 | 8.5 8 | -5.70 | -11.00 | -10.70 | -12.00 | -16.20 | -16.00 | -10.70 | -12.30 | -12.10 | -16.40 | -16.90 |

**Table 179. Intra-ocular pressure actual measured values of each group of rabbits measured on the tenth day, the fifteenth day and the twentieth day when drug administration is performed for 10 days on end starting with the tenth day**

| | IOP actual measured value (mmHg) | | | | | | | | | | | | |
|---|---|---|---|---|---|---|---|---|---|---|---|---|---|
| Ti m e (d ) | Nor mal con trol gro up | M od el gro up | Treat ment grou p 7 | Embo dimen t group 105 | Embo dimen t group 106 | Embo dimen t group 107 | Embo dimen t group 108 | Embo dimen t group 109 | Embo dimen t group 110 | Embo dimen t group 111 | Embo dimen t group 112 | Embo dimen t group 113 | Embo dimen t group 114 |
| 10 d | 6.0 0 | 26. 75 | 25.0 0 | 26.00 | 25.00 | 26.00 | 26.70 | 26.00 | 25.70 | 26.50 | 26.00 | 26.70 | 27.00 |
| 15 d | 6.5 0 | 29. 00 | 21.0 0 | 20.50 | 20.00 | 20.70 | 19.00 | 20.70 | 20.30 | 21.00 | 20.30 | 18.50 | 16.50 |
| 20 d | 5.5 0 | 35. 33 | 19.3 0 | 15.00 | 14.30 | 14.00 | 10.50 | 10.00 | 15.00 | 14.20 | 13.90 | 10.30 | 10.10 |

### Embodiments 116 to 120: a preparation including netarsudil dimesylate, timolol mesylate and tafluprost

**Table 180. Preparation of a local ophthalmic medicine composition solution for reducing an intra-ocular pressure as follows:**

| No | Component | Embodiment 116 | Embodiment 117 | Embodiment 118 | Embodiment 119 | Embodiment 120 |
|---|---|---|---|---|---|---|
| 1. | Netarsudil dimesylate | 14.2 mg | 14.2 mg | 28.5 mg | 28.5 mg | 57.0 mg |
| 2. | Timolol mesylate | 325 mg | 650 mg | 325 mg | 650 mg | 1300 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 181. Contents of various main medicine components in Embodiments 116 to 120 after being stored at 5°Cfor 24 months**

| No | Component | Embodiment 116 | Embodiment 117 | Embodiment 118 | Embodiment 119 | Embodiment 120 |
|---|---|---|---|---|---|---|
| | | Content % | | | | |
| 1. | Netarsudil dimesylate | 99.3 | 99.6 | 100.7 | 100.4 | 99.1 |
| 2. | Timolol mesylate | 100.3 | 100.2 | 100.5 | 99.5 | 99.8 |
| 3. | Tafluprost | 101.2 | 100.8 | 99.7 | 99.3 | 99.5 |

**Table 182. Contents of various main medicine components in commercially available eye drops (Timoptic^{®}, Rhopress^{®} and Tapros^{®}) after being stored at 5°Cfor 24 months**

| No | Component | Timoptic^{®} | Rhopress^{®} | Tapros^{®} |
|---|---|---|---|---|
| | | Content % | | |
| 1. | Timolol maleate | 99.7 | - | - |
| 2. | Netarsudil dimesylate | - | 99.9 | - |
| 3. | Tafluprost | - | - | 99.4 |

According to Embodiments 116 to 120, the preparation including netarsudil dimesylate, timolol mesylate and tafluprost has good stability after being stored under a storage condition of 5°Cfor 24 months, which meets a requirement for a storage condition of the commercially available eye drops (Timoptic^{®}, Rhopress^{®} and Tapros^{®}) before opening.

**Table 183. Contents of various main medicine components in Embodiments 116 to 120 after being stored at 25°Cfor 6 weeks**

| No | Component | Embodiment 116 | Embodiment 117 | Embodiment 118 | Embodiment 119 | Embodiment 120 |
|---|---|---|---|---|---|---|
| | | Content % | | | | |
| 1. | Netarsudil dimesylate | 99.1 | 99.4 | 100.4 | 100.3 | 99.4 |
| 2. | Timolol mesylate | 100.3 | 99.6 | 99.6 | 99.9 | 99.6 |
| 3. | Tafluprost | 100.2 | 99.1 | 101.4 | 99.7 | 100.5 |

**Table 184. Contents of various main medicine components in commercially available eye drops (Timoptic^{®}, Rhopress^{®} and Tapros^{®}) after being stored at 25°Cfor 6 weeks**

| No | Component | Timoptic^{®} | Rhopress^{®} | Tapros^{®} |
|---|---|---|---|---|
| | | Content % | | |
| 1. | Timolol maleate | 99.5 | - | - |
| 2. | Netarsudil dimesylate | - | 99.8 | - |
| 3. | Tafluprost | - | - | 99.3 |

According to Embodiments 116 to 120, the preparation including netarsudil dimesylate, timolol mesylate and tafluprost has good stability after being stored under a storage condition of 25°Cfor 6 weeks, which meets a requirement for a storage condition of the commercially available eye drops (Timoptic^{®}, Rhopress^{®} and Tapros^{®}) after opening.

**Table 185. Contents of various main medicine components in Embodiments 116 to 120 after being stored at 40°Cfor 14 days**

| No | Component | Embodiment 116 | Embodiment 117 | Embodiment 118 | Embodiment 119 | Embodiment 120 |
|---|---|---|---|---|---|---|
| | | Content % | | | | |
| 1. | Netarsudil dimesylate | 100.8 | 100.4 | 99.8 | 100.6 | 100.4 |
| 2. | Timolol mesylate | 100.7 | 100.4 | 99.7 | 99.4 | 100.1 |
| 3. | Tafluprost | 99.5 | 99.9 | 99.4 | 99.8 | 100.2 |

**Table 186. Contents of various main medicine components in commercially available eye drops (Timoptic^{®}, Rhopress^{®} and Tapros^{®}) after being stored at 40°Cfor 14 days**

| No | Component | Timoptic^{®} | Rhopress^{®} | Tapros^{®} |
|---|---|---|---|---|
| | | Content % | | |
| 1. | Timolol maleate | 99.8 | - | - |
| 2. | Netarsudil dimesylate | - | 99.5 | - |
| 3. | Tafluprost | - | - | 99.8 |

According to Embodiments 116 to 120, the preparation including netarsudil dimesylate, timolol mesylate and tafluprost has good stability after being stored under a storage condition of 40°Cfor 14 days, which meets a requirement for departing from a storage condition of the commercially available eye drops (Timoptic^{®}, Rhopress^{®}, Tapros^{®}) for a short time.

### Embodiments 121 to 125: a preparation including netarsudil maleate, timolol maleate and tafluprost

**Table 187. Preparation of a local ophthalmic medicine composition solution for reducing an intra-ocular pressure as follows:**

| No | Component | Embodiment 121 | Embodiment 122 | Embodiment 123 | Embodiment 124 | Embodiment 125 |
|---|---|---|---|---|---|---|
| 1. | Netarsudil maleate | 12.6 mg | 12.6 mg | 25.1 mg | 25.1 mg | 50.2 mg |
| 2. | Timolol maleate | 341.7 mg | 683.5 mg | 341.7 mg | 683.5 mg | 1367 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 188. Contents of various main medicine components in Embodiments 121 to 125 after being stored at 5°Cfor 24 months**

| No | Component | Embodiment 121 | Embodiment 122 | Embodiment 123 | Embodiment 124 | Embodiment 125 |
|---|---|---|---|---|---|---|
| | | Content % | | | | |
| 1. | Netarsudil maleate | 100.1 | 100.4 | 100.5 | 99.5 | 99.2 |
| 2. | Timolol maleate | 100.8 | 99.3 | 99.4 | 100.1 | 100.3 |
| 3. | Tafluprost | 101.2 | 100.1 | 99.3 | 99.4 | 100.1 |

According to Embodiments 121 to 125, the preparation including netarsudil maleate, timolol maleate and tafluprost has good stability after being stored under a storage condition of 5°Cfor 24 months, which meets a requirement for a storage condition of the commercially available eye drops (Timoptic^{®}, Rhopress^{®} and Tapro^{®}) before opening.

**Table 189. Contents of various main medicine components in Embodiments 121 to 125 after being stored at 25°Cfor 6 weeks**

| No | Component | Embodiment 121 | Embodiment 122 | Embodiment 123 | Embodiment 124 | Embodiment 125 |
|---|---|---|---|---|---|---|
| | | Content % | | | | |
| 1. | Netarsudil maleate | 100.1 | 100.8 | 99.5 | 99.8 | 100.5 |
| 2. | Timolol maleate | 99.3 | 99.3 | 100.5 | 100.4 | 100.1 |
| 3. | Tafluprost | 99.2 | 100.1 | 100.7 | 99.1 | 98.9 |

According to Embodiments 121 to 125, the preparation including netarsudil maleate, timolol maleate and tafluprost has good stability after being stored under a storage condition of 25°Cfor 6 weeks, which meets a requirement for a storage condition of the commercially available eye drops (Timoptic^{®}, Rhopress^{®} and Tapros^{®}) after opening.

**Table 190. Contents of various main medicine components in Embodiments 121 to 125 after being stored at 40°Cfor 14 days**

| No | Component | Embodiment 121 | Embodiment 122 | Embodiment 123 | Embodiment 124 | Embodiment 125 |
|---|---|---|---|---|---|---|
| | | Content % | | | | |
| 1. | Netarsudil maleate | 100.1 | 98.9 | 100.9 | 100.1 | 98.8 |
| 2. | Timolol maleate | 100.9 | 100.8 | 100.5 | 99.9 | 100.1 |
| 3. | Tafluprost | 99.3 | 99.1 | 100.1 | 99.5 | 100.9 |

According to Embodiments 121 to 125, the preparation including netarsudil maleate, timolol maleate and tafluprost has good stability after being stored under a storage condition of 40°Cfor 14 days, which meets a requirement for departing from a storage condition of the commercially available eye drops (Timoptic^{®}, Rhopress^{®} and Tapros^{®}) for a short time.

### Embodiment 126: evaluation of pharmaceutical effects of the ophthalmic medicine composition solution in Embodiments 116 to 125 by using a Japanese white rabbit glaucoma model

### Experimental grouping:

Normal control group: no processing.

Model group: an intraocular hypertension model is established, and no eye drop is administered.

Treatment group 8: an intraocular hypertension model is established, and commercially available tafluprost and timolol maleate eye drops (Tapcom^{®}) are administered.

Embodiment group 116: an intraocular hypertension model is established, and an ophthalmic medicine composition solution in Embodiment 116 is administered.

Embodiment group 117: an intraocular hypertension model is established, and an ophthalmic medicine composition solution in Embodiment 117 is administered.

Embodiment group 118: an intraocular hypertension model is established, and an ophthalmic medicine composition solution in Embodiment 118 is administered.

Embodiment group 119: an intraocular hypertension model is established, and an ophthalmic medicine composition solution in Embodiment 119 is administered.

Embodiment group 120: an intraocular hypertension model is established, and an ophthalmic medicine composition solution in Embodiment 120 is administered.

Embodiment group 121: an intraocular hypertension model is established, and an ophthalmic medicine composition solution in Embodiment 121 is administered.

Embodiment group 122: an intraocular hypertension model is established, and an ophthalmic medicine composition solution in Embodiment 122 is administered.

Embodiment group 123: an intraocular hypertension model is established, and an ophthalmic medicine composition solution in Embodiment 123 is administered.

Embodiment group 124: an intraocular hypertension model is established, and an ophthalmic medicine composition solution in Embodiment 124 is administered.

Embodiment group 125: an intraocular hypertension model is established, and an ophthalmic medicine composition solution in Embodiment 125 is administered.

A dosage regimen: according to a current clinical dosage regimen of commercially available tafluprost and timolol maleate eye drops (Tapcom^{®}), this experiment is mainly divided into the following groups: (1) the treatment group 8: 50 µL the commercially available tafluprost and timolol maleate eye drops (Tapcom^{®}) are administered to each eye each time once a day (9:00 PM); and (2) Embodiment groups 116 to 125: 50 µL eye drops in Embodiments 116 to 125 are administered to each eye each time once a day (9:00 PM). Drug administration is started in all the groups on the tenth day when the models are established successfully and lasts for 10 days. No processing is made to the normal control group and the model group. The dosage regimen is shown in FIG. 11.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 8 and Embodiment groups 116, 117, 118, 121, 122 and 123, the IOP can be reduced significantly by the ophthalmic composition solutions of Embodiment groups 119, 120, 124 and 125, however, IOP actual measured values and IOP variation values in Embodiment groups 119, 120, 124 and 125 have no significant difference.

**Table 191. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| Tim e (d ) | IOP variation value (mmHg) | | | | | | | | | | | | |
|---|---|---|---|---|---|---|---|---|---|---|---|---|---|
| | Nor mal con trol gro up | M od el gro up | Treat ment grou p 8 | Embo dimen t group 116 | Embo dimen t group 117 | Embo dimen t group 118 | Embo dimen t group 119 | Embo dimen t group 120 | Embo dimen t group 121 | Embo dimen t group 122 | Embo dimen t group 123 | Embo dimen t group 124 | Embo dimen t group 125 |
| 10 d | - | - | - | - | - | - | - | - | - | - | - | - | - |
| 15 d | 0.5 0 | 2.2 5 | -5.70 | -6.00 | -5.00 | -4.70 | -8.00 | -6.70 | -7.20 | -5.30 | -6.80 | -8.70 | -11.00 |
| 20 d | -0. 50 | 8.5 8 | -7.30 | -11.30 | -11.70 | -11.80 | -16.20 | -15.30 | -11.30 | -12.80 | -12.00 | -16.10 | -16.80 |

**Table 192. Intra-ocular pressure actual measured values of each group of rabbits measured on the tenth day, the fifteenth day and the twentieth day when drug administration is performed for 10 days on end starting with the tenth day**

| Ti m e (d ) | IOP actual measured value (mmHg) | | | | | | | | | | | | |
|---|---|---|---|---|---|---|---|---|---|---|---|---|---|
| | Nor mal con trol gro up | M od el gro up | Treat ment grou p 8 | Embo dimen t group 116 | Embo dimen t group 117 | Embo dimen t group 118 | Embo dimen t group 119 | Embo dimen t group 120 | Embo dimen t group 121 | Embo dimen t group 122 | Embo dimen t group 123 | Embo dimen t group 124 | Embo dimen t group 125 |
| 10 d | 6.0 0 | 26. 75 | 26.0 0 | 26.50 | 26.30 | 26.00 | 27.00 | 26.00 | 26.50 | 27.00 | 26.50 | 27.00 | 27.30 |
| 15 d | 6.5 0 | 29. 00 | 20.3 0 | 20.50 | 21.30 | 21.30 | 19.00 | 19.30 | 19.30 | 21.70 | 19.70 | 18.30 | 16.30 |
| 20 d | 5.5 0 | 35. 33 | 18.7 0 | 15.20 | 14.60 | 14.20 | 10.80 | 10.70 | 15.20 | 14.20 | 14.50 | 10.90 | 10.50 |

**Embodiment 127: a preparation including netarsudil dimesylate, timolol hydrobromide and latanoprost**

**Table 193. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol hydrobromide | 628 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 194. Contents of various main medicine components in Embodiment 127 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.4 | 100.4 |
| 2. | Timolol hydrobromide | 100.1 | 99.6 |
| 3. | Latanoprost | 100.3 | 99.5 |

According to Embodiment 127, the preparation including netarsudil dimesylate, timolol hydrobromide and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 128: a preparation including netarsudil dimesylate, timolol sulfate and latanoprost

**Table 195. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol sulfate | 655 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 196. Contents of various main medicine components in Embodiment 128 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.2 | 100.5 |
| 2. | Timolol sulfate | 100.5 | 100.1 |
| 3. | Latanoprost | 100.4 | 100.6 |

According to Embodiment 128, the preparation including netarsudil dimesylate, timolol sulfate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiments 129: a preparation including netarsudil dimesylate, timolol esilate and latanoprost

**Table 197. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol esilate | 674 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 198. Contents of various main medicine components in Embodiment 129 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.8 | 100.6 |
| 2. | Timolol esilate | 100.1 | 100.3 |
| 3. | Latanoprost | 100.4 | 100.1 |

According to Embodiment 129, the preparation including netarsudil dimesylate, timolol esilate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 130: a preparation including netarsudil dimesylate, timolol nitrate and latanoprost

**Table 199. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol nitrate | 600 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 mL |

A preparation process may refer to the preparation process in Embodiments 83 to 87.

**Table 200. Contents of various main medicine components in Embodiment 130 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.3 | 100.4 |
| 2. | Timolol nitrate | 99.4 | 99.8 |
| 3. | Latanoprost | 100.6 | 100.2 |

According to Embodiment 130, the preparation including netarsudil dimesylate, timolol nitrate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 131: a preparation including netarsudil dimesylate, timolol citrate and latanoprost

**Table 201. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol citrate | 804 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 202. Contents of various main medicine components in Embodiment 131 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.2 | 100.4 |
| 2. | Timolol citrate | 100.5 | 99.8 |
| 3. | Latanoprost | 100.7 | 99.5 |

According to Embodiment 131, the preparation including netarsudil dimesylate, timolol citrate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 132: a preparation including netarsudil dimesylate, timolol tartrate and latanoprost

**Table 203. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol tartrate | 737 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 204. Contents of various main medicine components in Embodiment 132 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.6 | 100.1 |
| 2. | Timolol tartrate | 100.6 | 99.9 |
| 3. | Latanoprost | 100.1 | 100.3 |

According to Embodiment 132, the preparation including netarsudil dimesylate, timolol tartrate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 133: a preparation including netarsudil dimesylate, timolol salicylate and latanoprost

**Table 205. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol salicylate | 718 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 206. Contents of various main medicine components in Embodiment 133 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.5 | 100.1 |
| 2. | Timolol salicylate | 99.4 | 99.8 |
| 3. | Latanoprost | 100.3 | 100.4 |

According to Embodiment 133, the preparation including netarsudil dimesylate, timolol salicylate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 134: a preparation including netarsudil dimesylate, timolol malate and latanoprost

**Table 207. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol malate | 712 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 208. Contents of various main medicine components in Embodiment 134 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.3 | 100.1 |
| 2. | Timolol malate | 100.1 | 100.5 |
| 3. | Latanoprost | 100.7 | 100.8 |

According to Embodiment 134, the preparation including netarsudil dimesylate, timolol malate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 135: a preparation including netarsudil dimesylate, timolol lactate and latanoprost

**Table 209. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol lactate | 642 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 L |

A preparation process may refer to the preparation process in Embodiments 83 to 87.

**Table 210. Contents of various main medicine components in Embodiment 135 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.8 | 100.4 |
| 2. | Timolol lactate | 100.3 | 100.5 |
| 3. | Latanoprost | 99.9 | 100.2 |

According to Embodiment 135, the preparation including netarsudil dimesylate, timolol lactate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 136: a preparation including netarsudil dimesylate, timolol phenylacetate and latanoprost

**Table 211. Preparation of a local ophthalmic medicine composition solution for reducing an intra-ocular pressure as follows:**

| No. | Component | Weight |
|---|---|---|
| 1. | Netarsudil dimesylate | 28.5 mg |
| 2. | Timolol phenylacetate | 715 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 212. Contents of various main medicine components in Embodiment 136 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.3 | 100.1 |
| 2. | Timolol phenylacetate | 100.3 | 100.6 |
| 3. | Latanoprost | 100.6 | 100.5 |

According to Embodiment 136, the preparation including netarsudil dimesylate, timolol phenylacetate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 137: a preparation including netarsudil dihydrobromide, timolol maleate and latanoprost

**Table 213. Preparation of a local ophthalmic medicine composition solution for reducing an intra-ocular pressure as follows:**

| No. | Component | Weight |
|---|---|---|
| 1. | Netarsudil dihydrobromide | 27.1 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 214. Contents of various main medicine components in Embodiment 137 after being stored at 5°Cfor 24 months and at 25°Cfor 6 weeks**

| No. | Component | 5°C | 25°C |
|---|---|---|---|
| | | Content % | |
| 1. | Netarsudil dihydrobromide | 100.4 | 100.1 |
| 2. | Timolol maleate | 99.5 | 100.4 |
| 3. | Latanoprost | 100.5 | 100.7 |

According to Embodiment 137, the preparation including netarsudil dihydrobromide, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 138: a preparation including netarsudil dihydrochloride, timolol maleate and latanoprost

**Table 215. Preparation of a local ophthalmic medicine composition solution for reducing an intra-ocular pressure as follows:**

| No. | Component | Weight |
|---|---|---|
| 1. | Netarsudil dihydrochloride | 23.2 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 216. Contents of various main medicine components in Embodiment 138 after being stored at 5°Cfor 24 months and at 25°Cfor 6 weeks**

| No. | Component | 5°C | 25°C |
|---|---|---|---|
| | | Content % | |
| 1. | Netarsudil dihydrochloride | 100.5 | 100.6 |
| 2. | Timolol maleate | 100.3 | 99.8 |
| 3. | Latanoprost | 100.4 | 100.3 |

According to Embodiment 138, the preparation including netarsudil dihydrochloride, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 139: a preparation including netarsudil sulfate, timolol maleate and latanoprost

**Table 217. Preparation of a local ophthalmic medicine composition solution for reducing an intra-ocular pressure as follows:**

| No. | Component | Weight |
|---|---|---|
| 1. | Netarsudil sulfate | 24.3 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 218. Contents of various main medicine components in Embodiment 139 after being stored at 5°Cfor 24 months and at 25°Cfor 6 weeks**

| No. | Component | 5°C | 25°C |
|---|---|---|---|
| | | Content % | |
| 1. | Netarsudil sulfate | 99.5 | 100.1 |
| 2. | Timolol maleate | 100.2 | 100.5 |
| 3. | Latanoprost | 100.3 | 100.1 |

According to Embodiment 139, the preparation including netarsudil sulfate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 140: a preparation including netarsudil diformate, timolol maleate and latanoprost

**Table 219. Preparation of a local ophthalmic medicine composition solution for reducing an intra-ocular pressure as follows:**

| No. | Component | Weight |
|---|---|---|
| 1. | Netarsudil diformate | 24.1 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 220. Contents of various main medicine components in Embodiment 140 after being stored at 5°Cfor 24 months and at 25°Cfor 6 weeks**

| No. | Component | 5°C | 25°C |
|---|---|---|---|
| | | Content % | |
| 1. | Netarsudil diformate | 100.6 | 100.2 |
| 2. | Timolol maleate | 100.6 | 100.7 |
| 3. | Latanoprost | 100.4 | 100.2 |

According to Embodiment 140, the preparation including netarsudil diformate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 141: a preparation including netarsudil dinitrate, timolol maleate and latanoprost

**Table 221. Preparation of a local ophthalmic medicine composition solution for reducing an intra-ocular pressure as follows:**

| No. | Component | Weight |
|---|---|---|
| 1. | Netarsudil dinitrate | 25.6 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 222. Contents of various main medicine components in Embodiment 141 after being stored at 5°Cfor 24 months and at 25°Cfor 6 weeks**

| No. | Component | 5°C | 25°C |
|---|---|---|---|
| | | Content % | |
| 1. | Netarsudil dinitrate | 100.3 | 100.2 |
| 2. | Timolol maleate | 100.5 | 100.1 |
| 3. | Latanoprost | 100.6 | 100.4 |

According to Embodiment 141, the preparation including netarsudil dinitrate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 142: a preparation including netarsudil diacetate, timolol maleate and latanoprost

**Table 223. Preparation of a local ophthalmic medicine composition solution for reducing an intra-ocular pressure as follows:**

| No. | Component | Weight |
|---|---|---|
| 1. | Netarsudil diacetate | 27.8 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 224. Contents of various main medicine components in Embodiment 142 after being stored at 5°Cfor 24 months and at 25°Cfor 6 weeks**

| No. | Component | 5°C | 25°C |
|---|---|---|---|
| | | Content % | |
| 1. | Netarsudil diacetate | 100.8 | 100.5 |
| 2. | Timolol maleate | 100.3 | 100.1 |
| 3. | Latanoprost | 100.4 | 100.5 |

According to Embodiment 142, the preparation including netarsudil diacetate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 143: a preparation including netarsudil dibenzoate, timolol maleate and latanoprost

**Table 225. Preparation of a local ophthalmic medicine composition solution for reducing an intra-ocular pressure as follows:**

| No. | Component | Weight |
|---|---|---|
| 1. | Netarsudil dibenzoate | 30.8 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 226. Contents of various main medicine components in Embodiment 143 after being stored at 5°Cfor 24 months and at 25°Cfor 6 weeks**

| No. | Component | 5°C | 25°C |
|---|---|---|---|
| | | Content % | |
| 1. | Netarsudil dibenzoate | 100.5 | 99.9 |
| 2. | Timolol maleate | 100.1 | 100.2 |
| 3. | Latanoprost | 99.8 | 100.1 |

According to Embodiment 143, the preparation including netarsudil dibenzoate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 144: a preparation including netarsudil oxalate, timolol maleate and latanoprost

**Table 227. Preparation of a local ophthalmic medicine composition solution for reducing an intra-ocular pressure as follows:**

| No. | Component | Weight |
|---|---|---|
| 1. | Netarsudil oxalate | 27.9 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 228. Contents of various main medicine components in Embodiment 144 after being stored at 5°Cfor 24 months and at 25°Cfor 6 weeks**

| No. | Component | 5°C | 25°C |
|---|---|---|---|
| | | Content % | |
| 1. | Netarsudil oxalate | 100.4 | 100.2 |
| 2. | Timolol maleate | 99.4 | 99.8 |
| 3. | Latanoprost | 100.4 | 100.2 |

According to Embodiment 144, the preparation including netarsudil oxalate, timolol maleate and latanoprost has good stability after being stored under a storage condition of 5°Cfor 24 months, which meets a requirement for a storage condition of the commercially available eye drops (Timoptic^{®}, Rhopress^{®} andXalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 145: a preparation including netarsudil succinate, timolol maleate and latanoprost

**Table 229. Preparation of a local ophthalmic medicine composition solution for reducing an intra-ocular pressure as follows:**

| No. | Component | Weight |
|---|---|---|
| 1. | Netarsudil succinate | 25.2 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 230. Contents of various main medicine components in Embodiment 145 after being stored at 5°Cfor 24 months and at 25°Cfor 6 weeks**

| No. | Component | 5°C | 25°C |
|---|---|---|---|
| | | Content % | |
| 1. | Netarsudil succinate | 100.4 | 100.2 |
| 2. | Timolol maleate | 100.2 | 99.8 |
| 3. | Latanoprost | 100.4 | 99.5 |

According to Embodiment 145, the preparation including netarsudil succinate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Embodiment 146: a preparation including netarsudil diphenylacetate, timolol maleate and latanoprost

**Table 231. Preparation of a local ophthalmic medicine composition solution for reducing an intra-ocular pressure as follows:**

| No. | Component | Weight |
|---|---|---|
| 1. | Netarsudil diphenylacetate | 32.0 mg |
| 2. | Timolol maleate | 683.5 mg |
| 3. | Latanoprost | 5 mg |
| 4. | Boric acid | 50 mg |
| 5. | Mannitol | 5000 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 232. Contents of various main medicine components in Embodiment 146 after being stored at 5°Cfor 24 months and at 25°Cfor 6 weeks**

| No. | Component | 5°C | 25°C |
|---|---|---|---|
| | | Content % | |
| 1. | Netarsudil diphenylacetate | 100.1 | 100.3 |
| 2. | Timolol maleate | 99.4 | 100.2 |
| 3. | Latanoprost | 99.1 | 99.5 |

According to Embodiment 146, the preparation including netarsudil diphenylacetate, timolol maleate and latanoprost has good stability after being stored under a storage condition of 5°Cfor 24 months, which meets a requirement for a storage condition of the commercially available eye drops (Timoptic^{®}, Rhopress^{®} and Xalatan^{®}) before opening; and has good stability after being stored under a storage condition of 25°Cfor 6 weeks, which meets a requirement for a storage condition of the commercially available eye drops (Timoptic^{®}, Rhopress^{®} and Xalatan^{®}) after opening.

### Comparative examples 49 to 52: a preparation including netarsudil dimesylate, timolol maleate and latanoprost

**Table 233. Preparation of a local ophthalmic medicine composition solution for reducing an intra-ocular pressure as follows:**

| No. | Component | Comparative example 25 | Comparative example 26 | Comparative example 27 | Comparative example 28 |
|---|---|---|---|---|---|
| 1. | Netarsudil dimesylate | 14.2 mg | 14.2 mg | 28.5 mg | 28.5 mg |
| 2. | Timolol maleate | 341.7 mg | 683.5 mg | 341.7 mg | 683.5 mg |
| 3. | Latanoprost | 5 mg | 5 mg | 5 mg | 5 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 mL |

A preparation process may refer to the preparation process in Embodiments 83 to 87.

**Table 234. Contents of various main medicine components in Comparative examples 49 to 52 after being stored at 5°Cfor 7 days**

| No. | Component | Comparative example 25 | Comparative example 26 | Comparative example 27 | Comparative example 28 |
|---|---|---|---|---|---|
| | | Content % | | | |
| 1. | Netarsudil dimesylate | <30 | <30 | <30 | <30 |
| 2. | Timolol maleate | 99.3 | 99.5 | 100.3 | 100.1 |
| 3. | Latanoprost | 100.2 | 100.3 | 99.8 | 100.3 |

According to Comparative examples 49 to 52, the preparation including netarsudil dimesylate, timolol maleate and latanoprost has incompatibility under the storage condition of 5°C, and netarsudil dimesylate is precipitated.

### Comparative examples 53 to 56: a preparation including netarsudil dimesylate, maleic acid and latanoprost

**Table 235. Preparation of a local ophthalmic medicine composition solution for reducing an intra-ocular pressure as follows:**

| No. | Component | Comparative example 29 | Comparative example 30 | Comparative example 31 | Comparative example 32 |
|---|---|---|---|---|---|
| 1. | Netarsudil dimesylate | 14.2 mg | 14.2 mg | 28.5 mg | 28.5 mg |
| 2. | Maleic acid | 91.8 mg | 183.5 mg | 91.8 mg | 183.5 mg |
| 3. | Latanoprost | 5 mg | 5 mg | 5 mg | 5 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 236. Contents of various main medicine components in Comparative examples 53 to 56 after being stored at 5°Cfor 7 days**

| No. | Component | Comparative example 29 | Comparative example 30 | Comparative example 31 | Comparative example 32 |
|---|---|---|---|---|---|
| | | Content % | | | |
| 1. | Netarsudil dimesylate | <30 | <30 | <30 | <30 |
| 2. | Latanoprost | 99.9 | 99.5 | 100.4 | 100.2 |

According to Comparative examples 53 to 56, the preparation including netarsudil dimesylate, maleic acid and latanoprost has incompatibility under the storage condition of 5°C, and netarsudil dimesylate is precipitated.

### Comparative examples 33 to 36: a preparation including netarsudil dimesylate, timolol maleate and bimatoprost

**Table 237. Preparation of a local ophthalmic medicine composition solution for reducing an intra-ocular pressure as follows:**

| No. | Component | Comparative example 33 | Comparative example 34 | Comparative example 35 | Comparative example 36 |
|---|---|---|---|---|---|
| 1. | Netarsudil dimesylate | 14.2 mg | 14.2 mg | 28.5 mg | 28.5 mg |
| 2. | Timolol maleate | 341.7 mg | 683.5 mg | 341.7 mg | 683.5 mg |
| 3. | Bimatoprost | 30 mg | 30 mg | 30 mg | 30 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 238. Contents of various main medicine components in Comparative examples 33 to 36 after being stored at 5°Cfor 7 days**

| No. | Component | Comparative example 33 | Comparative example 34 | Comparative example 35 | Comparative example 36 |
|---|---|---|---|---|---|
| | | Content % | | | |
| 1. | Netarsudil dimesylate | <30 | <30 | <30 | <30 |
| 2. | Timolol maleate | 99.7 | 99.6 | 100.1 | 99.5 |
| 3. | Bimatoprost | 100.1 | 100.3 | 99.8 | 100.3 |

According to Comparative examples 33 to 36, the preparation including netarsudil dimesylate, timolol maleate and bimatoprost has incompatibility under the storage condition of 5°C, and netarsudil dimesylate is precipitated.

### Comparative examples 37 to 40: a preparation including netarsudil dimesylate, maleic acid and bimatoprost

**Table 239. Preparation of a local ophthalmic medicine composition solution for reducing an intra-ocular pressure as follows:**

| No. | Component | Comparative example 37 | Comparative example 38 | Comparative example 39 | Comparative example 40 |
|---|---|---|---|---|---|
| 1. | Netarsudil dimesylate | 14.2 mg | 14.2 mg | 28.5 mg | 28.5 mg |
| 2. | Maleic acid | 91.8 mg | 183.5 mg | 91.8 mg | 183.5 mg |
| 3. | Bimatoprost | 30 mg | 30 mg | 30 mg | 30 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 240. Contents of various main medicine components in Comparative examples 37 to 40 after being stored at 5°Cfor 7 days**

| No. | Component | Comparative example 37 | Comparative example 38 | Comparative example 39 | Comparative example 40 |
|---|---|---|---|---|---|
| | | Content % | | | |
| 1. | Netarsudil dimesylate | <30 | <30 | <30 | <30 |
| 2. | Bimatoprost | 99.4 | 99.3 | 100.1 | 100.0 |

According to Comparative examples 37 to 40, the preparation including netarsudil dimesylate, maleic acid and bimatoprost has incompatibility under the storage condition of 5°C, and netarsudil dimesylate is precipitated.

### Comparative examples 41 to 44: a preparation including netarsudil dimesylate, timolol maleate and travoprost

**Table 241. Preparation of a local ophthalmic medicine composition solution for reducing an intra-ocular pressure as follows:**

| No. | Component | Comparative example 41 | Comparative example 42 | Comparative example 43 | Comparative example 44 |
|---|---|---|---|---|---|
| 1. | Netarsudil dimesylate | 14.2 mg | 14.2 mg | 28.5 mg | 28.5 mg |
| 2. | Timolol maleate | 341.7 mg | 683.5 mg | 341.7 mg | 683.5 mg |
| 3. | Travoprost | 4 mg | 4 mg | 4 mg | 4 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 15 mg | 15 mg | 15 mg | 15 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 242. Contents of various main medicine components in Comparative examples 41 to 44 after being stored at 5°Cfor 7 days**

| No. | Component | Comparative example 41 | Comparative example 42 | Comparative example 43 | Comparative example 44 |
|---|---|---|---|---|---|
| | | Content % | | | |
| 1. | Netarsudil dimesylate | <30 | <30 | <30 | <30 |
| 2. | Timolol maleate | 99.2 | 99.7 | 99.1 | 99.6 |
| 3. | Travoprost | 100.4 | 100.2 | 99.9 | 100.1 |

According to Comparative examples 41 to 44, the preparation including netarsudil dimesylate, timolol maleate and travoprost has incompatibility under the storage condition of 5°C, and netarsudil dimesylate is precipitated.

### Comparative examples 45 to 48: a preparation including netarsudil dimesylate, maleic acid and travoprost

**Table 243. Preparation of a local ophthalmic medicine composition solution for reducing an intra-ocular pressure as follows:**

| No. | Component | Comparative example 45 | Comparative example 46 | Comparative example 47 | Comparative example 48 |
|---|---|---|---|---|---|
| 1. | Netarsudil dimesylate | 14.2 mg | 14.2 mg | 28.5 mg | 28.5 mg |
| 2. | Maleic acid | 91.8 mg | 183.5 mg | 91.8 mg | 183.5 mg |
| 3. | Travoprost | 4 mg | 4 mg | 4 mg | 4 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 15 mg | 15 mg | 15 mg | 15 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 244. Contents of various main medicine components in Comparative examples 45 to 48 after being stored at 5°Cfor 7 days**

| No. | Component | Comparative example 45 | Comparative example 46 | Comparative example 47 | Comparative example 48 |
|---|---|---|---|---|---|
| | | Content % | | | |
| 1. | Netarsudil dimesylate | <30 | <30 | <30 | <30 |
| 2. | Travoprost | 99.2 | 100.3 | 100.9 | 100.4 |

According to Comparative examples 45 to 48, the preparation including netarsudil dimesylate, maleic acid and travoprost has incompatibility under the storage condition of 5°C, and netarsudil dimesylate is precipitated.

### Comparative examples 49 to 52: a preparation including netarsudil dimesylate, timolol maleate and tafluprost

**Table 245. Preparation of a local ophthalmic medicine composition solution for reducing an intra-ocular pressure as follows:**

| No. | Component | Comparative example 49 | Comparative example 50 | Comparative example 51 | Comparative example 52 |
|---|---|---|---|---|---|
| 1. | Netarsudil dimesylate | 14.2 mg | 14.2 mg | 28.5 mg | 28.5 mg |
| 2. | Timolol maleate | 341.7 mg | 683.5 mg | 341.7 mg | 683.5 mg |
| 3. | Tafluprost | 1.5 mg | 1.5 mg | 1.5 mg | 1.5 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 246. Contents of various main medicine components in Comparative examples 49 to 52 after being stored at 5°Cfor 7 days**

| No. | Component | Comparative example 49 | Comparative example 50 | Comparative example 51 | Comparative example 52 |
|---|---|---|---|---|---|
| | | Content % | | | |
| 1. | Netarsudil dimesylate | <30 | <30 | <30 | <30 |
| 2. | Timolol maleate | 99.2 | 99.7 | 99.1 | 99.6 |
| 3. | Tafluprost | 100.4 | 100.2 | 99.9 | 100.1 |

According to Comparative examples 49 to 52, the preparation including netarsudil dimesylate, timolol maleate and tafluprost has incompatibility under the storage condition of 5°C, and netarsudil dimesylate is precipitated.

### Comparative examples 53 to 56: a preparation including netarsudil dimesylate, maleic acid and tafluprost

**Table 247. Preparation of a local ophthalmic medicine composition solution for reducing an intra-ocular pressure as follows:**

| No. | Component | Comparative example 53 | Comparative example 54 | Comparative example 55 | Comparative example 56 |
|---|---|---|---|---|---|
| 1. | Netarsudil dimesylate | 14.2 mg | 14.2 mg | 28.5 mg | 28.5 mg |
| 2. | Maleic acid | 91.8 mg | 183.5 mg | 91.8 mg | 183.5 mg |
| 3. | Tafluprost | 1.5 mg | 1.5 mg | 1.5 mg | 1.5 mg |
| 4. | Boric acid | 50 mg | 50 mg | 50 mg | 50 mg |
| 5. | Mannitol | 5000 mg | 5000 mg | 5000 mg | 5000 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 248. Contents of various main medicine components in Comparative examples 53 to 56 after being stored at 5°Cfor 7 days**

| No. | Component | Comparative example 53 | Comparative example 54 | Comparative example 55 | Comparative example 56 |
|---|---|---|---|---|---|
| | | Content % | | | |
| 1. | Netarsudil dimesylate | <30 | <30 | <30 | <30 |
| 2. | Tafluprost | 99.9 | 99.3 | 99.9 | 100.1 |

According to Comparative examples 53 to 56, the preparation including netarsudil dimesylate, maleic acid and tafluprost has incompatibility under the storage condition of 5°C, and netarsudil dimesylate is precipitated.

### Embodiments 147 to 151: a preparation including netarsudil dimesylate, carteolol mesylate and latanoprost

**Table 249. Preparation of a local ophthalmic medicine composition solution for reducing an intra-ocular pressure through a conventional method as follows:**

| No. | Component | Embodiment 147 | Embodiment 148 | Embodiment 149 | Embodiment 150 | Embodiment 151 |
|---|---|---|---|---|---|---|
| 1. | Netarsudil dimesylate | 14.2 mg | 14.2 mg | 28.5 mg | 28.5 mg | 57.0 mg |
| 2. | Carteolol mesylate | 1329 mg | 2658 mg | 1329 mg | 2658 mg | 5316 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 250. Contents of various main medicine components in Embodiments 147 to 151 after being stored at 5°Cfor 24 months**

| No. | Component | Embodiment 147 | Embodiment 148 | Embodiment 149 | Embodiment 150 | Embodiment 151 |
|---|---|---|---|---|---|---|
| | | Content % | | | | |
| 1. | Netarsudil dimesylate | 100.1 | 99.5 | 101.1 | 100.9 | 99.7 |
| 2. | Carteolol mesylate | 101.3 | 101.5 | 100.8 | 100.8 | 100.9 |
| 3. | Latanoprost | 100.1 | 99.9 | 99.2 | 99.9 | 100.1 |

**Table 251. Contents of various main medicine components in commercially available eye drops (Mikelan^{®}, Rhopress^{®} and Xalatan^{®}) after being stored at 5°Cfor 24 months**

| No. | Component | Mikelan^{®} | Rhopress^{®} | Xalatan^{®} |
|---|---|---|---|---|
| | | Content % | | |
| 1. | Carteolol hydrochloride | 99.9 | - | - |
| 2. | Netarsudil dimesylate | - | 99.9 | - |
| 3. | Latanoprost | - | - | 99.8 |

According to Embodiments 147 to 151, the preparation including netarsudil dimesylate, carteolol mesylate and latanoprost has good stability after being stored under a storage condition of 5°Cfor 24 months, which meets a requirement for a storage condition of the commercially available eye drops (Mikelan^{®}, Rhopress^{®} andXalatan^{®}) before opening.

**Table 252. Contents of various main medicine components in Embodiments 147 to 151 after being stored at 25°Cfor 6 weeks**

| No. | Component | Embodiment 147 | Embodiment 148 | Embodiment 149 | Embodiment 150 | Embodiment 151 |
|---|---|---|---|---|---|---|
| | | Content % | | | | |
| 1. | Netarsudil dimesylate | 100.0 | 99.1 | 99.9 | 101.1 | 99.9 |
| 2. | Carteolol mesylate | 100.5 | 101.3 | 99.1 | 101.8 | 100.7 |
| 3. | Latanoprost | 100.2 | 99.8 | 98.7 | 99.9 | 100.0 |

**Table 253. Contents of various main medicine components in commercially available eye drops (Mikelan^{®}, Rhopress^{®} and Xalatan^{®}) after being stored at 25°Cfor 6 weeks**

| No. | Component | Mikelan^{®} | Rhopress^{®} | Xalatan^{®} |
|---|---|---|---|---|
| | | Content % | | |
| 1. | Carteolol hydrochloride | 99.9 | - | - |
| 2. | Netarsudil dimesylate | - | 99.8 | - |
| 3. | Latanoprost | - | - | 99.6 |

According to Embodiments 147 to 151, the preparation including netarsudil dimesylate, carteolol mesylate and latanoprost has good stability after being stored under a storage condition of 25°Cfor 6 weeks, which meets a requirement for a storage condition of the commercially available eye drops (Mikelan^{®}, Rhopress^{®} andXalatan^{®}) after opening.

**Table 254. Contents of various main medicine components in Embodiments 147 to 151 after being stored at 40°Cfor 14 days**

| No. | Component | Embodiment 147 | Embodiment 148 | Embodiment 149 | Embodiment 150 | Embodiment 151 |
|---|---|---|---|---|---|---|
| | | Content % | | | | |
| 1. | Netarsudil dimesylate | 99.9 | 98.2 | 98.7 | 99.8 | 99.8 |
| 2. | Carteolol mesylate | 100.0 | 101.9 | 99.9 | 100.4 | 100.9 |
| 3. | Latanoprost | 98.9 | 99.3 | 99.6 | 98.2 | 99.1 |

**Table 255. Contents of various main medicine components in commercially available eye drops (Mikelan^{®}, Rhopress^{®} and Xalatan^{®}) after being stored at 40°Cfor 14 days**

| No. | Component | Mikelan^{®} | Rhopress^{®} | Xalatan^{®} |
|---|---|---|---|---|
| | | Content % | | |
| 1. | Carteolol hydrochloride | 101.1 | - | - |
| 2. | Netarsudil dimesylate | - | 99.5 | - |
| 3. | Latanoprost | - | - | 99.7 |

According to Embodiments 147 to 151, the preparation including netarsudil dimesylate, carteolol mesylate and latanoprost has good stability after being stored under a storage condition of 40°Cfor 14 days, which meets a requirement for departing from a storage condition of the commercially available eye drops (Mikelan^{®}, Rhopress^{®} and Xalatan^{®}) for a short time.

### Embodiments 152 to 156: a preparation including netarsudil dihydrochloride, carteolol hydrochloride and latanoprost

**Table 256. Preparation of a local ophthalmic medicine composition solution for reducing an intra-ocular pressure through a conventional method as follows:**

| No. | Component | Embodiment 152 | Embodiment 153 | Embodiment 154 | Embodiment 155 | Embodiment 156 |
|---|---|---|---|---|---|---|
| 1. | Netarsudil dihydrochloride | 11.6 mg | 11.6 mg | 23.2 mg | 23.2 mg | 46.4 mg |
| 2. | Carteolol hydrochloride | 1000 mg | 2000 mg | 1000 mg | 2000 mg | 4000 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 257. Contents of various main medicine components in Embodiments 152 to 156 after being stored at 5°Cfor 24 months**

| No. | Component | Embodiment 152 | Embodiment 153 | Embodiment 154 | Embodiment 155 | Embodiment 156 |
|---|---|---|---|---|---|---|
| | | Content % | | | | |
| 1. | Netarsudil dihydrochloride | 98.5 | 99.3 | 98.8 | 99.0 | 99.8 |
| 2. | Carteolol hydrochloride | 99.7 | 100.0 | 100.1 | 99.9 | 99.8 |
| 3. | Latanoprost | 99.3 | 100.5 | 99.1 | 100.9 | 100.3 |

According to Embodiments 152 to 156, the preparation including netarsudil dihydrochloride, carteolol hydrochloride and latanoprost has good stability after being stored under a storage condition of 5°Cfor 24 months, which meets a requirement for a storage condition of the commercially available eye drops (Mikelan^{®}, Rhopress^{®} andXalatan^{®}) before opening.

**Table 258. Contents of various main medicine components in Embodiments 152 to 156 after being stored at 25°Cfor 6 weeks**

| No. | Component | Embodiment 70 | Embodiment 71 | Embodiment 72 | Embodiment 73 | Embodiment 74 |
|---|---|---|---|---|---|---|
| | | Content % | | | | |
| 1. | Netarsudil dihydrochloride | 100.1 | 99.9 | 99.4 | 100.3 | 99.8 |
| 2. | Carteolol hydrochloride | 98.6 | 99.5 | 99.9 | 100.4 | 100.2 |
| 3. | Latanoprost | 100.5 | 100.1 | 100.1 | 101.9 | 100.4 |

According to Embodiments 152 to 156, the preparation including netarsudil dihydrochloride, carteolol hydrochloride and latanoprost has good stability after being stored under a storage condition of 25°Cfor 6 weeks, which meets a requirement for a storage condition of the commercially available eye drops (Mikelan^{®}, Rhopress^{®} and Xalatan^{®}) before opening.

**Table 259. Contents of various main medicine components in Embodiments 152 to 156 after being stored at 40°Cfor 14 days**

| No. | Component | Embodiment 152 | Embodiment 153 | Embodiment 154 | Embodiment 155 | Embodiment 156 |
|---|---|---|---|---|---|---|
| | | Content % | | | | |
| 1. | Netarsudil dihydrochloride | 100.5 | 99.3 | 99.6 | 100.9 | 100.0 |
| 2. | Carteolol hydrochloride | 99.9 | 99.7 | 101.2 | 101.8 | 99.6 |
| 3. | Latanoprost | 99.9 | 100.2 | 99.9 | 99.7 | 98.2 |

According to Embodiments 152 to 156, the preparation including netarsudil dihydrochloride, carteolol hydrochloride and latanoprost has good stability after being stored under a storage condition of 40°Cfor 14 days, which meets a requirement for departing from a storage condition of the commercially available eye drops (Mikelan^{®}, Rhopress^{®} and Xalatan^{®}) for a short time.

### Embodiment 157: evaluation of pharmaceutical effects of the ophthalmic medicine composition solution in Embodiments 147 to 156 by using a Japanese white rabbit glaucoma model

### Experimental grouping:

Normal control group: no processing.

Model group: an intraocular hypertension model is established, and no eye drop is administered.

Treatment group 5: an intraocular hypertension model is established, and commercially available latanoprost and netarsudil eye drops (Rocklatan^{®}) are administered.

Treatment group 9: an intraocular hypertension model is established, and commercially available carteolol hydrochloride and latanoprost compound eye drops (Mikeluna^{®}) are administered.

Embodiment group 147: an intraocular hypertension model is established, and an ophthalmic medicine composition solution in Embodiment 147 is administered.

Embodiment group 148: an intraocular hypertension model is established, and an ophthalmic medicine composition solution in Embodiment 148 is administered.

Embodiment group 149: an intraocular hypertension model is established, and an ophthalmic medicine composition solution in Embodiment 149 is administered.

Embodiment group 150: an intraocular hypertension model is established, and an ophthalmic medicine composition solution in Embodiment 150 is administered.

Embodiment group 151: an intraocular hypertension model is established, and an ophthalmic medicine composition solution in Embodiment 151 is administered.

Embodiment group 152: an intraocular hypertension model is established, and an ophthalmic medicine composition solution in Embodiment 152 is administered.

Embodiment group 153: an intraocular hypertension model is established, and an ophthalmic medicine composition solution in Embodiment 153 is administered.

Embodiment group 154: an intraocular hypertension model is established, and an ophthalmic medicine composition solution in Embodiment 154 is administered.

Embodiment group 155: an intraocular hypertension model is established, and an ophthalmic medicine composition solution in Embodiment 155 is administered.

Embodiment group 156: an intraocular hypertension model is established, and an ophthalmic medicine composition solution in Embodiment 156 is administered.

A dosage regimen: according to a current clinical dosage regimen of commercially available latanoprost and netarsudil eye drops (Rocklatan^{®}) and commercially available carteolol hydrochloride and latanoprost compound eye drops (Mikeluna^{®}), this experiment is mainly divided into the following groups: (1) the treatment group 5: 50 µL the commercially available latanoprost and netarsudil eye drops (Rocklatan^{®}) are administered to each eye each time once a day (9:00 PM); (2) the treatment group 9: 50 µL the commercially available carteolol hydrochloride and latanoprost compound eye drops (Mikeluna^{®}) are administered to each eye each time once a day (9:00 PM); and (3) Embodiment groups 147 to 156: 50 µL eye drops in Embodiments 147 to 156 are administered to each eye each time once a day (9:00 PM). Drug administration is started in all the groups on the tenth day when the models are established successfully and lasts for 10 days. Eye drops are not administered to the normal control group and the model group. The dosage regimen is shown in FIG. 13.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 5, the treatment group 9 and Embodiment groups 147, 148, 149, 152, 153 and 154, the IOP can be reduced significantly by the ophthalmic composition solutions of Embodiment groups 150, 151, 155 and 156, however, IOP actual measured values and IOP variation values in Embodiment groups 150, 151, 155 and 156 have no significant difference.

**Table 260. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| IOP variation value (mmHg) | | | | | | | | | | | | | | |
|---|---|---|---|---|---|---|---|---|---|---|---|---|---|---|
| Tim e (d) | Nor mal contr ol grou p | Mod el grou p | Treat ment grou p 5 | Treat ment grou p 9 | Emb odim ent grou p 147 | Emb odim ent grou p 148 | Emb odim ent grou p 149 | Emb odim ent grou p 150 | Emb odim ent grou p 151 | Emb odim ent grou p 152 | Emb odim ent grou p 153 | Emb odim ent grou p 154 | Emb odim ent grou p 155 | Emb odim ent grou p 156 |
| 10d | - | - | - | - | - | - | - | - | - | - | - | - | - | - |
| 15d | 0.50 | 2.25 | 1.50 | -2.48 | -6.12 | -6.56 | -5.50 | -6.22 | -6.80 | -5.70 | -6.40 | -7.40 | -8.40 | -8.10 |
| 20 d | -0.5 | 8.58 | -4.50 | -4.88 | -8.46 | -11.0 | -10.5 | -14.2 | -14.0 | -7.80 | -10.2 | -9.90 | -15.5 | -15.2 |
| 0 | | | | | | | | 0 | 0 | 3 | 0 | 0 | 0 | 0 |

**Table 261. Intra-ocular pressure actual measured values of each group of rabbits measured on the tenth day, the fifteenth day and the twentieth day when drug administration is performed for 10 days on end starting with the tenth day**

| | IOP actual measured value (mmHg) | | | | | | | | | | | | | |
|---|---|---|---|---|---|---|---|---|---|---|---|---|---|---|
| Ti m e (d ) | No rm al co ntr ol gro up | M od el gr ou p | Trea tme nt grou p 5 | Trea tme nt grou p 9 | Embo dime nt group 147 | Embo dime nt group 148 | Embo dime nt group 149 | Embo dime nt group 150 | Embo dime nt group 151 | Embo dime nt group 152 | Embo dime nt group 153 | Embo dime nt group 154 | Embo dime nt group 155 | Embo dime nt group 156 |
| 1 0 d | 6.0 0 | 26 .7 5 | 24.8 3 | 24.8 8 | 25.56 | 26.00 | 25.50 | 24.33 | 24.00 | 25.00 | 25.40 | 25.00 | 25.50 | 25.20 |
| 1 5 d | 6.5 0 | 29 .0 0 | 26.3 3 | 22.4 0 | 19.44 | 19.44 | 20.00 | 18.11 | 17.20 | 19.30 | 19.00 | 17.60 | 17.10 | 17.10 |
| 2 0 d | 5.5 0 | 35 .3 3 | 20.3 3 | 20.0 0 | 17.10 | 15.00 | 15.00 | 10.10 | 10.00 | 17.20 | 15.20 | 15.10 | 10.00 | 10.00 |

### Embodiments 158 to 162: a preparation including netarsudil dimesylate, carteolol mesylate and bimatoprost

**Table 262. Preparation of a local ophthalmic medicine composition solution for reducing an intra-ocular pressure through a conventional method as follows:**

| No. | Component | Embodiment 158 | Embodiment 159 | Embodiment 160 | Embodiment 161 | Embodiment 162 |
|---|---|---|---|---|---|---|
| 1. | Netarsudil dimesylate | 14.2 mg | 14.2 mg | 28.5 mg | 28.5 mg | 57.0 mg |
| 2. | Carteolol mesylate | 1329 mg | 2658 mg | 1329 mg | 2658 mg | 5316 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 263. Contents of various main medicine components in Embodiments 158 to 162 after being stored at 5°Cfor 24 months**

| No. | Component | Embodiment 158 | Embodiment 159 | Embodiment 160 | Embodiment 161 | Embodiment 162 |
|---|---|---|---|---|---|---|
| | | Content % | | | | |
| 1. | Netarsudil dimesylate | 99.9 | 99.9 | 99.8 | 100.0 | 101.1 |
| 2. | Carteolol mesylate | 100.5 | 100.0 | 99.9 | 100.1 | 100.0 |
| 3. | Bimatoprost | 99.9 | 101.0 | 100.0 | 99.8 | 99.8 |

**Table 264. Contents of various main medicine components in commercially available eye drops (Mikelan^{®}, Rhopress^{®} and Lumigan^{®}) after being stored at 5°Cfor 24 months**

| No. | Component | Mikelan^{®} | Rhopress^{®} | Lumigan^{®} |
|---|---|---|---|---|
| | | Content % | | |
| 1. | Carteolol hydrochloride | 99.9 | - | - |
| 2. | Netarsudil dimesylate | - | 99.9 | - |
| 3. | Bimatoprost | - | - | 99.4 |

According to Embodiments 158 to 162, the preparation including netarsudil dimesylate, carteolol mesylate and bimatoprost has good stability after being stored under a storage condition of 5°Cfor 24 months, which meets a requirement for a storage condition of the commercially available eye drops (Mikelan^{®}, Rhopress^{®} andLumigan^{®}) before opening.

**Table 265. Contents of various main medicine components in Embodiments 158 to 162 after being stored at 25°Cfor 6 weeks**

| No. | Component | Embodiment 158 | Embodiment 159 | Embodiment 160 | Embodiment 161 | Embodiment 162 |
|---|---|---|---|---|---|---|
| | | Content % | | | | |
| 1. | Netarsudil dimesylate | 100.0 | 98.1 | 99.6 | 99.3 | 99.9 |
| 2. | Carteolol mesylate | 99.9 | 100.1 | 99.8 | 101.3 | 99.8 |
| 3. | Bimatoprost | 99.5 | 99.3 | 99.9 | 100.0 | 99.5 |

**Table 266. Contents of various main medicine components in commercially available eye drops (Mikelan^{®}, Rhopress^{®} and Lumigan^{®}) after being stored at 25°Cfor 6 weeks**

| No. | Component | Mikelan^{®} | Rhopress^{®} | Lumigan^{®} |
|---|---|---|---|---|
| | | Content % | | |
| 1. | Carteolol hydrochloride | 99.9 | - | - |
| 2. | Netarsudil dimesylate | - | 99.8 | - |
| 3. | Bimatoprost | - | - | 99.5 |

According to Embodiments 158 to 162, the preparation including netarsudil dimesylate, carteolol mesylate and bimatoprost has good stability after being stored under a storage condition of 25°Cfor 6 weeks, which meets a requirement for a storage condition of the commercially available eye drops (Mikelan^{®}, Rhopress^{®} andLumigan^{®}) after opening.

**Table 267. Contents of various main medicine components in Embodiments 158 to 162 after being stored at 40°Cfor 14 days**

| No. | Component | Embodiment 158 | Embodiment 159 | Embodiment 160 | Embodiment 161 | Embodiment 162 |
|---|---|---|---|---|---|---|
| | | Content % | | | | |
| 1. | Netarsudil dimesylate | 99.8 | 98.5 | 100.0 | 99.1 | 99.4 |
| 2. | Carteolol mesylate | 100.5 | 101.9 | 99.9 | 100.8 | 100.3 |
| 3. | Bimatoprost | 99.2 | 99.6 | 100.6 | 100.5 | 99.8 |

**Table 268. Contents of various main medicine components in commercially available eye drops (Mikelan^{®}, Rhopress^{®} and Lumigan^{®}) after being stored at 40°Cfor 14 days**

| No. | Component | Mikelan^{®} | Rhopress^{®} | Lumigan^{®} |
|---|---|---|---|---|
| | | Content % | | |
| 1. | Carteolol hydrochloride | 101.1 | - | - |
| 2. | Netarsudil dimesylate | - | 99.5 | - |
| 3. | Bimatoprost | - | - | 99.2 |

According to Embodiments 158 to 162, the preparation including netarsudil dimesylate, carteolol mesylate and bimatoprost has good stability after being stored under a storage condition of 40°Cfor 14 days, which meets a requirement for departing from a storage condition of the commercially available eye drops (Mikelan^{®}, Rhopress^{®} andLumigan^{®}) for a short time.

### Embodiments 163 to 167: a preparation including netarsudil dihydrochloride, carteolol hydrochloride and bimatoprost

**Table 269. Preparation of a local ophthalmic medicine composition solution for reducing an intra-ocular pressure through a conventional method as follows:**

| No. | Component | Embodiment 163 | Embodiment 164 | Embodiment 165 | Embodiment 166 | Embodiment 167 |
|---|---|---|---|---|---|---|
| 1. | Netarsudil dihydrochloride | 11.6 mg | 11.6 mg | 23.2 mg | 23.2 mg | 46.4 mg |
| 2. | Carteolol hydrochloride | 1000 mg | 2000 mg | 1000 mg | 2000 mg | 4000 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 270. Contents of various main medicine components in Embodiments 163 to 167 after being stored at 5°Cfor 24 months**

| No. | Component | Embodiment 163 | Embodiment 164 | Embodiment 165 | Embodiment 166 | Embodiment 167 |
|---|---|---|---|---|---|---|
| | | Content % | | | | |
| 1. | Netarsudil dihydrochloride | 98.1 | 99.9 | 100.6 | 99.1 | 99.3 |
| 2. | Carteolol hydrochloride | 100.0 | 100.3 | 101.4 | 100.0 | 99.8 |
| 3. | Bimatoprost | 101.8 | 99.4 | 99.8 | 101.3 | 100.5 |

According to Embodiments 163 to 167, the preparation including netarsudil dihydrochloride, carteolol hydrochloride and bimatoprost has good stability after being stored under a storage condition of 5°Cfor 24 months, which meets a requirement for a storage condition of the commercially available eye drops (Mikelan^{®}, Rhopress^{®} andLumigan^{®}) before opening.

**Table 271. Contents of various main medicine components in Embodiments 163 to 167 after being stored at 25°Cfor 6 weeks**

| No. | Component | Embodiment 163 | Embodiment 164 | Embodiment 165 | Embodiment 166 | Embodiment 167 |
|---|---|---|---|---|---|---|
| | | Content % | | | | |
| 1. | Netarsudil dihydrochloride | 99.2 | 100.0 | 99.1 | 101.5 | 99.6 |
| 2. | Carteolol hydrochloride | 100.8 | 99.5 | 100.2 | 99.1 | 100.6 |
| 3. | Bimatoprost | 99.2 | 101.9 | 99.2 | 100.7 | 98.2 |

According to Embodiments 163 to 167, the preparation including netarsudil dihydrochloride, carteolol hydrochloride and bimatoprost has good stability after being stored under a storage condition of 25°Cfor 6 weeks, which meets a requirement for a storage condition of the commercially available eye drops (Mikelan^{®}, Rhopress^{®} and Lumigan^{®}) after opening.

**Table 272. Contents of various main medicine components in Embodiments 163 to 167 after being stored at 40°Cfor 14 days**

| No. | Component | Embodiment 81 | Embodiment 82 | Embodiment 83 | Embodiment 84 | Embodiment 85 |
|---|---|---|---|---|---|---|
| | | Content % | | | | |
| 1. | Netarsudil dihydrochloride | 100.7 | 99.3 | 101.8 | 99.2 | 100.2 |
| 2. | Carteolol hydrochloride | 98.7 | 100.5 | 99.7 | 100.0 | 99.1 |
| 3. | Bimatoprost | 100.3 | 98.2 | 101.5 | 99.3 | 100.4 |

According to Embodiments 163 to 167, the preparation including netarsudil dihydrochloride, carteolol hydrochloride and bimatoprost has good stability after being stored under a storage condition of 40°Cfor 14 days, which meets a requirement for departing from a storage condition of the commercially available eye drops (Mikelan^{®}, Rhopress^{®} and Lumigan^{®}) for a short time.

### Embodiment 168: evaluation of pharmaceutical effects of the ophthalmic medicine composition solutions in Embodiments 158 to 167 by using a Japanese white rabbit glaucoma model

### Experimental grouping:

Normal control group: no processing.

Model group: an intraocular hypertension model is established, and no eye drop is administered.

Treatment group 5: an intraocular hypertension model is established, and commercially available latanoprost and netarsudil eye drops (Rocklatan^{®}) are administered.

Treatment group 6: an intraocular hypertension model is established, and commercially available bimatoprost and timolol maleate eye drops (Ganfort^{®}) are administered.

Embodiment group 158: an intraocular hypertension model is established, and an ophthalmic medicine composition solution in Embodiment 158 is administered.

Embodiment group 159: an intraocular hypertension model is established, and an ophthalmic medicine composition solution in Embodiment 159 is administered.

Embodiment group 160: an intraocular hypertension model is established, and an ophthalmic medicine composition solution in Embodiment 160 is administered.

Embodiment group 161: an intraocular hypertension model is established, and an ophthalmic medicine composition solution in Embodiment 161 is administered.

Embodiment group 162: an intraocular hypertension model is established, and an ophthalmic medicine composition solution in Embodiment 162 is administered.

Embodiment group 163: an intraocular hypertension model is established, and an ophthalmic medicine composition solution in Embodiment 163 is administered.

Embodiment group 164: an intraocular hypertension model is established, and an ophthalmic medicine composition solution in Embodiment 164 is administered.

Embodiment group 165: an intraocular hypertension model is established, and an ophthalmic medicine composition solution in Embodiment 165 is administered.

Embodiment group 166: an intraocular hypertension model is established, and an ophthalmic medicine composition solution in Embodiment 166 is administered.

Embodiment group 167: an intraocular hypertension model is established, and an ophthalmic medicine composition solution in Embodiment 167 is administered.

A dosage regimen: according to a current clinical dosage regimen of commercially available latanoprost and netarsudil eye drops (Rocklatan^{®}) and commercially available bimatoprost and timolol maleate eye drops (Ganfort^{®}), this experiment is mainly divided into the following groups: (1) the treatment group 5: 50 µLf the commercially available latanoprost and netarsudil eye drops (Rocklatan^{®}) are administered to each eye each time once a day (9:00 PM); (2) the treatment group 6: 50 µL the commercially available bimatoprost and timolol maleate eye drops (Ganfort^{®}) are administered to each eye each time once a day (9:00 PM); and (3) Embodiment groups 158 to 167: 50 µL eye drops in Embodiments 158 to 167 are administered to each eye each time once a day (9:00 PM). Drug administration is started in all the groups on the tenth day when the models are established successfully and lasts for 10 days. Eye drops are not administered to the normal control group and the model group. The dosage regimen is shown in FIG. 15.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 5, the treatment group 6 and Embodiment groups 158, 159, 160, 163, 164 and 165, the IOP can be reduced significantly by the ophthalmic composition solutions of Embodiment groups 161, 162, 166 and 167, however, IOP actual measured values and IOP variation values in Embodiment groups 161, 162, 166 and 167 have no significant difference.

**Table 273. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| Ti m e (d ) | IOP variation value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 5 | Trea tme nt grou p 6 | Embo dime nt group 158 | Embo dime nt group 159 | Embo dime nt group 160 | Embo dime nt group 161 | Embo dime nt group 162 | Embo dime nt group 163 | Embo dime nt group 164 | Embo dime nt group 165 | Embo dime nt group 166 | Embo dime nt group 167 |
| 1 0 d | - | - | - | - | - | - | - | - | - | - | - | - | - | - |
| 1 5 d | 0.5 0 | 2. 25 | 1.50 | -0.8 3 | -2.44 | -6.70 | -7.39 | -7.90 | -7.30 | -3.38 | -5.45 | -4.47 | -7.25 | -7.70 |
| 2 0 d | -0. 50 | 8. 58 | -4.5 0 | -4.5 0 | -7.07 | -9.30 | -10.4 0 | -14.7 7 | -14.1 9 | -8.38 | -10.3 5 | -9.50 | -14.5 7 | -15.0 0 |

**Table 274. Intra-ocular pressure actual measured values of each group of rabbits measured on the tenth day, the fifteenth day and the twentieth day when drug administration is performed for 10 days on end starting with the tenth day**

| Time | IOP actual measured value (mmHg) | | | | | | | | |
|---|---|---|---|---|---|---|---|---|---|
| | Normal control group | Model group | Treatment group 5 | Treatment group 6 | Embodiment group 158 | Embodiment group 159 | Embodiment group 160 | Embodiment group 161 | Embodime group 162 |
| 10 d | 6.00 | 26.75 | 24.83 | 24.00 | 25.00 | 24.50 | 25.50 | 26.00 | 25.30 |
| 15 d | 6.50 | 29.00 | 26.33 | 23.17 | 22.56 | 17.80 | 18.11 | 18.10 | 18.00 |
| 20 d | 5.50 | 35.33 | 20.33 | 19.50 | 16.93 | 15.20 | 15.10 | 11.23 | 11.11 |

### Embodiments 169 to 173: a preparation including netarsudil dimesylate, carteolol mesylate and travoprost

**Table 275. Preparation of a local ophthalmic medicine composition solution for reducing an intra-ocular pressure through a conventional method as follows:**

| No. | Component | Embodiment 169 | Embodiment 170 | Embodiment 171 | Embodiment 172 | Embodiment 173 |
|---|---|---|---|---|---|---|
| 1. | Netarsudil dimesylate | 14.2 mg | 14.2 mg | 28.5 mg | 28.5 mg | 57.0 mg |
| 2. | Carteolol mesylate | 1329 mg | 2658 mg | 1329 mg | 2658 mg | 5316 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 276. Contents of various main medicine components in Embodiments 169 to 173 after being stored at 5°Cfor 24 months**

| No. | Component | Embodiment 169 | Embodiment 170 | Embodiment 171 | Embodiment 172 | Embodiment 173 |
|---|---|---|---|---|---|---|
| | | Content % | | | | |
| 1. | Netarsudil dimesylate | 99.4 | 100.1 | 99.1 | 100.3 | 99.9 |
| 2. | Carteolol mesylate | 100.0 | 98.2 | 100.8 | 99.3 | 98.4 |
| 3. | Travoprost | 99.9 | 98.9 | 100.1 | 99.1 | 99.8 |

**Table 277. Contents of various main medicine components in commercially available eye drops (Mikelan^{®}, Rhopress^{®} and Travatan^{®}) after being stored at 5°Cfor 24 months**

| No. | Component | Mikelan^{®} | Rhopress^{®} | Travatan^{®} |
|---|---|---|---|---|
| | | Content % | | |
| 1. | Carteolol hydrochloride | 99.9 | - | - |
| 2. | Netarsudil dimesylate | - | 99.9 | - |
| 3. | Travoprost | - | - | 99.8 |

According to Embodiments 169 to 173, the preparation including netarsudil dimesylate, carteolol mesylate and travoprost has good stability after being stored under a storage condition of 5°Cfor 24 months, which meets a requirement for a storage condition of the commercially available eye drops (Mikelan^{®}, Rhopress^{®} andTravatan^{®}) before opening.

**Table 278. Contents of various main medicine components in Embodiments 169 to 173 after being stored at 25°Cfor 6 weeks**

| No. | Component | Embodiment 169 | Embodiment 170 | Embodiment 171 | Embodiment 172 | Embodiment 173 |
|---|---|---|---|---|---|---|
| | | Content % | | | | |
| 1. | Netarsudil dimesylate | 100.1 | 99.1 | 100.5 | 100.3 | 99.5 |
| 2. | Carteolol mesylate | 99.2 | 100.0 | 99.3 | 99.9 | 100.1 |
| 3. | Travoprost | 101.5 | 99.9 | 99.8 | 100.2 | 100.3 |

**Table 279. Contents of various main medicine components in commercially available eye drops (Mikelan^{®}, Rhopress^{®} and Travatan^{®}) after being stored at 25°Cfor 6 weeks**

| No. | Component | Mikelan^{®} | Rhopress^{®} | Travatan^{®} |
|---|---|---|---|---|
| | | Content % | | |
| 1. | Carteolol hydrochloride | 99.9 | - | - |
| 2. | Netarsudil dimesylate | - | 99.8 | - |
| 3. | Travoprost | - | - | 99.6 |

According to Embodiments 169 to 173, the preparation including netarsudil dimesylate, carteolol mesylate and travoprost has good stability after being stored under a storage condition of 25°Cfor 6 weeks, which meets a requirement for a storage condition of the commercially available eye drops (Mikelan^{®}, Rhopress^{®} and Travatan^{®}) after opening.

**Table 280. Contents of various main medicine components in Embodiments 169 to 173 after being stored at 40°Cfor 14 days**

| No. | Component | Embodiment 169 | Embodiment 170 | Embodiment 171 | Embodiment 172 | Embodiment 173 |
|---|---|---|---|---|---|---|
| | | Content % | | | | |
| 1. | Netarsudil dimesylate | 99.6 | 98.9 | 100.2 | 98.3 | 99.1 |
| 2. | Carteolol mesylate | 99.8 | 100.3 | 99.7 | 99.8 | 100.0 |
| 3. | Travoprost | 100.7 | 99.4 | 99.8 | 100.1 | 99.5 |

**Table 281. Contents of various main medicine components in commercially available eye drops (Mikelan^{®}, Rhopress^{®} and Travatan^{®}) after being stored at 40°Cfor 14 days**

| No. | Component | Mikelan^{®} | Rhopress^{®} | Travatan^{®} |
|---|---|---|---|---|
| | | Content % | | |
| 1. | Carteolol hydrochloride | 101.1 | - | - |
| 2. | Netarsudil dimesylate | - | 99.5 | - |
| 3. | Travoprost | - | - | 99.7 |

According to Embodiments 169 to 173, the preparation including netarsudil dimesylate, carteolol mesylate and travoprost has good stability after being stored under a storage condition of 40°Cfor 14 days, which meets a requirement for departing from a storage condition of the commercially available eye drops (Mikelan^{®}, Rhopress^{®} and Travatan^{®}) for a short time.

### Embodiments 174 to 178: a preparation including netarsudil dihydrochloride, carteolol hydrochloride and travoprost

**Table 282. Preparation of a local ophthalmic medicine composition solution for reducing an intra-ocular pressure through a conventional method as follows:**

| No. | Component | Embodiment 174 | Embodiment 175 | Embodiment 176 | Embodiment 177 | Embodiment 178 |
|---|---|---|---|---|---|---|
| 1. | Netarsudil dihydrochloride | 11.6 mg | 11.6 mg | 23.2 mg | 23.2 mg | 46.4 mg |
| 2. | Carteolol hydrochloride | 1000 mg | 2000 mg | 1000 mg | 2000 mg | 4000 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 283. Contents of various main medicine components in Embodiments 174 to 178 after being stored at 5°Cfor 24 months**

| No. | Component | Embodiment 174 | Embodiment 175 | Embodiment 176 | Embodiment 177 | Embodiment 178 |
|---|---|---|---|---|---|---|
| | | Content % | | | | |
| 1. | Netarsudil dihydrochloride | 99.8 | 99.9 | 100.3 | 98.60 | 99.10 |
| 2. | Carteolol hydrochloride | 100.1 | 99.9 | 101.1 | 98.9 | 101.2 |
| 3. | Travoprost | 99.9 | 100.8 | 99.3 | 100.2 | 99.7 |

According to Embodiments 174 to 178, the preparation including netarsudil dihydrochloride, carteolol hydrochloride and travoprost has good stability after being stored under a storage condition of 5°Cfor 24 months, which meets a requirement for a storage condition of the commercially available eye drops (Mikelan^{®}, Rhopress^{®} and Travatan^{®}) before opening.

**Table 284. Contents of various main medicine components in Embodiments 174 to 178 after being stored at 25°Cfor 6 weeks**

| No. | Component | Embodiment 174 | Embodiment 175 | Embodiment 176 | Embodiment 177 | Embodiment 178 |
|---|---|---|---|---|---|---|
| | | Content % | | | | |
| 1. | Netarsudil dihydrochloride | 100.5 | 99.1 | 100.3 | 99.5 | 99.9 |
| 2. | Carteolol hydrochloride | 99.9 | 98.9 | 100.5 | 100.1 | 100.2 |
| 3. | Travoprost | 99.3 | 99.8 | 99.7 | 99.8 | 99.9 |

According to Embodiments 174 to 178, the preparation including netarsudil dihydrochloride, carteolol hydrochloride and travoprost has good stability after being stored under a storage condition of 25°Cfor 6 weeks, which meets a requirement for a storage condition of the commercially available eye drops (Mikelan^{®}, Rhopress^{®} and Travatan^{®}) after opening.

**Table 285. Contents of various main medicine components in Embodiments 174 to 178 after being stored at 40°Cfor 14 days**

| No. | Component | Embodiment 174 | Embodiment 175 | Embodiment 176 | Embodiment 177 | Embodiment 178 |
|---|---|---|---|---|---|---|
| | | Content % | | | | |
| 1. | Netarsudil dihydrochloride | 99.8 | 99.9 | 101.2 | 100.8 | 100.2 |
| 2. | Carteolol hydrochloride | 100.2 | 100.4 | 100.2 | 99.9 | 99.3 |
| 3. | Travoprost | 99.9 | 99.2 | 100.3 | 99.5 | 100.1 |

According to Embodiments 174 to 178, the preparation including netarsudil dihydrochloride, carteolol hydrochloride and travoprost has good stability after being stored under a storage condition of 40°Cfor 14 days, which meets a requirement for departing from a storage condition of the commercially available eye drops (Mikelan^{®}, Rhopress^{®} and Travatan^{®}) for a short time.

### Embodiment 179: evaluation of pharmaceutical effects of the ophthalmic medicine composition solutions in Embodiments 169 to 178 by using a Japanese white rabbit glaucoma model

### Experimental grouping:

Normal control group: no processing.

Model group: an intraocular hypertension model is established, and no eye drop is administered.

Treatment group 5: an intraocular hypertension model is established, and commercially available latanoprost and netarsudil eye drops (Rocklatan^{®}) are administered.

Treatment group 7: an intraocular hypertension model is established, and commercially available travoprost and timolol maleate eye drops (DuoTrav^{®}) are administered.

Embodiment group 169: an intraocular hypertension model is established, and an ophthalmic medicine composition solution in Embodiment 169 is administered.

Embodiment group 170: an intraocular hypertension model is established, and an ophthalmic medicine composition solution in Embodiment 170 is administered.

Embodiment group 171: an intraocular hypertension model is established, and an ophthalmic medicine composition solution in Embodiment 171 is administered.

Embodiment group 172: an intraocular hypertension model is established, and an ophthalmic medicine composition solution in Embodiment 172 is administered.

Embodiment group 173: an intraocular hypertension model is established, and an ophthalmic medicine composition solution in Embodiment 173 is administered.

Embodiment group 174: an intraocular hypertension model is established, and an ophthalmic medicine composition solution in Embodiment 174 is administered.

Embodiment group 175: an intraocular hypertension model is established, and an ophthalmic medicine composition solution in Embodiment 175 is administered.

Embodiment group 176: an intraocular hypertension model is established, and an ophthalmic medicine composition solution in Embodiment 176 is administered.

Embodiment group 177: an intraocular hypertension model is established, and an ophthalmic medicine composition solution in Embodiment 177 is administered.

Embodiment group 178: an intraocular hypertension model is established, and an ophthalmic medicine composition solution in Embodiment 178 is administered.

A dosage regimen: according to a current clinical dosage regimen of commercially available latanoprost and netarsudil eye drops (Rocklatan^{®}) and commercially available travoprost and timolol maleate eye drops (DuoTrav^{®}), this experiment is mainly divided into the following groups: (1) the treatment group 5: 50 µL the commercially available latanoprost and netarsudil eye drops (Rocklatan^{®}) are administered to each eye each time once a day (9:00 PM); (2) the treatment group 7: 50 µL the commercially available travoprost and timolol maleate eye drops (DuoTrav^{®}) is administered to each eye each time once a day (9:00 PM); and (3) Embodiment groups 169 to 178: 50 µL eye drops in Embodiments 169 to 178 are administered to each eye each time once a day (9:00 PM). Drug administration is started in all the groups on the tenth day when the models are established successfully and lasts for 10 days. Eye drops are not administered to the normal control group and the model group. The dosage regimen is shown in FIG. 17.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 5, the treatment group 7 and Embodiment groups 169, 170, 171, 174, 175 and 176, the IOP can be reduced significantly by the ophthalmic composition solutions of Embodiment groups 172, 173, 177 and 178, however, IOP actual measured values and IOP variation values in Embodiment groups 172, 173, 177 and 178 have no significant difference.

**Table 286. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| Ti m e (d ) | IOP variation value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 5 | Trea tme nt grou p 7 | Embo dime nt group 169 | Embo dime nt group 170 | Embo dime nt group 171 | Embo dime nt group 172 | Embo dime nt group 173 | Embo dime nt group 174 | Embo dime nt group 175 | Embo dime nt group 176 | Embo dime nt group 177 | Embo dime nt group 178 |
| 1 0 d | - | - | - | - | - | - | - | - | - | - | - | - | - | - |
| 1 5 d | 0.5 0 | 2. 25 | 1.50 | -4.0 0 | -3.05 | -4.67 | -5.40 | -8.60 | -9.60 | -3.49 | -6.55 | -6.20 | -8.65 | -9.40 |
| 2 0 d | -0. 50 | 8. 58 | -4.5 0 | -5.7 0 | -8.20 | -9.50 | -10.3 0 | -14.7 0 | -13.9 0 | -8.70 | -10.3 9 | -10.8 0 | -14.9 5 | -15.3 8 |

**Table 287. Intra-ocular pressure actual measured values of each group of rabbits measured on the tenth day, the fifteenth day and the twentieth day when drug administration is performed for 10 days on end starting with the tenth day**

| Ti m e (d ) | IOP actual measured value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 5 | Trea tme nt grou p 7 | Embo dime nt group 169 | Embo dime nt group 170 | Embo dime nt group 171 | Embo dime nt group 172 | Embo dime nt group 173 | Embo dime nt group 174 | Embo dime nt group 175 | Embo dime nt group 176 | Embo dime nt group 177 | Embo dime nt group 178 |
| 1 0 d | 6.0 0 | 26 .7 5 | 24.8 3 | 25.0 0 | 25.00 | 24.80 | 25.30 | 25.10 | 24.80 | 24.60 | 26.50 | 25.70 | 25.15 | 25.50 |
| 1 5 d | 6.5 0 | 29 .0 0 | 26.3 3 | 21.0 0 | 21.95 | 20.13 | 19.90 | 16.50 | 15.20 | 21.11 | 19.95 | 19.50 | 16.50 | 16.10 |
| 2 | 5.5 | 35 | 20.3 | 19.3 | 16.80 | 15.30 | 15.00 | 10.40 | 10.90 | 15.90 | 16.11 | 14.90 | 10.20 | 10.12 |
| 0 d | 0 | .3 3 | 3 | 0 | | | | | | | | | | |

### Embodiments 180 to 184: a preparation including netarsudil dimesylate, carteolol mesylate and tafluprost

**Table 288. Preparation of a local ophthalmic medicine composition solution for reducing an intra-ocular pressure through a conventional method as follows:**

| No. | Component | Embodiment 180 | Embodiment 181 | Embodiment 182 | Embodiment 183 | Embodiment 184 |
|---|---|---|---|---|---|---|
| 1. | Netarsudil dimesylate | 14.2 mg | 14.2 mg | 28.5 mg | 28.5 mg | 57.0 mg |
| 2. | Carteolol mesylate | 1329 mg | 2658 mg | 1329 mg | 2658 mg | 5316 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 289. Contents of various main medicine components in Embodiments 180 to 184 after being stored at 5°Cfor 24 months**

| No. | Component | Embodiment 180 | Embodiment 181 | Embodiment 182 | Embodiment 183 | Embodiment 184 |
|---|---|---|---|---|---|---|
| | | Content % | | | | |
| 1. | Netarsudil dimesylate | 100.0 | 99.1 | 100.4 | 99.8 | 100.3 |
| 2. | Carteolol mesylate | 99.2 | 99.8 | 99.9 | 99.3 | 99.5 |
| 3. | Tafluprost | 99.9 | 100.3 | 99.1 | 100.6 | 100.2 |

**Table 290. Contents of various main medicine components in commercially available eye drops (Mikelan^{®}, Rhopress^{®} and Tapros^{®}) after being stored at 5°Cfor 24 months**

| No. | Component | Mikelan^{®} | Rhopress^{®} | Tapros^{®} |
|---|---|---|---|---|
| | | Content % | | |
| 1. | Carteolol hydrochloride | 99.9 | - | - |
| 2. | Netarsudil dimesylate | - | 99.9 | - |
| 3. | Tafluprost | - | - | 99.4 |

According to Embodiments 180 to 184, the preparation including netarsudil dimesylate, carteolol mesylate and tafluprost has good stability after being stored under a storage condition of 5°Cfor 24 months, which meets a requirement for a storage condition of the commercially available eye drops (Mikelan^{®}, Rhopress^{®} and Tapros^{®}) before opening.

**Table 291. Contents of various main medicine components in Embodiments 180 to 184 after being stored at 25°Cfor 6 weeks**

| No. | Component | Embodiment 180 | Embodiment 181 | Embodiment 182 | Embodiment 183 | Embodiment 184 |
|---|---|---|---|---|---|---|
| | | Content % | | | | |
| 1. | Netarsudil dimesylate | 99.3 | 100.5 | 101.8 | 99.1 | 99.8 |
| 2. | Carteolol mesylate | 98.1 | 99.6 | 99.9 | 99.3 | 100.2 |
| 3. | Tafluprost | 99.1 | 99.3 | 99.5 | 99.9 | 100.4 |

**Table 292. Contents of various main medicine components in commercially available eye drops (Mikelan^{®}, Rhopress^{®} andTapros^{®}) after being stored at 25°Cfor 6 weeks**

| No. | Component | Mikelan^{®} | Rhopress^{®} | Tapros^{®} |
|---|---|---|---|---|
| | | Content % | | |
| 1. | Carteolol hydrochloride | 99.9 | - | - |
| 2. | Netarsudil dimesylate | - | 99.8 | - |
| 3. | Tafluprost | - | - | 99.3 |

According to Embodiments 180 to 184, the preparation including netarsudil dimesylate, carteolol mesylate and tafluprost has good stability after being stored under a storage condition of 25°Cfor 6 weeks, which meets a requirement for a storage condition of the commercially available eye drops (Mikelan^{®}, Rhopress^{®} andTapros^{®}) after opening.

**Table 293. Contents of various main medicine components in Embodiments 180 to 184 after being stored at 40°Cfor 14 days**

| No. | Component | Embodiment 180 | Embodiment 181 | Embodiment 182 | Embodiment 183 | Embodiment 184 |
|---|---|---|---|---|---|---|
| | | Content % | | | | |
| 1. | Netarsudil dimesylate | 99.5 | 100.2 | 99.9 | 100.7 | 100.2 |
| 2. | Carteolol mesylate | 99.1 | 100.3 | 99.2 | 101.3 | 100.0 |
| 3. | Tafluprost | 99.9 | 100.2 | 99.1 | 99.2 | 100.6 |

**Table 294. Contents of various main medicine components in commercially available eye drops (Mikelan^{®}, Rhopress^{®} and Tapros^{®}) after being stored at 40°Cfor 14 days**

| No. | Component | Mikelan^{®} | Rhopress^{®} | Tapros^{®} |
|---|---|---|---|---|
| | | Content % | | |
| 1. | Carteolol hydrochloride | 101.1 | - | - |
| 2. | Netarsudil dimesylate | - | 99.5 | - |
| 3. | Tafluprost | - | - | 99.8 |

According to Embodiments 180 to 184, the preparation including netarsudil dimesylate, carteolol mesylate and tafluprost has good stability after being stored under a storage condition of 40°Cfor 14 days, which meets a requirement for departing from a storage condition of the commercially available eye drops (Mikelan^{®}, Rhopress^{®} and Tapros^{®}) for a short time.

### Embodiments 185 to 189: a preparation including netarsudil dihydrochloride, carteolol hydrochloride and tafluprost

**Table 295. Preparation of a local ophthalmic medicine composition solution for reducing an intra-ocular pressure through a conventional method as follows:**

| No. | Component | Embodiment 185 | Embodiment 186 | Embodiment 187 | Embodiment 188 | Embodiment 189 |
|---|---|---|---|---|---|---|
| 1. | Netarsudil dihydrochloride | 11.6 mg | 11.6 mg | 23.2 mg | 23.2 mg | 46.4 mg |
| 2. | Carteolol hydrochloride | 1000 mg | 2000 mg | 1000 mg | 2000 mg | 4000 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 3400 mg | 3400 mg | 3400 mg | 3400 mg | 3400 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 296. Contents of various main medicine components in Embodiments 185 to 189 after being stored at 5°Cfor 24 months**

| No. | Component | Embodiment 185 | Embodiment 186 | Embodiment 187 | Embodiment 188 | Embodiment 189 |
|---|---|---|---|---|---|---|
| | | Content % | | | | |
| 1. | Netarsudil dihydrochloride | 99.9 | 99.3 | 100.3 | 99.8 | 99.5 |
| 2. | Carteolol hydrochloride | 100.5 | 100.3 | 99.6 | 99.4 | 101.2 |
| 3. | Tafluprost | 99.7 | 101.6 | 100.9 | 100.3 | 101.9 |

According to Embodiments 185 to 189, the preparation including netarsudil dihydrochloride, carteolol hydrochloride and tafluprost has good stability after being stored under a storage condition of 5°Cfor 24 months, which meets a requirement for a storage condition of the commercially available eye drops (Mikelan^{®}, Rhopress^{®} and Taprost^{®}) before opening.

**Table 297. Contents of various main medicine components in Embodiments 185 to 189 after being stored at 25°Cfor 6 weeks**

| | | | | | | |
|---|---|---|---|---|---|---|
| No. | Component | Embodiment 185 | Embodiment 186 | Embodiment 187 | Embodiment 188 | Embodiment 189 |

| | | Content % | | | | |
|---|---|---|---|---|---|---|
| 1. | Netarsudil dihydrochloride | 99.3 | 101.1 | 100.4 | 99.4 | 100.6 |
| 2. | Carteolol hydrochloride | 100.7 | 99.9 | 100.2 | 99.1 | 100.4 |
| 3. | Tafluprost | 99.8 | 100.0 | 99.9 | 98.7 | 99.5 |

According to Embodiments 185 to 189, the preparation including netarsudil dihydrochloride, carteolol hydrochloride and tafluprost has good stability after being stored under a storage condition of 25°Cfor 6 weeks, which meets a requirement for a storage condition of the commercially available eye drops (Mikelan^{®}, Rhopress^{®} and Tapros^{®}) after opening.

**Table 298. Contents of various main medicine components in Embodiments 185 to 189 after being stored at 40°Cfor 14 days**

| No. | Component | Embodiment 185 | Embodiment 186 | Embodiment 187 | Embodiment 188 | Embodiment 189 |
|---|---|---|---|---|---|---|
| | | Content % | | | | |
| 1. | Netarsudil dihydrochloride | 99.4 | 100.3 | 98.3 | 99.1 | 100.9 |
| 2. | Carteolol hydrochloride | 99.9 | 100.2 | 99.4 | 99.8 | 100.1 |
| 3. | Tafluprost | 100.4 | 99.8 | 100.2 | 101.2 | 99.9 |

According to Embodiments 185 to 189, the preparation including netarsudil dihydrochloride, carteolol hydrochloride and tafluprost has good stability after being stored under a storage condition of 40°Cfor 14 days, which meets a requirement for departing from a storage condition of the commercially available eye drops (Mikelan^{®}, Rhopress^{®} and Tapros^{®}) for a short time.

### Embodiment 190: evaluation of pharmaceutical effects of the ophthalmic medicine composition solutions in Embodiments 180 to 189 by using a Japanese white rabbit glaucoma model

### Experimental grouping:

Normal control group: no processing.

Model group: an intraocular hypertension model is established, and no eye drop is administered.

Treatment group 5: an intraocular hypertension model is established, and commercially available latanoprost and netarsudil eye drops (Rocklatan^{®}) are administered.

Treatment group 8: an intraocular hypertension model is established, and commercially available tafluprost and timolol maleate eye drops (Tapcom^{®}) are administered.

Embodiment group 180: an intraocular hypertension model is established, and an ophthalmic medicine composition solution in Embodiment 180 is administered.

Embodiment group 181: an intraocular hypertension model is established, and an ophthalmic medicine composition solution in Embodiment 181 is administered.

Embodiment group 182: an intraocular hypertension model is established, and an ophthalmic medicine composition solution in Embodiment 182 is administered.

Embodiment group 183: an intraocular hypertension model is established, and an ophthalmic medicine composition solution in Embodiment 183 is administered.

Embodiment group 184: an intraocular hypertension model is established, and an ophthalmic medicine composition solution in Embodiment 184 is administered.

Embodiment group 185: an intraocular hypertension model is established, and an ophthalmic medicine composition solution in Embodiment 185 is administered.

Embodiment group 186: an intraocular hypertension model is established, and an ophthalmic medicine composition solution in Embodiment 186 is administered.

Embodiment group 187: an intraocular hypertension model is established, and an ophthalmic medicine composition solution in Embodiment 187 is administered.

Embodiment group 188: an intraocular hypertension model is established, and an ophthalmic medicine composition solution in Embodiment 188 is administered.

Embodiment group 189: an intraocular hypertension model is established, and an ophthalmic medicine composition solution in Embodiment 189 is administered.

A dosage regimen: according to a current clinical dosage regimen of commercially available latanoprost and netarsudil eye drops (Rocklatan^{®}) and commercially available tafluprost and timolol maleate eye drops (Tapcom^{®}), this experiment is mainly divided into the following groups: (1) the treatment group 5: 50 µL the commercially available latanoprost and netarsudil eye drops (Rocklatan^{®}) are administered to each eye each time once a day (9:00 PM); (2) the treatment group 8: 50 µL the commercially available tafluprost and timolol maleate eye drops (Tapcom^{®}) are administered to each eye each time once a day (9:00 PM); and (3) Embodiment groups 180 to 189: 50 µL eye drops in Embodiments 180 to 189 are administered to each eye each time once a day (9:00 PM). Drug administration is started in all the groups on the tenth day when the models are established successfully and lasts for 10 days. Eye drops are not administered to the normal control group and the model group. Eye drops are not administered to the normal control group and the model group. The dosage regimen is shown in FIG. 19.

The rest of experimental operation procedures (experimental animal, model establishment, and a test index and method) may refer to the description of Embodiment 11.

Experiment results:
intra-ocular pressure: IOP values in all the treatment groups and the embodiment groups are reduced significantly at all time points after drug administration is performed during a study period compared with the model group (Student's paired t-test). Meanwhile, compared with the treatment group 5, the treatment group 8 and Embodiment groups 180, 181, 182, 185, 186 and 187, the IOP can be reduced significantly by the ophthalmic composition solutions of Embodiment groups 183, 184, 188 and 189, however, IOP actual measured values and IOP variation values in Embodiment groups 183, 184, 188 and 189 have no significant difference.

**Table 299. Difference values between intra-ocular pressure actual measured values of each group of rabbits measured on the tenth day, the fifteenth day and the twentieth day and an initial intra-ocular pressure (the intra-ocular pressure actual measured value on the tenth day) when drug administration is performed for 10 days on end starting with the tenth day**

| Ti m e (d ) | IOP variation value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 5 | Trea tme nt grou p 8 | Embo dime nt group 180 | Embo dime nt group 181 | Embo dime nt group 182 | Embo dime nt group 183 | Embo dime nt group 184 | Embo dime nt group 185 | Embo dime nt group 186 | Embo dime nt group 187 | Embo dime nt group 188 | Embo dime nt group 189 |
| 1 0 d | - | - | - | - | - | - | - | - | - | - | - | - | - | - |
| 1 5 d | 0.5 0 | 2. 25 | 1.50 | -5.7 0 | -6.47 | -6.78 | -6.90 | -8.70 | -8.10 | -6.00 | -7.30 | -5.84 | -8.05 | -8.95 |
| 2 0 d | -0. 50 | 8. 58 | -4.5 0 | -6.3 0 | -8.47 | -9.79 | -10.0 0 | -14.5 0 | -14.5 0 | -8.00 | -10.7 0 | -10.1 5 | -15.4 5 | -14.7 4 |

**Table 300. Intra-ocular pressure actual measured values of each group of rabbits measured on the tenth day, the fifteenth day and the twentieth day when drug administration is performed for 10 days on end starting with the tenth day**

| Ti m e (d ) | IOP actual measured value (mmHg) | | | | | | | | | | | | | |
|---|---|---|---|---|---|---|---|---|---|---|---|---|---|---|
| | No rm al co ntr ol gro up | M od el gr ou p | Trea tme nt grou p 5 | Trea tme nt grou p 8 | Embo dime nt group 180 | Embo dime nt group 181 | Embo dime nt group 182 | Embo dime nt group 183 | Embo dime nt group 184 | Embo dime nt group 185 | Embo dime nt group 186 | Embo dime nt group 187 | Embo dime nt group 188 | Embo dime nt group 189 |
| 1 0 d | 6.0 0 | 26 .7 5 | 24.8 3 | 26.0 0 | 25.87 | 25.10 | 25.00 | 24.80 | 24.50 | 25.00 | 25.60 | 25.15 | 25.45 | 24.95 |
| 1 5 d | 6.5 0 | 29 .0 0 | 26.3 3 | 20.3 0 | 19.40 | 18.32 | 18.10 | 16.10 | 16.40 | 19.00 | 18.30 | 19.31 | 17.40 | 16.00 |
| 2 0 d | 5.5 0 | 35 .3 3 | 20.3 3 | 19.7 0 | 17.40 | 15.31 | 15.00 | 10.30 | 10.00 | 17.00 | 14.90 | 15.00 | 10.00 | 10.21 |

### Embodiment 191: a preparation including netarsudil dimesylate, carteolol hydrobromide and latanoprost

**Table 301. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol hydrobromide | 2550 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 302. Contents of various main medicine components in Embodiment 191 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.2 | 100.3 |
| 2. | Carteolol hydrobromide | 100.1 | 99.8 |
| 3. | Latanoprost | 99.7 | 100.5 |

According to Embodiment 191, the preparation including netarsudil dimesylate, carteolol hydrobromide and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 192: a preparation including netarsudil dimesylate, carteolol sulfate and latanoprost

**Table 303. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol sulfate | 2680 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 304. Contents of various main medicine components in Embodiment 192 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.8 | 100.4 |
| 2. | Carteolol sulfate | 100.3 | 99.5 |
| 3. | Latanoprost | 99.9 | 100.1 |

According to Embodiment 192, the preparation including netarsudil dimesylate, carteolol sulfate and latanoprost has good stability after being stored under a storage condition of 5°Cfor 24 months, which meets a requirement for a storage condition of the commercially available eye drops (Mikelan^{®}, Rhopress^{®} and Xalatan^{®})before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 193: a preparation including netarsudil dimesylate, carteolol esilate and latanoprost

**Table 305. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol esilate | 3340 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 306. Contents of various main medicine components in Embodiment 193 after being stored at 5°Cfor 24 months and at 25°Cfor 6 weeks**

| No. | Component | 5°C | 25°C |
|---|---|---|---|
| | | Content % | |
| 1. | Netarsudil dimesylate | 101.5 | 100.2 |
| 2. | Carteolol esilate | 99.8 | 100.5 |
| 3. | Latanoprost | 100.1 | 99.1 |

According to Embodiment 193, the preparation including netarsudil dimesylate, carteolol esilate and latanoprost has good stability after being stored under a storage condition of 5°Cfor 24 months, which meets a requirement for a storage condition of the commercially available eye drops (Mikelan^{®}, Rhopress^{®} and Xalatan^{®})before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 194: a preparation including netarsudil dimesylate, carteolol nitrate and latanoprost

**Table 307. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol nitrate | 2430 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 308. Contents of various main medicine components in Embodiment 194 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.9 | 99.1 |
| 2. | Carteolol nitrate | 98.2 | 99.0 |
| 3. | Latanoprost | 100.5 | 100.1 |

According to Embodiment 194, the preparation including netarsudil dimesylate, carteolol nitrate and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 195: a preparation including netarsudil dimesylate, carteolol citrate and latanoprost

**Table 309. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol citrate | 3770 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 310. Contents of various main medicine components in Embodiment 195 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.7 | 100.1 |
| 2. | Carteolol citrate | 99.9 | 99.8 |
| 3. | Latanoprost | 100.4 | 100.0 |

According to Embodiment 195, the preparation including netarsudil dimesylate, carteolol citrate and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 196: a preparation including netarsudil dimesylate, carteolol tartrate and latanoprost

**Table 311. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol tartrate | 3030 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 312. Contents of various main medicine components in Embodiment 196 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.1 | 99.9 |
| 2. | Carteolol tartrate | 99.8 | 99.6 |
| 3. | Latanoprost | 99.1 | 98.2 |

According to Embodiment 196, the preparation including netarsudil dimesylate, carteolol tartrate and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 197: a preparation including netarsudil dimesylate, carteolol salicylate and latanoprost

**Table 313. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol salicylate | 2940 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 314. Contents of various main medicine components in Embodiment 197 after being stored at 5°Cfor 24 months and at 25°Cfor 6 weeks**

| No. | Component | 5°C | 25°C |
|---|---|---|---|
| | | Content % | |
| 1. | Netarsudil dimesylate | 98.4 | 99.2 |
| 2. | Carteolol salicylate | 99.7 | 99.3 |
| 3. | Latanoprost | 100.3 | 100.1 |

According to Embodiment 197, the preparation including netarsudil dimesylate, carteolol salicylate and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 198: a preparation including netarsudil dimesylate, carteolol malate and latanoprost

**Table 315. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol malate | 3690 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 316. Contents of various main medicine components in Embodiment 198 after being stored at 5°Cfor 24 months and at 25°Cfor 6 weeks**

| No. | Component | 5°C | 25°C |
|---|---|---|---|
| | | Content % | |
| 1. | Netarsudil dimesylate | 101.4 | 100.2 |
| 2. | Carteolol malate | 99.3 | 99.8 |
| 3. | Latanoprost | 100.2 | 100.6 |

According to Embodiment 198, the preparation including netarsudil dimesylate, carteolol malate and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 199: a preparation including netarsudil dimesylate, carteolol lactate and latanoprost

**Table 317. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol lactate | 2620 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 318. Contents of various main medicine components in Embodiment 199 after being stored at 5°Cfor 24 months and at 25°Cfor 6 weeks**

| No. | Component | 5°C | 25°C |
|---|---|---|---|
| | | Content % | |
| 1. | Netarsudil dimesylate | 100.3 | 100.0 |
| 2. | Carteolol lactate | 101.9 | 100.4 |
| 3. | Latanoprost | 99.8 | 99.7 |

According to Embodiment 199, the preparation including netarsudil dimesylate, carteolol lactate and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 200: a preparation including netarsudil dimesylate, carteolol phenylacetate and latanoprost

**Table 319. Preparation of a local ophthalmic medicine composition solution for reducing an intra-ocular pressure through a conventional method as follows:**

| No. | Component | Weight |
|---|---|---|
| 1. | Netarsudil dimesylate | 28.5 mg |
| 2. | Carteolol phenylacetate | 3410 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 320. Contents of various main medicine components in Embodiment 200 after being stored at 5°Cfor 24 months and at 25°Cfor 6 weeks**

| No. | Component | 5°C | 25°C |
|---|---|---|---|
| | | Content % | |
| 1. | Netarsudil dimesylate | 99.4 | 99.1 |
| 2. | Carteolol phenylacetate | 99.3 | 99.0 |
| 3. | Latanoprost | 99.6 | 98.6 |

According to Embodiment 200, the preparation including netarsudil dimesylate, carteolol phenylacetate and latanoprost has good stability after being stored under a storage condition of 5°Cfor 24 months, which meets a requirement for a storage condition of the commercially available eye drops (Mikelan^{®}, Rhopress^{®} andXalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 201: a preparation including netarsudil dihydrobromide, carteolol hydrochloride and latanoprost

**Table 321. Preparation of a local ophthalmic medicine composition solution for reducing an intra-ocular pressure through a conventional method as follows:**

| No. | Component | Weight |
|---|---|---|
| 1. | Netarsudil dihydrobromide | 27.1 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 322. Contents of various main medicine components in Embodiment 201 after being stored at 5°Cfor 24 months and at 25°Cfor 6 weeks**

| No. | Component | 5°C | 25°C |
|---|---|---|---|
| | | Content % | |
| 1. | Netarsudil dihydrobromide | 100.0 | 101.3 |
| 2. | Carteolol hydrochloride | 99.9 | 98.2 |
| 3. | Latanoprost | 99.2 | 99.6 |

According to Embodiment 201, the preparation including netarsudil dihydrobromide, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 202: a preparation including netarsudil sulfate, carteolol hydrochloride and latanoprost

**Table 323. Preparation of a local ophthalmic medicine composition solution for reducing an intra-ocular pressure through a conventional method as follows:**

| No. | Component | Weight |
|---|---|---|
| 1. | Netarsudil sulfate | 24.3 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 324. Contents of various main medicine components in Embodiment 202 after being stored at 5°Cfor 24 months and at 25°Cfor 6 weeks**

| No. | Component | 5°C | 25°C |
|---|---|---|---|
| | | Content % | |
| 1. | Netarsudil sulfate | 99.4 | 99.6 |
| 2. | Carteolol hydrochloride | 99.2 | 100.2 |
| 3. | Latanoprost | 100.7 | 100.5 |

According to Embodiment 202, the preparation including netarsudil sulfate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 203: a preparation including netarsudil diformate, carteolol hydrochloride and latanoprost

**Table 325. Preparation of a local ophthalmic medicine composition solution for reducing an intra-ocular pressure through a conventional method as follows:**

| No. | Component | Weight |
|---|---|---|
| 1. | Netarsudil diformate | 24.1 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 326. Contents of various main medicine components in Embodiment 203 after being stored at 5°Cfor 24 months and at 25°Cfor 6 weeks**

| No. | Component | 5°C | 25°C |
|---|---|---|---|
| | | Content % | |
| 1. | Netarsudil diformate | 99.6 | 99.8 |
| 2. | Carteolol hydrochloride | 99.1 | 99.0 |
| 3. | Latanoprost | 99.9 | 99.7 |

According to Embodiment 203, the preparation including netarsudil diformate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 204: a preparation including netarsudil dinitrate, carteolol hydrochloride and latanoprost

**Table 327. Preparation of a local ophthalmic medicine composition solution for reducing an intra-ocular pressure through a conventional method as follows:**

| No. | Component | Weight |
|---|---|---|
| 1. | Netarsudil dinitrate | 25.6 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 328. Contents of various main medicine components in Embodiment 204 after being stored at 5°Cfor 24 months and at 25°Cfor 6 weeks**

| No. | Component | 5°C | 25°C |
|---|---|---|---|
| | | Content % | |
| 1. | Netarsudil dinitrate | 99.3 | 100.5 |
| 2. | Carteolol hydrochloride | 100.7 | 99.9 |
| 3. | Latanoprost | 99.8 | 99.1 |

According to Embodiment 204, the preparation including netarsudil dinitrate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 205: a preparation including netarsudil diacetate, carteolol hydrochloride and latanoprost

**Table 329. Preparation of a local ophthalmic medicine composition solution for reducing an intra-ocular pressure through a conventional method as follows:**

| No. | Component | Weight |
|---|---|---|
| 1. | Netarsudil diacetate | 27.8 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 330. Contents of various main medicine components in Embodiment 205 after being stored at 5°Cfor 24 months and at 25°Cfor 6 weeks**

| No. | Component | 5°C | 25°C |
|---|---|---|---|
| | | Content % | |
| 1. | Netarsudil diacetate | 100.7 | 100.2 |
| 2. | Carteolol hydrochloride | 99.3 | 99.6 |
| 3. | Latanoprost | 98.0 | 99.3 |

According to Embodiment 205, the preparation including netarsudil diacetate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 206: a preparation including netarsudil dibenzoate, carteolol hydrochloride and latanoprost

**Table 331. Preparation of a local ophthalmic medicine composition solution for reducing an intra-ocular pressure through a conventional method as follows:**

| No. | Component | Weight |
|---|---|---|
| 1. | Netarsudil dibenzoate | 30.8 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 332. Contents of various main medicine components in Embodiment 206 after being stored at 5°Cfor 24 months and at 25°Cfor 6 weeks**

| No. | Component | 5°C | 25°C |
|---|---|---|---|
| | | Content % | |
| 1. | Netarsudil dibenzoate | 99.4 | 100.2 |
| 2. | Carteolol hydrochloride | 99.6 | 100.0 |
| 3. | Latanoprost | 98.0 | 99.1 |

According to Embodiment 206, the preparation including netarsudil dibenzoate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 207: a preparation including netarsudil oxalate, carteolol hydrochloride and latanoprost

**Table 333. Preparation of a local ophthalmic medicine composition solution for reducing an intra-ocular pressure through a conventional method as follows:**

| No. | Component | Weight |
|---|---|---|
| 1. | Netarsudil oxalate | 27.9 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 334. Contents of various main medicine components in Embodiment 207 after being stored at 5°Cfor 24 months and at 25°Cfor 6 weeks**

| No. | Component | 5°C | 25°C |
|---|---|---|---|
| | | Content % | |
| 1. | Netarsudil oxalate | 100.6 | 100.3 |
| 2. | Carteolol hydrochloride | 99.8 | 99.3 |
| 3. | Latanoprost | 99.5 | 101.3 |

According to Embodiment 207, the preparation including netarsudil oxalate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 208: a preparation including netarsudil succinate, carteolol hydrochloride and latanoprost

**Table 335. Preparation of a local ophthalmic medicine composition solution for reducing an intra-ocular pressure through a conventional method as follows:**

| No. | Component | Weight |
|---|---|---|
| 1. | Netarsudil succinate | 25.2 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 336. Contents of various main medicine components in Embodiment 208 after being stored at 5°Cfor 24 months and at 25°Cfor 6 weeks**

| No. | Component | 5°C | 25°C |
|---|---|---|---|
| | | Content % | |
| 1. | Netarsudil succinate | 99.5 | 99.0 |
| 2. | Carteolol hydrochloride | 100.7 | 100.1 |
| 3. | Latanoprost | 101.3 | 101.5 |

According to Embodiment 208, the preparation including netarsudil succinate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 209: a preparation including netarsudil diphenylacetate, carteolol hydrochloride and latanoprost

**Table 337. Preparation of a local ophthalmic medicine composition solution for reducing an intra-ocular pressure through a conventional method as follows:**

| No. | Component | Weight |
|---|---|---|
| 1. | Netarsudil diphenylacetate | 32.0 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 338. Contents of various main medicine components in Embodiment 209 after being stored at 5°Cfor 24 months and at 25°Cfor 6 weeks**

| No. | Component | 5°C | 25°C |
|---|---|---|---|
| | | Content % | |
| 1. | Netarsudil diphenylacetate | 100.9 | 100.3 |
| 2. | Carteolol hydrochloride | 99.7 | 99.1 |
| 3. | Latanoprost | 99.0 | 98.3 |

According to Embodiment 209, the preparation including netarsudil diphenylacetate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®}) after opening.

### Embodiment 210: a preparation including netarsudil maleate, carteolol hydrochloride and latanoprost

**Table 339. Preparation of a local ophthalmic medicine composition solution for reducing an intra-ocular pressure through a conventional method as follows:**

| No. | Component | Weight |
|---|---|---|
| 1. | Netarsudil maleate | 25.1 mg |
| 2. | Carteolol hydrochloride | 2000 mg |
| 3. | Latanoprost | 5 mg |
| 4. | Boric acid | 50 mg |
| 5. | Mannitol | 3400 mg |
| 6. | Benzalkonium chloride | 20 mg |
| 7. | Sodium hydroxide | q.s. |
| 8. | Water for injection added by | 100 |

A preparation process may refer to the preparation process in Embodiments 83 to 87.

**Table 340. Contents of various main medicine components in Embodiment 210 after being stored at 5°Cfor 24 months and at 25°Cfor 6 weeks**

| No. | Component | 5°C | 25°C |
|---|---|---|---|
| | | Content % | |
| 1. | Netarsudil maleate | 100.1 | 100.3 |
| 2. | Carteolol hydrochloride | 99.7 | 99.1 |
| 3. | Latanoprost | 99.3 | 99.6 |

According to Embodiment 210, the preparation including netarsudil maleate, carteolol hydrochloride and latanoprost has good stability after being stored under a storage condition of 5°Cfor 24 months, which meets a requirement for a storage condition of the commercially available eye drops (Mikelan^{®}, Rhopress^{®} and Xalatan^{®}) before opening; and has good stability after being stored under a storage condition of 25°Cfor 6 weeks, which meets a requirement for a storage condition of the commercially available eye drops (Mikelan^{®}, Rhopress^{®} and Xalatan^{®})after opening.

### Comparative examples 81 to 84: a preparation including netarsudil dimesylate and carteolol hydrochloride

**Table 341. Preparation of a local ophthalmic medicine composition solution for reducing an intra-ocular pressure through a conventional method as follows:**

| No. | Component | Comparative example 57 | Comparative example 58 | Comparative example 59 | Comparative example 60 |
|---|---|---|---|---|---|
| 1. | Netarsudil dimesylate | 14.2 mg | 14.2 mg | 28.5 mg | 28.5 mg |
| 2. | Carteolol hydrochloride | 1000 mg | 2000 mg | 1000 mg | 2000 mg |
| 3. | Latanoprost | 5 mg | 5 mg | 5 mg | 5 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 342. Contents of various main medicine components in Comparative examples 81 to 84 after being stored at 5°Cfor 7 days**

| No. | Component | Comparative example 57 | Comparative example 58 | Comparative example 59 | Comparative example 60 |
|---|---|---|---|---|---|
| | | Content % | | | |
| 1. | Netarsudil dimesylate | <30 | <30 | <30 | <30 |
| 2. | Carteolol hydrochloride | 99.9 | 100.4 | 100.5 | 100.7 |
| 3. | Latanoprost | 99.7 | 99.6 | 100.1 | 99.5 |

According to Comparative examples 81 to 84: the preparation including netarsudil dimesylate, carteolol hydrochloride and latanoprost has incompatibility under the storage condition of 5°C, and netarsudil dimesylate is precipitated.

### Comparative examples 85 to 88: a preparation including netarsudil dimesylate and hydrochloric acid

**Table 343. Preparation of a local ophthalmic medicine composition solution for reducing an intra-ocular pressure through a conventional method as follows:**

| No. | Component | Comparative example 61 | Comparative example 62 | Comparative example 63 | Comparative example 64 |
|---|---|---|---|---|---|
| 1. | Netarsudil dimesylate | 14.2 mg | 14.2 mg | 28.5 mg | 28.5 mg |
| 2. | Hydrochloric acid | 0.111 mL | 0.188 mL | 0.094 mL | 0.188 mL |
| 3. | Latanoprost | 5 mg | 5 mg | 5 mg | 5 mg |
| 3. | Boric acid | 50 mg | 50 mg | 50 mg | 50 mg |
| 4. | Mannitol | 3400 mg | 3400 mg | 3400 mg | 3400 mg |
| 5. | Benzalkonium chloride | 20 mg | 20 mg | 20 mg | 20 mg |
| 6. | Sodium hydroxide | q.s. | q.s. | q.s. | q.s. |
| 7. | Water for injection added by | 100 mL | 100 mL | 100 mL | 100 |

A preparation process may refer to the preparation process in Embodiments 83 to 87.

**Table 344. Contents of various main medicine components in Comparative examples 85 to 88 after being stored at 5°Cfor 7 days**

| No. | Component | Comparative example 61 | Comparative example 62 | Comparative example 63 | Comparative example 64 |
|---|---|---|---|---|---|
| | | Content % | | | |
| 1. | Netarsudil dimesylate | <30 | <30 | <30 | <30 |
| 2. | Latanoprost | 99.1 | 98.7 | 99.5 | 99.8 |

According to Comparative examples 85 to 88, the preparation including netarsudil dimesylate, hydrochloric acid and latanoprost has incompatibility under the storage condition of 5°C, and netarsudil dimesylate is precipitated.

### Comparative examples 65 to 68: a preparation including netarsudil dimesylate, carteolol hydrochloride and bimatoprost

**Table 345. Preparation of a local ophthalmic medicine composition solution for reducing an intra-ocular pressure as follows:**

| No. | Component | Comparative example 65 | Comparative example 66 | Comparative example 67 | Comparative example 68 |
|---|---|---|---|---|---|
| 1. | Netarsudil dimesylate | 14.2 mg | 14.2 mg | 28.5 mg | 28.5 mg |
| 2. | Carteolol hydrochloride | 1000 mg | 2000 mg | 1000 mg | 2000 mg |
| 3. | Bimatoprost | 30 mg | 30 mg | 30 mg | 30 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 346. Contents of various main medicine components in Comparative examples 65 to 68 after being stored at 5°Cfor 7 days**

| No. | Component | Comparative example 65 | Comparative example 66 | Comparative example 67 | Comparative example 68 |
|---|---|---|---|---|---|
| | | Content % | | | |
| 1. | Netarsudil dimesylate | <30 | <30 | <30 | <30 |
| 2. | Carteolol hydrochloride | 100.1 | 100.3 | 100.0 | 100.2 |
| 3. | Bimatoprost | 99.8 | 101.3 | 100.9 | 99.8 |

According to Comparative examples 65 to 68: the preparation including netarsudil dimesylate, carteolol hydrochloride and bimatoprost has incompatibility under the storage condition of 5°C, and netarsudil dimesylate is precipitated.

### Comparative examples 69 to 72: a preparation including netarsudil dimesylate, hydrochloric acid and bimatoprost

**Table 347. Preparation of a local ophthalmic medicine composition solution for reducing an intra-ocular pressure as follows:**

| No. | Component | Comparative example 69 | Comparative example 70 | Comparative example 71 | Comparative example 72 |
|---|---|---|---|---|---|
| 1. | Netarsudil dimesylate | 14.2 mg | 14.2 mg | 28.5 mg | 28.5 mg |
| 2. | Hydrochloric acid | 0.094 mL | 0.188 mL | 0.094 mL | 0.188 mL |
| 3. | Bimatoprost | 30 mg | 30 mg | 30 mg | 30 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 348. Contents of various main medicine components in Comparative examples 69 to 72 after being stored at 5°Cfor 7 days**

| No. | Component | Comparative example 69 | Comparative example 70 | Comparative example 71 | Comparative example 72 |
|---|---|---|---|---|---|
| | | Content % | | | |
| 1. | Netarsudil dimesylate | <30 | <30 | <30 | <30 |
| 2. | Bimatoprost | 99.7 | 99.5 | 99.5 | 99.6 |

According to Comparative examples 69 to 72, the preparation including netarsudil dimesylate, hydrochloric acid and bimatoprost has incompatibility under the storage condition of 5°C, and netarsudil dimesylate is precipitated.

### Comparative examples 73 to 76: a preparation including netarsudil dimesylate, carteolol hydrochloride and travoprost

**Table 349. Preparation of a local ophthalmic medicine composition solution for reducing an intra-ocular pressure as follows:**

| No. | Component | Comparative example 73 | Comparative example 74 | Comparative example 75 | Comparative example 76 |
|---|---|---|---|---|---|
| 1. | Netarsudil dimesylate | 14.2 mg | 14.2 mg | 28.5 mg | 28.5 mg |
| 2. | Carteolol hydrochloride | 1000 mg | 2000 mg | 1000 mg | 2000 mg |
| 3. | Travoprost | 4 mg | 4 mg | 4 mg | 4 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 350. Contents of various main medicine components in Comparative examples 73 to 76 after being stored at 5°Cfor 7 days**

| No. | Component | Comparative example 73 | Comparative example 74 | Comparative example 75 | Comparative example 76 |
|---|---|---|---|---|---|
| | | | Content % | | |
| 1. | Netarsudil dimesylate | <30 | <30 | <30 | <30 |
| 2. | Carteolol hydrochloride | 100.2 | 100.5 | 100.8 | 100.1 |
| 3. | Travoprost | 99.8 | 99.7 | 99.5 | 99.1 |

According to Comparative examples 73 to 76, the preparation including netarsudil dimesylate, carteolol hydrochloride and travoprost has incompatibility under the storage condition of 5°C, and netarsudil dimesylate is precipitated.

### Comparative examples 77 to 80: a preparation including netarsudil dimesylate, hydrochloric acid and travoprost

**Table 351. Preparation of a local ophthalmic medicine composition solution for reducing an intra-ocular pressure as follows:**

| No. | Component | Comparative example 77 | Comparative example 78 | Comparative example 79 | Comparative example 80 |
|---|---|---|---|---|---|
| 1. | Netarsudil dimesylate | 14.2 mg | 14.2 mg | 28.5 mg | 28.5 mg |
| 2. | Hydrochloric acid | 0.094 mL | 0.188 mL | 0.094 mL | 0.188 mL |
| 3. | Travoprost | 4 mg | 4 mg | 4 mg | 4 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 352. Contents of various main medicine components in Comparative examples 77 to 80 after being stored at 5°Cfor 7 days**

| No. | Component | Comparative example 77 | Comparative example 78 | Comparative example 79 | Comparative example 80 |
|---|---|---|---|---|---|
| | | Content % | | | |
| 1. | Netarsudil dimesylate | <30 | <30 | <30 | <30 |
| 2. | Travoprost | 100.3 | 99.8 | 100.1 | 99.9 |

According to Comparative examples 77 to 80, the preparation including netarsudil dimesylate, hydrochloric acid and travoprost has incompatibility under the storage condition of 5°C, and netarsudil dimesylate is precipitated.

### Comparative examples 81 to 84: a preparation including netarsudil dimesylate, carteolol hydrochloride and tafluprost

**Table 353. Preparation of a local ophthalmic medicine composition solution for reducing an intra-ocular pressure as follows:**

| No. | Component | Comparative example 81 | Comparative example 82 | Comparative example 83 | Comparative example 84 |
|---|---|---|---|---|---|
| 1. | Netarsudil dimesylate | 14.2 mg | 14.2 mg | 28.5 mg | 28.5 mg |
| 2. | Carteolol hydrochloride | 1000 mg | 2000 mg | 1000 mg | 2000 mg |
| 3. | Tafluprost | 1.5 mg | 1.5 mg | 1.5 mg | 1.5 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 354. Contents of various main medicine components in Comparative examples 81 to 84 after being stored at 5°Cfor 7 days**

| No. | Component | Comparative example 81 | Comparative example 82 | Comparative example 83 | Comparative example 84 |
|---|---|---|---|---|---|
| | | Content % | | | |
| 1. | Netarsudil dimesylate | <30 | <30 | <30 | <30 |
| 2. | Carteolol hydrochloride | 100.3 | 100.0 | 99.9 | 100.1 |
| 3. | Tafluprost | 99.5 | 99.8 | 99.1 | 99.4 |

According to Comparative examples 81 to 84, the preparation including netarsudil dimesylate, carteolol hydrochloride and tafluprost has incompatibility under the storage condition of 5°C, and netarsudil dimesylate is precipitated.

### Comparative examples 85 to 88: a preparation including netarsudil dimesylate, hydrochloric acid and tafluprost

**Table 355. Preparation of a local ophthalmic medicine composition solution for reducing an intra-ocular pressure as follows:**

| No. | Component | Comparative example 85 | Comparative example 86 | Comparative example 87 | Comparative example 88 |
|---|---|---|---|---|---|
| 1. | Netarsudil dimesylate | 14.2 mg | 14.2 mg | 28.5 mg | 28.5 mg |
| 2. | Hydrochloric acid | 0.094 mL | 0.188 mL | 0.094 mL | 0.188 mL |
| 3. | Tafluprost | 1.5 mg | 1.5 mg | 1.5 mg | 1.5 mg |
| 4. | Boric acid | 50 mg | 50 mg | 50 mg | 50 mg |
| 5. | Mannitol | 3400 mg | 3400 mg | 3400 mg | 3400 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 356. Contents of various main medicine components in Comparative examples 85 to 88 after being stored at 5°Cfor 7 days**

| No. | Component | Comparative example 85 | Comparative example 86 | Comparative example 87 | Comparative example 88 |
|---|---|---|---|---|---|
| | | Content % | | | |
| 1. | Netarsudil dimesylate | <30 | <30 | <30 | <30 |
| 2. | Tafluprost | 99.9 | 99.3 | 99.9 | 100.1 |

According to Comparative examples 85 to 88, the preparation including netarsudil dimesylate, hydrochloric acid and tafluprost has incompatibility under the storage condition of 5°C, and netarsudil dimesylate is precipitated.

### Embodiments 211 to 215: a preparation including netarsudil dimesylate, betaxolol mesylate and latanoprost

**Table 357. Preparation of a local ophthalmic medicine composition solution for reducing an intra-ocular pressure through a conventional method as follows:**

| No . | Component | Embodimen t211 | Embodimen t212 | Embodimen t 213 | Embodimen t214 | Embodimen t215 |
|---|---|---|---|---|---|---|
| 1. | Netarsudil dimesylate | 14.2 mg | 14.2 mg | 28.5 mg | 28.5 mg | 57.0 mg |
| 2. | Betaxolol mesylate | 164.1 mg | 328.2 mg | 164.1 mg | 328.2 mg | 656.3 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 358. Contents of various main medicine components in Embodiments 211 to 215 after being stored at 5°Cfor 24 months**

| No. | Component | Embodiment 211 | Embodiment 212 | Embodiment 213 | Embodiment 214 | Embodiment 215 |
|---|---|---|---|---|---|---|
| | | Content % | | | | |
| 1. | Netarsudil dimesylate | 100.1 | 99.2 | 100.3 | 99.9 | 99.3 |
| 2. | Betaxolol mesylate | 100.3 | 101.5 | 100.3 | 100.5 | 99.9 |
| 3. | Latanoprost | 100.1 | 99.2 | 99.5 | 99.5 | 100.3 |

**Table 359. Contents of various main medicine components in commercially available eye drops (Betoptic^{®}, Rhopress^{®} and Xalatan^{®}) after being stored at 5°Cfor 24 months**

| No. | Component | Betoptic^{®} | Rhopress^{®} | Xalatan^{®} |
|---|---|---|---|---|
| | | Content % | | |
| 1. | Betaxolol hydrochloride | 99.9 | - | - |
| 2. | Netarsudil dimesylate | - | 99.9 | - |
| 3. | Latanoprost | - | - | 99.8 |

According to Embodiments 211 to 215, the preparation including netarsudil dimesylate, betaxolol mesylate and latanoprost has good stability after being stored under a storage condition of 5°Cfor 24 months, which meets a requirement for a storage condition of the commercially available eye drops (Betoptic^{®}, Rhopress^{®} andXalatan^{®}) before opening.

**Table 360. Contents of various main medicine components in Embodiments 211 to 215 after being stored at 25°Cfor 6 weeks**

| No. | Component | Embodiment 211 | Embodiment 212 | Embodiment 213 | Embodiment 214 | Embodiment 215 |
|---|---|---|---|---|---|---|
| | | Content % | | | | |
| 1. | Netarsudil dimesylate | 100.0 | 99.1 | 99.9 | 101.1 | 99.9 |
| 2. | Betaxolol mesylate | 100.5 | 100.3 | 101.1 | 101.8 | 100.7 |
| 3. | Latanoprost | 100.2 | 99.8 | 98.7 | 99.9 | 100.0 |

**Table 361. Contents of various main medicine components in commercially available eye drops (Betoptic^{®}, Rhopress^{®} and Xalatan^{®}) after being stored at 25°Cfor 6 weeks**

| No. | Component | Betoptic^{®} | Rhopress^{®} | Xalatan^{®} |
|---|---|---|---|---|
| | | Content % | | |
| 1. | Betaxolol hydrochloride | 99.7 | - | - |
| 2. | Netarsudil dimesylate | - | 99.8 | - |
| 3. | Latanoprost | - | - | 99.6 |

According to Embodiments 211 to 215, the preparation including netarsudil dimesylate, betaxolol mesylate and latanoprost has good stability after being stored under a storage condition of 25°Cfor 6 weeks, which meets a requirement for a storage condition of the commercially available eye drops (Betoptic^{®}, Rhopress^{®} and Xalatan^{®}) after opening.

**Table 362. Contents of various main medicine components in Embodiments 211 to 215 after being stored at 40°Cfor 14 days**

| No. | Component | Embodiment 211 | Embodiment 212 | Embodiment 213 | Embodiment 214 | Embodiment 215 |
|---|---|---|---|---|---|---|
| | | Content % | | | | |
| 1. | Netarsudil dimesylate | 99.3 | 99.2 | 99.1 | 98.9 | 99.1 |
| 2. | Betaxolol mesylate | 100.9 | 101.1 | 98.9 | 100.1 | 101.9 |
| 3. | Latanoprost | 99.1 | 99.2 | 98.6 | 98.9 | 99.2 |

**Table 363. Contents of various main medicine components in commercially available eye drops (Betoptic^{®}, Rhopress^{®} and Xalatan^{®}) after being stored at 40°Cfor 14 days**

| No. | Component | Betoptic^{®} | Rhopress^{®} | Xalatan^{®} |
|---|---|---|---|---|
| | | Content % | | |
| 1. | Betaxolol hydrochloride | 100.1 | - | - |
| 2. | Netarsudil dimesylate | - | 99.5 | - |
| 3. | Latanoprost | - | - | 99.7 |

According to Embodiments 211 to 215, the preparation including netarsudil dimesylate, betaxolol mesylate and latanoprost has good stability after being stored under a storage condition of 40°Cfor 14 days, which meets a requirement for departing from a storage condition of the commercially available eye drops (Betoptic^{®}, Rhopress^{®} and Xalatan^{®}) for a short time.

### Embodiments 216 to 220: a preparation including netarsudil dihydrochloride, betaxolol hydrochloride and latanoprost

**Table 364. Preparation of a local ophthalmic medicine composition solution for reducing an intra-ocular pressure through a conventional method as follows:**

| No. | Component | Embodiment 216 | Embodiment 217 | Embodiment 218 | Embodiment 219 | Embodiment 220 |
|---|---|---|---|---|---|---|
| 1. | Netarsudil dihydrochloride | 11.6 mg | 11.6 mg | 23.2 mg | 23.2 mg | 46.4 mg |
| 2. | Betaxolol hydrochloride | 139.8 mg | 279.6 mg | 139.8 mg | 279.6 mg | 559.3 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 365. Contents of various main medicine components in Embodiments 216 to 220 after being stored at 5°Cfor 24 months**

| No. | Component | Embodiment 216 | Embodiment 217 | Embodiment 218 | Embodiment 219 | Embodiment 220 |
|---|---|---|---|---|---|---|
| | | Content % | | | | |
| 1. | Netarsudil dihydrochloride | 99.5 | 99.2 | 99.7 | 99.3 | 99.9 |
| 2. | Betaxolol hydrochloride | 99.3 | 100.1 | 100.9 | 99.7 | 99.9 |
| 3. | Latanoprost | 99.8 | 100.3 | 99.8 | 100.5 | 100.1 |

According to Embodiments 216 to 220, the preparation including netarsudil dihydrochloride, betaxolol hydrochloride and latanoprost has good stability after being stored under a storage condition of 5°Cfor 24 months, which meets a requirement for a storage condition of the commercially available eye drops (Betoptic^{®}, Rhopress^{®} and Xalatan^{®}) before opening.

**Table 366. Contents of various main medicine components in Embodiments 216 to 220 after being stored at 25°Cfor 6 weeks**

| No. | Component | Embodiment 216 | Embodiment 217 | Embodiment 218 | Embodiment 219 | Embodiment 220 |
|---|---|---|---|---|---|---|
| | | Content % | | | | |
| 1. | Netarsudil dihydrochloride | 100.4 | 99.5 | 99.9 | 100.1 | 99.9 |
| 2. | Betaxolol hydrochloride | 99.6 | 99.4 | 99.3 | 100.1 | 100.1 |
| 3. | Latanoprost | 101.5 | 101.1 | 100.7 | 100.9 | 100.2 |

According to Embodiments 216 to 220, the preparation including netarsudil dihydrochloride, betaxolol hydrochloride and latanoprost has good stability after being stored under a storage condition of 25°Cfor 6 weeks, which meets a requirement for a storage condition of the commercially available eye drops (Betoptic^{®}, Rhopress^{®} and Xalatan^{®}) before opening.

**Table 367. Contents of various main medicine components in Embodiments 216 to 220 after being stored at 40°Cfor 14 days**

| No. | Component | Embodiment 216 | Embodiment 217 | Embodiment 218 | Embodiment 219 | Embodiment 220 |
|---|---|---|---|---|---|---|
| | | Content % | | | | |
| 1. | Netarsudil dihydrochloride | 100.1 | 99.1 | 99.8 | 100.1 | 100.8 |
| 2. | Betaxolol hydrochloride | 99.2 | 99.6 | 101.3 | 100.8 | 99.3 |
| 3. | Latanoprost | 99.4 | 100.3 | 99.4 | 99.8 | 99.2 |

According to Embodiments 216 to 220, the preparation including netarsudil dihydrochloride, betaxolol hydrochloride and latanoprost has good stability after being stored under a storage condition of 40°Cfor 14 days, which meets a requirement for departing from a storage condition of the commercially available eye drops (Betoptic^{®}, Rhopress^{®} and Xalatan^{®}) for a short time.

### Embodiments 221 to 225: a preparation including netarsudil dimesylate, betaxolol mesylate and bimatoprost

**Table 368. Preparation of a local ophthalmic medicine composition solution for reducing an intra-ocular pressure through a conventional method as follows:**

| No . | Component | Embodiment 221 | Embodiment 222 | Embodiment 223 | Embodiment 224 | Embodiment 225 |
|---|---|---|---|---|---|---|
| 1. | Netarsudil dimesylate | 14.2 mg | 14.2 mg | 28.5 mg | 28.5 mg | 57.0 mg |
| 2. | Betaxolol mesylate | 164.1 mg | 328.2 mg | 164.1 mg | 328.2 mg | 656.3 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 5000 mg | 5000 mg | 5000 mg | 5000 mg | 5000 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 369. Contents of various main medicine components in Embodiments 221 to 225 after being stored at 5°Cfor 24 months**

| No. | Component | Embodiment 221 | Embodiment 222 | Embodiment 223 | Embodiment 224 | Embodiment 225 |
|---|---|---|---|---|---|---|
| | | Content % | | | | |
| 1. | Netarsudil dimesylate | 99.2 | 99.5 | 100.8 | 100.3 | 100.1 |
| 2. | Betaxolol mesylate | 100.1 | 100.2 | 100.9 | 100.9 | 100.4 |
| 3. | Bimatoprost | 99.6 | 101.2 | 100.4 | 99.3 | 99.9 |

**Table 370. Contents of various main medicine components in commercially available eye drops (Betoptic^{®}, Rhopress^{®}, Lumigan^{®}) after being stored at 5°Cfor 24 months**

| No. | Component | Betoptic^{®} | Rhopress^{®} | Lumigan^{®} |
|---|---|---|---|---|
| | | Content % | | |
| 1. | Betaxolol hydrochloride | 99.9 | - | - |
| 2. | Netarsudil dimesylate | - | 99.9 | - |
| 3. | Bimatoprost | - | - | 99.4 |

According to Embodiments 221 to 225, the preparation including netarsudil dimesylate, betaxolol mesylate and bimatoprost has good stability after being stored under a storage condition of 5°Cfor 24 months, which meets a requirement for a storage condition of the commercially available eye drops (Betoptic^{®}, Rhopress^{®} andLumigan^{®}) before opening.

**Table 371. Contents of various main medicine components in Embodiments 221 to 225 after being stored at 25°Cfor 6 weeks**

| No. | Component | Embodiment 221 | Embodiment 222 | Embodiment 223 | Embodiment 224 | Embodiment 225 |
|---|---|---|---|---|---|---|
| | | Content % | | | | |
| 1. | Netarsudil dimesylate | 100.1 | 99.1 | 99.7 | 99.4 | 99.0 |
| 2. | Betaxolol mesylate | 99.5 | 101.1 | 99.5 | 100.3 | 99.3 |
| 3. | Bimatoprost | 99.9 | 99.2 | 100.9 | 100.3 | 100.5 |

**Table 372. Contents of various main medicine components in commercially available eye drops (Betoptic^{®}, Rhopress^{®}, Lumigan^{®}) after being stored at 25°Cfor 6 weeks**

| No. | Component | Betoptic^{®} | Rhopress^{®} | Lumigan^{®} |
|---|---|---|---|---|
| | | Content % | | |
| 1. | Betaxolol hydrochloride | 99.7 | - | - |
| 2. | Netarsudil dimesylate | - | 99.8 | - |
| 3. | Bimatoprost | - | - | 99.5 |

According to Embodiments 221 to 225, the preparation including netarsudil dimesylate, betaxolol mesylate and bimatoprost has good stability after being stored under a storage condition of 25°Cfor 6 weeks, which meets a requirement for a storage condition of the commercially available eye drops (Betoptic^{®}, Rhopress^{®} and Lumigan^{®}) after opening.

**Table 373. Contents of various main medicine components in Embodiments 221 to 225 after being stored at 40°Cfor 14 days**

| No. | Component | Embodiment 221 | Embodiment 222 | Embodiment 223 | Embodiment 224 | Embodiment 225 |
|---|---|---|---|---|---|---|
| | | Content % | | | | |
| 1. | Netarsudil dimesylate | 99.5 | 99.5 | 100.1 | 99.5 | 99.8 |
| 2. | Betaxolol mesylate | 100.1 | 100.9 | 99.7 | 101.8 | 100.1 |
| 3. | Bimatoprost | 99.7 | 99.9 | 100.1 | 98.5 | 99.5 |

**Table 374. Contents of various main medicine components in commercially available eye drops (Betoptic^{®}, Rhopress^{®} and Lumigan^{®}) after being stored at 40°Cfor 14 days**

| No. | Component | Betoptic^{®} | Rhopress^{®} | Lumigan^{®} |
|---|---|---|---|---|
| | | Content % | | |
| 1. | Betaxolol hydrochloride | 100.1 | - | - |
| 2. | Netarsudil dimesylate | - | 99.5 | - |
| 3. | Bimatoprost | - | - | 99.2 |

According to Embodiments 221 to 225, the preparation including netarsudil dimesylate, betaxolol mesylate and bimatoprost has good stability after being stored under a storage condition of 40°Cfor 14 days, which meets a requirement for departing from a storage condition of the commercially available eye drops (Betoptic^{®}, Rhopress^{®} and Lumigan^{®}) for a short time.

### Embodiments 266 to 270: a preparation including netarsudil dihydrochloride, betaxolol hydrochloride and bimatoprost

**Table 375. Preparation of a local ophthalmic medicine composition solution for reducing an intra-ocular pressure through a conventional method as follows:**

| No. | Component | Embodiment 226 | Embodiment 227 | Embodiment 228 | Embodiment 229 | Embodiment 230 |
|---|---|---|---|---|---|---|
| 1. | Netarsudil | 11.6mg | 11.6mg | 23.2mg | 23.2mg | 46.4mg |
| | dihydrochloride | | | | | |
| 2. | Betaxolol hydrochloride | 139.8 mg | 279.6 mg | 139.8 mg | 279.6 mg | 559.3 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 4800 mg | 4800 mg | 4800 mg | 4800 mg | 4800 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 376. Contents of various main medicine components in Embodiments 266 to 270 after being stored at 5°Cfor 24 months**

| No. | Component | Embodiment 226 | Embodiment 227 | Embodiment 228 | Embodiment 229 | Embodiment 230 |
|---|---|---|---|---|---|---|
| | | Content % | | | | |
| 1. | Netarsudil dihydrochloride | 98.4 | 99.5 | 98.6 | 99.8 | 98.3 |
| 2. | Betaxolol hydrochloride | 100.6 | 101.3 | 101.2 | 100.3 | 100.8 |
| 3. | Bimatoprost | 101.2 | 100.4 | 99.9 | 100.3 | 101.5 |

According to Embodiments 266 to 270, the preparation including netarsudil dihydrochloride, betaxolol hydrochloride and bimatoprost has good stability after being stored under a storage condition of 5°Cfor 24 months, which meets a requirement for a storage condition of the commercially available eye drops (Betoptic^{®}, Rhopress^{®} and Lumigan^{®}) before opening.

**Table 377. Contents of various main medicine components in Embodiments 266 to 270 after being stored at 25°Cfor 6 weeks**

| No. | Component | Embodiment 226 | Embodiment 227 | Embodiment 228 | Embodiment 229 | Embodiment 230 |
|---|---|---|---|---|---|---|
| | | Content % | | | | |
| 1. | Netarsudil dihydrochloride | 100.2 | 100.5 | 99.7 | 101.3 | 99.7 |
| 2. | Betaxolol hydrochloride | 100.2 | 101.5 | 100.1 | 99.8 | 100.2 |
| 3. | Bimatoprost | 99.5 | 100.9 | 99.0 | 100.5 | 99.2 |

According to Embodiments 266 to 270, the preparation including netarsudil dihydrochloride, betaxolol hydrochloride and bimatoprost has good stability after being stored under a storage condition of 25°Cfor 6 weeks, which meets a requirement for a storage condition of the commercially available eye drops (Betoptic^{®}, Rhopress^{®} and Lumigan^{®}) after opening.

**Table 378. Contents of various main medicine components in Embodiments 266 to 270 after being stored at 40°Cfor 14 days**

| No. | Component | Embodiment 226 | Embodiment 227 | Embodiment 228 | Embodiment 229 | Embodiment 230 |
|---|---|---|---|---|---|---|
| | | Content % | | | | |
| 1. | Netarsudil dihydrochloride | 100.2 | 99.7 | 100.8 | 99.7 | 100.2 |
| 2. | Betaxolol hydrochloride | 98.9 | 100.2 | 99.4 | 100.9 | 99.3 |
| 3. | Bimatoprost | 100.3 | 98.4 | 101.2 | 99.0 | 100.2 |

According to Embodiments 266 to 270, the preparation including netarsudil dihydrochloride, betaxolol hydrochloride and bimatoprost has good stability after being stored under a storage condition of 40°Cfor 14 days, which meets a requirement for departing from a storage condition of the commercially available eye drops (Betoptic^{®}, Rhopress^{®} and Lumigan^{®}) for a short time.

### Embodiments 231 to 235: a preparation including netarsudil dimesylate, betaxolol mesylate and travoprost

**Table 379. Preparation of a local ophthalmic medicine composition solution for reducing an intra-ocular pressure through a conventional method as follows:**

| No. | Component | Embodiment 231 | Embodiment 232 | Embodiment 233 | Embodiment 234 | Embodiment 235 |
|---|---|---|---|---|---|---|
| 1. | Netarsudil dimesylate | 14.2 mg | 14.2 mg | 28.5 mg | 28.5 mg | 57.0 mg |
| 2. | Betaxolol mesylate | 164.1 mg | 328.2 mg | 164.1 mg | 328.2 mg | 656.3 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 4800 mg | 4800 mg | 4800 mg | 4800 mg | 4800 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 380. Contents of various main medicine components in Embodiments 231 to 235 after being stored at 5°Cfor 24 months**

| No. | Component | Embodiment 231 | Embodiment 232 | Embodiment 233 | Embodiment 234 | Embodiment 235 |
|---|---|---|---|---|---|---|
| | | Content % | | | | |
| 1. | Netarsudil dimesylate | 99.8 | 100.2 | 99.1 | 100.1 | 100.4 |
| 2. | Betaxolol mesylate | 100.2 | 99.8 | 100.8 | 99.9 | 99.3 |
| 3. | Travoprost | 99.4 | 99.6 | 99.9 | 99.8 | 99.9 |

**Table 381. Contents of various main medicine components in commercially available eye drops (Betoptic^{®}, Rhopress^{®} and Travatan^{®}) after being stored at 5°Cfor 24 months**

| No. | Component | Betoptic^{®} | Rhopress^{®} | Travatan^{®} |
|---|---|---|---|---|
| | | Content % | | |
| 1. | Betaxolol hydrochloride | 99.9 | - | - |
| 2. | Netarsudil dimesylate | - | 99.9 | - |
| 3. | Travoprost | - | - | 99.8 |

According to Embodiments 231 to 235, the preparation including netarsudil dimesylate, betaxolol mesylate and travoprost has good stability after being stored under a storage condition of 5°Cfor 24 months, which meets a requirement for a storage condition of the commercially available eye drops (Betoptic^{®}, Rhopress^{®} and Travatan^{®}) before opening.

**Table 382. Contents of various main medicine components in Embodiments 231 to 235 after being stored at 25°Cfor 6 weeks**

| No. | Component | Embodiment 231 | Embodiment 232 | Embodiment 233 | Embodiment 234 | Embodiment 235 |
|---|---|---|---|---|---|---|
| | | Content % | | | | |
| 1. | Netarsudil dimesylate | 100.5 | 100.9 | 99.7 | 100.4 | 99.9 |
| 2. | Betaxolol mesylate | 99.5 | 99.4 | 99.8 | 100.4 | 100.3 |
| 3. | Travoprost | 100.0 | 99.9 | 99.6 | 100.9 | 100.6 |

**Table 383. Contents of various main medicine components in commercially available eye drops (Betoptic^{®}, Rhopress^{®} and Travatan^{®}) after being stored at 25°Cfor 6 weeks**

| No. | Component | Betoptic^{®} | Rhopress^{®} | Travatan^{®} |
|---|---|---|---|---|
| | | | Content % | |
| 1. | Betaxolol hydrochloride | 99.7 | - | - |
| 2. | Netarsudil dimesylate | - | 99.8 | - |
| 3. | Travoprost | - | - | 99.5 |

According to Embodiments 231 to 235, the preparation including netarsudil dimesylate, betaxolol mesylate and travoprost has good stability after being stored under a storage condition of 25°Cfor 6 weeks, which meets a requirement for a storage condition of the commercially available eye drops (Betoptic^{®}, Rhopress^{®} andTravatan^{®}) after opening.

**Table 384. Contents of various main medicine components in Embodiments 231 to 235 after being stored at 40°Cfor 14 days**

| No. | Component | Embodiment 231 | Embodiment 232 | Embodiment 233 | Embodiment 234 | Embodiment 235 |
|---|---|---|---|---|---|---|
| | | Content % | | | | |
| 1. | Netarsudil dimesylate | 99.7 | 99.7 | 100.6 | 99.5 | 99.9 |
| 2. | Betaxolol mesylate | 99.6 | 100.7 | 99.9 | 99.9 | 99.7 |
| 3. | Travoprost | 99.5 | 99.4 | 100.9 | 100.9 | 99.6 |

**Table 385. Contents of various main medicine components in commercially available eye drops (Betoptic^{®}, Rhopress^{®} and Travatan^{®}) after being stored at 40°Cfor 14 days**

| No. | Component | Betoptic^{®} | Rhopress^{®} | Travatan^{®} |
|---|---|---|---|---|
| | | | Content % | |
| 1. | Betaxolol hydrochloride | 100.1 | - | - |
| 2. | Netarsudil dimesylate | - | 99.5 | - |
| 3. | Travoprost | - | - | 99.7 |

According to Embodiments 231 to 235, the preparation including netarsudil dimesylate, betaxolol mesylate and travoprost has good stability after being stored under a storage condition of 40°Cfor 14 days, which meets a requirement for departing from a storage condition of the commercially available eye drops(Betoptic^{®}, Rhopress^{®} and Travatan^{®}) for a short time.

### Embodiments 236 to 240: a preparation including netarsudil dihydrochloride, betaxolol hydrochloride and travoprost

**Table 386. Preparation of a local ophthalmic medicine composition solution for reducing an intra-ocular pressure through a conventional method as follows:**

| No. | Component | Embodiment 236 | Embodiment 237 | Embodiment 238 | Embodiment 239 | Embodiment 240 |
|---|---|---|---|---|---|---|
| 1. | Netarsudil dihydrochloride | 11.6 mg | 11.6 mg | 23.2 mg | 23.2 mg | 46.4 mg |
| 2. | Betaxolol hydrochloride | 139.8 mg | 279.6 mg | 139.8 mg | 279.6 mg | 559.3 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 4800 mg | 4800 mg | 4800 mg | 4800 mg | 4800 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 387. Contents of various main medicine components in Embodiments 236 to 240 after being stored at 5°Cfor 24 months**

| No. | Component | Embodiment 236 | Embodiment 237 | Embodiment 238 | Embodiment 239 | Embodiment 240 |
|---|---|---|---|---|---|---|
| | | Content % | | | | |
| 1. | Netarsudil dihydrochloride | 99.9 | 99.4 | 100.7 | 99.3 | 100.2 |
| 2. | Betaxolol hydrochloride | 100.6 | 99.5 | 100.0 | 99.5 | 101.3 |
| 3. | Travoprost | 99.7 | 100.8 | 100.6 | 100.3 | 99.7 |

According to Embodiments 236 to 240, the preparation including netarsudil dihydrochloride, betaxolol hydrochloride and travoprost has good stability after being stored under a storage condition of 5°Cfor 24 months, which meets a requirement for a storage condition of the commercially available eye drops (Betoptic^{®}, Rhopress^{®} andTravatan^{®}) before opening.

**Table 388. Contents of various main medicine components in Embodiments 236 to 240 after being stored at 25°Cfor 6 weeks**

| No. | Component | Embodiment 236 | Embodiment 237 | Embodiment 238 | Embodiment 239 | Embodiment 240 |
|---|---|---|---|---|---|---|
| | | Content % | | | | |
| 1. | Netarsudil dihydrochloride | 100.6 | 98.5 | 100.5 | 99.8 | 99.6 |
| 2. | Betaxolol hydrochloride | 99.8 | 100.9 | 100.7 | 100.7 | 100.7 |
| 3. | Travoprost | 99.3 | 99.7 | 99.9 | 99.5 | 99.5 |

According to Embodiments 236 to 240, the preparation including netarsudil dihydrochloride, betaxolol hydrochloride and travoprost has good stability after being stored under a storage condition of 25°Cfor 6 weeks, which meets a requirement for a storage condition of the commercially available eye drops (Betoptic^{®}, Rhopress^{®} and Travatan^{®}) after opening.

**Table 389. Contents of various main medicine components in Embodiments 236 to 240 after being stored at 40°Cfor 14 days**

| No. | Component | Embodiment 236 | Embodiment 237 | Embodiment 238 | Embodiment 239 | Embodiment 240 |
|---|---|---|---|---|---|---|
| | | Content % | | | | |
| 1. | Netarsudil dihydrochloride | 99.6 | 99.9 | 99.2 | 100.4 | 99.9 |
| 2. | Betaxolol hydrochloride | 100.9 | 100.7 | 100.3 | 99.7 | 99.5 |
| 3. | Travoprost | 100.6 | 99.7 | 100.3 | 100.3 | 100.6 |

According to Embodiments 236 to 240, the preparation including netarsudil dihydrochloride, betaxolol hydrochloride and travoprost has good stability after being stored under a storage condition of 40°Cfor 14 days, which meets a requirement for departing from a storage condition of the commercially available eye drops (Betoptic^{®}, Rhopress^{®} and Travatan^{®}) for a short time.

### Embodiments 241 to 245: a preparation including netarsudil dimesylate, betaxolol mesylate and tafluprost

**Table 390. Preparation of a local ophthalmic medicine composition solution for reducing an intra-ocular pressure through a conventional method as follows:**

| No. | Component | Embodiment 241 | Embodiment 242 | Embodiment 243 | Embodiment 344 | Embodiment 345 |
|---|---|---|---|---|---|---|
| 1. | Netarsudil dimesylate | 14.2 mg | 14.2 mg | 28.5 mg | 28.5 mg | 57.0 mg |
| 2. | Betaxolol mesylate | 164.1 mg | 328.2 mg | 164.1 mg | 328.2 mg | 656.3 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 4800 mg | 4800 mg | 4800 mg | 4800 mg | 4800 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 391. Contents of various main medicine components in Embodiments 241 to 245 after being stored at 5°Cfor 24 months**

| No. | Component | Embodiment 241 | Embodiment 242 | Embodiment 243 | Embodiment 344 | Embodiment 345 |
|---|---|---|---|---|---|---|
| | | Content % | | | | |
| 1. | Netarsudil dimesylate | 100.6 | 99.3 | 99.8 | 99.5 | 100.3 |
| 2. | Betaxolol mesylate | 100.9 | 99.3 | 100.3 | 100.4 | 99.6 |
| 3. | Tafluprost | 99.7 | 99.9 | 100.5 | 100.4 | 100.3 |

**Table 392. Contents of various main medicine components in commercially available eye drops (Betoptic^{®}, Rhopress^{®} and Tapros^{®}) after being stored at 5°Cfor 24 months**

| No. | Component | Betoptic^{®} | Rhopress^{®} | Tapros^{®} |
|---|---|---|---|---|
| | | Content % | | |
| 1. | Betaxolol hydrochloride | 99.9 | - | - |
| 2. | Netarsudil dimesylate | - | 99.9 | - |
| 3. | Tafluprost | - | - | 99.4 |

According to Embodiments 241 to 245, the preparation including netarsudil dimesylate, betaxolol mesylate and tafluprost has good stability after being stored under a storage condition of 5°Cfor 24 months, which meets a requirement for a storage condition of the commercially available eye drops (Betoptic^{®}, Rhopress^{®} and Tapros^{®}) before opening.

**Table 393. Contents of various main medicine components in Embodiments 241 to 245 after being stored at 25°Cfor 6 weeks**

| No. | Component | Embodiment 241 | Embodiment 242 | Embodiment 243 | Embodiment 344 | Embodiment 345 |
|---|---|---|---|---|---|---|
| | | Content % | | | | |
| 1. | Netarsudil dimesylate | 99.4 | 100.5 | 101.7 | 100.8 | 100.1 |
| 2. | Betaxolol mesylate | 99.6 | 100.2 | 99.5 | 99.5 | 100.4 |
| 3. | Tafluprost | 99.7 | 99.9 | 99.9 | 99.8 | 100.7 |

**Table 394. Contents of various main medicine components in commercially available eye drops (Betoptic^{®}, Rhopress^{®} and Tapros^{®}) after being stored at 25°Cfor 6 weeks**

| No. | Component | Betoptic^{®} | Rhopress^{®} | Tapros^{®} |
|---|---|---|---|---|
| | | Content % | | |
| 1. | Betaxolol hydrochloride | 99.7 | - | - |
| 2. | Netarsudil dimesylate | - | 99.8 | - |
| 3. | Tafluprost | - | - | 99.3 |

According to Embodiments 241 to 245, the preparation including netarsudil dimesylate, betaxolol mesylate and tafluprost has good stability after being stored under a storage condition of 25°Cfor 6 weeks, which meets a requirement for a storage condition of the commercially available eye drops (Betoptic^{®}, Rhopress^{®} andTapros^{®}) after opening.

**Table 395. Contents of various main medicine components in Embodiments 241 to 245 after being stored at 40°Cfor 14 days**

| No. | Component | Embodiment 241 | Embodiment 242 | Embodiment 243 | Embodiment 344 | Embodiment 345 |
|---|---|---|---|---|---|---|
| | | Content % | | | | |
| 1. | Netarsudil dimesylate | 99.7 | 99.8 | 99.9 | 99.2 | 100.3 |
| 2. | Betaxolol mesylate | 99.7 | 100.3 | 100.2 | 100.6 | 100.0 |
| 3. | Tafluprost | 99.8 | 100.2 | 100.6 | 100.7 | 100.5 |

**Table 396. Contents of various main medicine components in commercially available eye drops (Betoptic^{®}, Rhopress^{®} and Tapros^{®}) after being stored at 40°Cfor 14 days**

| No. | Component | Betoptic^{®} | Rhopress^{®} | Tapros^{®} |
|---|---|---|---|---|
| | | | Content % | |
| 1. | Betaxolol hydrochloride | 100.1 | - | - |
| 2. | Netarsudil dimesylate | - | 99.5 | - |
| 3. | Tafluprost | - | - | 99.8 |

According to Embodiments 241 to 245, the preparation including netarsudil dimesylate, betaxolol mesylate and tafluprost has good stability after being stored under a storage condition of 40°Cfor 14 days, which meets a requirement for departing from a storage condition of the commercially available eye drops (Betoptic^{®}, Rhopress^{®} and Tapros^{®}) for a short time.

### Embodiments 246 to 250: a preparation including netarsudil dihydrochloride, betaxolol hydrochloride and tafluprost

**Table 397. Preparation of a local ophthalmic medicine composition solution for reducing an intra-ocular pressure through a conventional method as follows:**

| No. | Component | Embodiment 246 | Embodiment 247 | Embodiment 248 | Embodiment 249 | Embodiment 250 |
|---|---|---|---|---|---|---|
| 1. | Netarsudil dihydrochloride | 11.6 mg | 11.6 mg | 23.2 mg | 23.2 mg | 46.4 mg |
| 2. | Betaxolol hydrochloride | 139.8 mg | 279.6 mg | 139.8 mg | 279.6 mg | 559.3 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 4800 mg | 4800 mg | 4800 mg | 4800 mg | 4800 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 398. Contents of various main medicine components in Embodiments 246 to 250 after being stored at 5°Cfor 24 months**

| No. | Component | Embodiment 246 | Embodiment 247 | Embodiment 248 | Embodiment 249 | Embodiment 250 |
|---|---|---|---|---|---|---|
| | | Content % | | | | |
| 1. | Netarsudil dihydrochloride | 99.6 | 100.7 | 100.5 | 100.9 | 99.7 |
| 2. | Betaxolol hydrochloride | 100.9 | 100.4 | 100.5 | 100.4 | 100.5 |
| 3. | Tafluprost | 99.2 | 100.1 | 101.5 | 100.5 | 100.3 |

According to Embodiments 246 to 250, the preparation including netarsudil dihydrochloride, betaxolol hydrochloride and tafluprost has good stability after being stored under a storage condition of 5°Cfor 24 months, which meets a requirement for a storage condition of the commercially available eye drops (Betoptic^{®}, Rhopress^{®} andTapros^{®}) before opening.

**Table 399. Contents of various main medicine components in Embodiments 246 to 250 after being stored at 25°Cfor 6 weeks**

| No. | Component | Embodiment 246 | Embodiment 247 | Embodiment 248 | Embodiment 249 | Embodiment 250 |
|---|---|---|---|---|---|---|
| | | Content % | | | | |
| 1. | Netarsudil dihydrochloride | 99.7 | 100.9 | 100.2 | 99.8 | 100.6 |
| 2. | Betaxolol hydrochloride | 100.6 | 99.9 | 100.2 | 100.6 | 99.5 |
| 3. | Tafluprost | 100.4 | 100.0 | 99.3 | 99.9 | 99.4 |

According to Embodiments 246 to 250, the preparation including netarsudil dihydrochloride, betaxolol hydrochloride and tafluprost has good stability after being stored under a storage condition of 25°Cfor 6 weeks, which meets a requirement for a storage condition of the commercially available eye drops (Betoptic^{®}, Rhopress^{®} and Tapros^{®}) after opening.

**Table 400. Contents of various main medicine components in Embodiments 246 to 250 after being stored at 40°Cfor 14 days**

| No. | Component | Embodiment 246 | Embodiment 247 | Embodiment 248 | Embodiment 249 | Embodiment 250 |
|---|---|---|---|---|---|---|
| | | Content % | | | | |
| 1. | Netarsudil dihydrochloride | 100.3 | 100.5 | 100.5 | 99.4 | 100.9 |
| 2. | Betaxolol hydrochloride | 99.2 | 100.8 | 101.7 | 99.6 | 100.7 |
| 3. | Tafluprost | 100.7 | 100.8 | 100.2 | 100.3 | 99.5 |

According to Embodiments 246 to 250, the preparation including netarsudil dihydrochloride, betaxolol hydrochloride and tafluprost has good stability after being stored under a storage condition of 40°Cfor 14 days, which meets a requirement for departing from a storage condition of the commercially available eye drops (Betoptic^{®}, Rhopress^{®} and Tapros^{®}) for a short time.

### Comparative examples 89 to 92: a preparation including netarsudil dimesylate, betaxolol hydrochloride and latanoprost

**Table 401. Preparation of a local ophthalmic medicine composition solution for reducing an intra-ocular pressure through a conventional method as follows:**

| No. | Component | Comparative example 89 | Comparative example 90 | Comparative example 91 | Comparative example 92 |
|---|---|---|---|---|---|
| 1. | Netarsudil dimesylate | 14.2 mg | 14.2 mg | 28.5 mg | 28.5 mg |
| 2. | Betaxolol hydrochloride | 139.8 mg | 279.6 mg | 139.8 mg | 279.6 mg |
| 3. | Latanoprost | 5 mg | 5 mg | 5 mg | 5 mg |
| 4. | Boric acid | 50 mg | 50 mg | 50 mg | 50 mg |
| 5. | Mannitol | 4800 mg | 4800 mg | 4800 mg | 4800 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 402. Contents of various main medicine components in Comparative examples 89 to 92 after being stored at 5°Cfor 7 days**

| No. | Component | Comparative example 89 | Comparative example 90 | Comparative example 91 | Comparative example 92 |
|---|---|---|---|---|---|
| | | Content % | | | |
| 1. | Netarsudil dimesylate | <30 | <30 | <30 | <30 |
| 2. | Betaxolol hydrochloride | 100.9 | 101.8 | 100.4 | 100.6 |
| 3. | Latanoprost | 100.7 | 99.7 | 99.1 | 99.7 |

According to Comparative examples 89 to 92, the preparation including netarsudil dimesylate, betaxolol hydrochloride and latanoprost has incompatibility under the storage condition of 5°C, and netarsudil dimesylate is precipitated.

### Comparative examples 117 to 120: a preparation including netarsudil dimesylate, betaxolol hydrochloride and bimatoprost

**Table 403. Preparation of a local ophthalmic medicine composition solution for reducing an intra-ocular pressure as follows:**

| No. | Component | Comparative example 93 | Comparative example 94 | Comparative example 95 | Comparative example 96 |
|---|---|---|---|---|---|
| 1. | Netarsudil dimesylate | 14.2 mg | 14.2 mg | 28.5 mg | 28.5 mg |
| 2. | Betaxolol hydrochloride | 139.8 mg | 279.6 mg | 139.8 mg | 279.6 mg |
| 3. | Bimatoprost | 30 mg | 30 mg | 30 mg | 30 mg |
| 4. | Boric acid | 50 mg | 50 mg | 50 mg | 50 mg |
| 5. | Mannitol | 4800 mg | 4800 mg | 4800 mg | 4800 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 404. Contents of various main medicine components in Comparative examples 117 to 120 after being stored at 5°Cfor 7 days**

| No. | Component | Comparative example 93 | Comparative example 94 | Comparative example 95 | Comparative example 96 |
|---|---|---|---|---|---|
| | | Content % | | | |
| 1. | Netarsudil dimesylate | <30 | <30 | <30 | <30 |
| 2. | Betaxolol hydrochloride | 100.5 | 100.2 | 100.5 | 100.6 |
| 3. | Bimatoprost | 99.2 | 101.5 | 100.3 | 99.7 |

According to Comparative examples 117 to 120, the preparation including netarsudil dimesylate, betaxolol hydrochloride and bimatoprost has incompatibility under the storage condition of 5°C, and netarsudil dimesylate is precipitated.

### Comparative examples 97 to 100: a preparation including netarsudil dimesylate, betaxolol hydrochloride and travoprost

**Table 405. Preparation of a local ophthalmic medicine composition solution for reducing an intra-ocular pressure as follows:**

| No. | Component | Comparative example 97 | Comparative example 98 | Comparative example 99 | Comparative example 100 |
|---|---|---|---|---|---|
| 1. | Netarsudil dimesylate | 14.2 mg | 14.2 mg | 28.5 mg | 28.5 mg |
| 2. | Betaxolol hydrochloride | 139.8 mg | 279.6 mg | 139.8 mg | 279.6 mg |
| 3. | Travoprost | 4 mg | 4 mg | 4 mg | 4 mg |
| 4. | Boric acid | 50 mg | 50 mg | 50 mg | 50 mg |
| 5. | Mannitol | 4800 mg | 4800 mg | 4800 mg | 4800 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 406. Contents of various main medicine components in Comparative examples 97 to 100 after being stored at 5°Cfor 7 days**

| No. | Component | Comparative example 97 | Comparative example 98 | Comparative example 99 | Comparative example 100 |
|---|---|---|---|---|---|
| | | Content % | | | |
| 1. | Netarsudil dimesylate | <30 | <30 | <30 | <30 |
| 2. | Betaxolol hydrochloride | 100.3 | 100.2 | 100.6 | 100.7 |
| 3. | Travoprost | 99.0 | 99.9 | 99.4 | 99.8 |

According to Comparative examples 97 to 100, the preparation including netarsudil dimesylate, betaxolol hydrochloride and travoprost has incompatibility under the storage condition of 5°C, and netarsudil dimesylate is precipitated.

### Comparative examples 101 to 104: a preparation including netarsudil dimesylate, betaxolol hydrochloride and tafluprost

**Table 407. Preparation of a local ophthalmic medicine composition solution for reducing an intra-ocular pressure as follows:**

| No. | Component | Comparative example 101 | Comparative example 102 | Comparative example 103 | Comparative example 104 |
|---|---|---|---|---|---|
| 1. | Netarsudil dimesylate | 14.2 mg | 14.2 mg | 28.5 mg | 28.5 mg |
| 2. | Betaxolol hydrochloride | 139.8 mg | 279.6 mg | 139.8 mg | 279.6 mg |
| 3. | Tafluprost | 1.5 mg | 1.5 mg | 1.5 mg | 1.5 mg |
| 4. | Boric acid | 50 mg | 50 mg | 50 mg | 50 mg |
| 5. | Mannitol | 4800 mg | 4800 mg | 4800 mg | 4800 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 |

A preparation process may refer to the preparation process in Embodiments 83 to 87.

**Table 408. Contents of various main medicine components in Comparative examples 101 to 104 after being stored at 5°Cfor 7 days**

| No. | Component | Comparative example 101 | Comparative example 102 | Comparative example 103 | Comparative example 104 |
|---|---|---|---|---|---|
| | | Content % | | | |
| 1. | Netarsudil dimesylate | <30 | <30 | <30 | <30 |
| 2. | Betaxolol hydrochloride | 100.5 | 100.1 | 99.3 | 100.8 |
| 3. | Tafluprost | 99.3 | 99.9 | 99.5 | 99.5 |

According to Comparative examples 101 to 104, the preparation including netarsudil dimesylate, betaxolol hydrochloride and tafluprost has incompatibility under the storage condition of 5°C, and netarsudil dimesylate is precipitated.

### Embodiments 251 to 255: a preparation including netarsudil dimesylate, metipranolol mesylate and latanoprost

**Table 409. Preparation of a local ophthalmic medicine composition solution for reducing an intra-ocular pressure through a conventional method as follows:**

| No. | Component | Embodiment 251 | Embodiment 252 | Embodiment 253 | Embodiment 254 | Embodiment 255 |
|---|---|---|---|---|---|---|
| 1. | Netarsudil dimesylate | 14.2 mg | 14.2 mg | 28.5 mg | 28.5 mg | 57.0 mg |
| 2. | Metipranolol mesylate | 131.1 mg | 393.2 mg | 131.1 mg | 393.2 mg | 786.4 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 410. Contents of various main medicine components in Embodiments 251 to 255 after being stored at 5°Cfor 24 months**

| No. | Component | Embodiment 251 | Embodiment 252 | Embodiment 253 | Embodiment 254 | Embodiment 255 |
|---|---|---|---|---|---|---|
| | | Content % | | | | |
| 1. | Netarsudil dimesylate | 100.4 | 99.7 | 100.4 | 100.9 | 99.0 |
| 2. | Metipranolol mesylate | 100.2 | 100.5 | 100.2 | 100.3 | 99.6 |
| 3. | Latanoprost | 100.6 | 99.5 | 100.5 | 100.5 | 100.2 |

**Table 411. Contents of various main medicine components in commercially available eye drops (Optipranolol^{®}, Rhopress^{®} and Xalatan^{®}) after being stored at 5°Cfor 24 months**

| No. | Component | Optipranolol^{®} | Rhopress^{®} | Xalatan^{®} |
|---|---|---|---|---|
| | | Content % | | |
| 1. | Metipranolol hydrochloride | 99.8 | - | - |
| 2. | Netarsudil dimesylate | - | 99.9 | - |
| 3. | Latanoprost | - | - | 99.8 |

According to Embodiments 251 to 255, the preparation including netarsudil dimesylate, metipranolol mesylate and latanoprost has good stability after being stored under a storage condition of 5°Cfor 24 months, which meets a requirement for a storage condition of the commercially available eye drops (Optipranolol^{®}, Rhopress^{®} and Xalatan^{®}) before opening.

**Table 412. Contents of various main medicine components in Embodiments 251 to 255 after being stored at 25°Cfor 6 weeks**

| No. | Component | Embodiment 251 | Embodiment 252 | Embodiment 253 | Embodiment 254 | Embodiment 255 |
|---|---|---|---|---|---|---|
| | | Content % | | | | |
| 1. | Netarsudil dimesylate | 100.6 | 99.5 | 100.9 | 100.1 | 99.5 |
| 2. | Metipranolol mesylate | 100.3 | 101.3 | 101.1 | 101.4 | 100.4 |
| 3. | Latanoprost | 100.1 | 100.8 | 100.7 | 99.6 | 100.2 |

**Table 413. Contents of various main medicine components in commercially available eye drops (Optipranolol^{®}, Rhopress^{®} and Xalatan^{®}) after being stored at 25°Cfor 6 weeks**

| No. | Component | Optipranolol^{®} | Rhopress^{®} | Xalatan^{®} |
|---|---|---|---|---|
| | | Content % | | |
| 1. | Metipranolol hydrochloride | 99.7 | - | - |
| 2. | Netarsudil dimesylate | - | 99.8 | - |
| 3. | Latanoprost | - | - | 99.6 |

According to Embodiments 251 to 255, the preparation including netarsudil dimesylate, metipranolol mesylate and latanoprost has good stability after being stored under a storage condition of 25°Cfor 6 weeks, which meets a requirement for a storage condition of the commercially available eye drops (Optipranolol^{®}, Rhopress^{®} andXalatan^{®}) after opening.

**Table 414. Contents of various main medicine components in Embodiments 251 to 255 after being stored at 40°Cfor 14 days**

| No. | Component | Embodiment 251 | Embodiment 252 | Embodiment 253 | Embodiment 254 | Embodiment 255 |
|---|---|---|---|---|---|---|
| | | Content % | | | | |
| 1. | Netarsudil dimesylate | 99.6 | 99.7 | 99.8 | 99.9 | 99.8 |
| 2. | Metipranolol mesylate | 100.7 | 100.1 | 99.9 | 100.5 | 100.9 |
| 3. | Latanoprost | 99.8 | 99.4 | 99.6 | 100.9 | 99.7 |

**Table 415. Contents of various main medicine components in commercially available eye drops (Optipranolol^{®}, Rhopress^{®} andXalatan^{®}) after being stored at 40°Cfor 14 days**

| No. | Component | Optipranolol^{®} | Rhopress^{®} | Xalatan^{®} |
|---|---|---|---|---|
| | | Content % | | |
| 1. | Metipranolol hydrochloride | 99.6 | - | - |
| 2. | Netarsudil dimesylate | - | 99.5 | - |
| 3. | Latanoprost | - | - | 99.7 |

According to Embodiments 251 to 255, the preparation including netarsudil dimesylate, metipranolol mesylate and latanoprost has good stability after being stored under a storage condition of 40°Cfor 14 days, which meets a requirement for departing from a storage condition of the commercially available eye drops (Optipranolol^{®}, Rhopress^{®} and Xalatan^{®}) for a short time.

### Embodiments 256 to 260: a preparation including netarsudil dihydrochloride, metipranolol hydrochloride and latanoprost

**Table 416. Preparation of a local ophthalmic medicine composition solution for reducing an intra-ocular pressure through a conventional method as follows:**

| No. | Component | Embodiment 256 | Embodiment 257 | Embodiment 258 | Embodiment 259 | Embodiment 260 |
|---|---|---|---|---|---|---|
| 1. | Netarsudil dihydrochloride | 11.6mg | 11.6mg | 23.2mg | 23.2mg | 46.4mg |
| 2. | Metipranolol hydrochloride | 111.8 mg | 335.4 mg | 111.8 mg | 335.4 mg | 670.8 mg |
| 3. | Latanoprost | 5 mg | 5 mg | 5 mg | 5 mg | 5 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 417. Contents of various main medicine components in Embodiments 256 to 260 after being stored at 5°Cfor 24 months**

| No. | Component | Embodiment 256 | Embodiment 257 | Embodiment 258 | Embodiment 259 | Embodiment 260 |
|---|---|---|---|---|---|---|
| | | Content % | | | | |
| 1. | Netarsudil dihydrochloride | 99.6 | 100.2 | 99.5 | 99.8 | 100.9 |
| 2. | Metipranolol hydrochloride | 99.2 | 100.7 | 100.1 | 100.7 | 101.9 |
| 3. | Latanoprost | 100.8 | 100.4 | 99.9 | 100.1 | 100.3 |

According to Embodiments 256 to 260, the preparation including netarsudil dihydrochloride, metipranolol hydrochloride and latanoprost has good stability after being stored under a storage condition of 5°Cfor 24 months, which meets a requirement for a storage condition of the commercially available eye drops (Optipranolol^{®}, Rhopress^{®} and Xalatan^{®}) before opening.

**Table 418. Contents of various main medicine components in Embodiments 256 to 260 after being stored at 25°Cfor 6 weeks**

| No. | Component | Embodiment 256 | Embodiment 257 | Embodiment 258 | Embodiment 259 | Embodiment 260 |
|---|---|---|---|---|---|---|
| | | Content % | | | | |
| 1. | Netarsudil dihydrochloride | 100.3 | 99.1 | 99.7 | 101.1 | 100.9 |
| 2. | Metipranolol hydrochloride | 100.6 | 99.9 | 99.1 | 100.9 | 100.3 |
| 3. | Latanoprost | 100.5 | 101.4 | 100.0 | 100.2 | 100.4 |

According to Embodiments 256 to 260, the preparation including netarsudil dihydrochloride, metipranolol hydrochloride and latanoprost has good stability after being stored under a storage condition of 25°Cfor 6 weeks, which meets a requirement for a storage condition of the commercially available eye drops (Optipranolol^{®} Rhopress^{®} and Xalatan^{®}) before opening.

**Table 419. Contents of various main medicine components in Embodiments 256 to 260 after being stored at 40°Cfor 14 days**

| No. | Component | Embodiment 256 | Embodiment 257 | Embodiment 258 | Embodiment 259 | Embodiment 260 |
|---|---|---|---|---|---|---|
| | | Content % | | | | |
| 1. | Netarsudil dihydrochloride | 101.1 | 100.1 | 99.3 | 100.1 | 100.9 |
| 2. | Metipranolol hydrochloride | 99.3 | 100.6 | 100.3 | 99.8 | 100.3 |
| 3. | Latanoprost | 100.4 | 100.8 | 99.1 | 99.9 | 99.4 |

According to Embodiments 256 to 260, the preparation including netarsudil dihydrochloride, metipranolol hydrochloride and latanoprost has good stability after being stored under a storage condition of 40°Cfor 14 days, which meets a requirement for departing from a storage condition of the commercially available eye drops (Optipranolol^{®}, Rhopress^{®} and Xalatan^{®}) for a short time.

### Embodiments 261 to 265: a preparation including netarsudil dimesylate, metipranolol mesylate and bimatoprost

**Table 420. Preparation of a local ophthalmic medicine composition solution for reducing an intra-ocular pressure through a conventional method as follows:**

| No. | Component | Embodiment 261 | Embodiment 262 | Embodiment 263 | Embodiment 264 | Embodiment 265 |
|---|---|---|---|---|---|---|
| 1. | Netarsudil dimesylate | 14.2 mg | 14.2 mg | 28.5 mg | 28.5 mg | 57.0 mg |
| 2. | Metipranolol mesylate | 131.1 mg | 393.2 mg | 131.1 mg | 393.2 mg | 786.4 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 421. Contents of various main medicine components in Embodiments 261 to 265 after being stored at 5°Cfor 24 months**

| No. | Component | Embodiment 261 | Embodiment 262 | Embodiment 263 | Embodiment 264 | Embodiment 265 |
|---|---|---|---|---|---|---|
| | | Content % | | | | |
| 1. | Netarsudil dimesylate | 100.2 | 99.9 | 99.8 | 100.2 | 99.1 |
| 2. | Metipranolol mesylate | 100.3 | 100.8 | 100.2 | 100.8 | 100.1 |
| 3. | Bimatoprost | 98.6 | 100.2 | 100.6 | 100.3 | 99.8 |

**Table 422. Contents of various main medicine components in commercially available eye drops (Optipranolol^{®}, Rhopress^{®} and Lumigan^{®}) after being stored at 5°Cfor 24 months**

| No. | Component | Optipranolol^{®} | Rhopress^{®} | Lumigan^{®} |
|---|---|---|---|---|
| | | Content % | | |
| 1. | Metipranolol hydrochloride | 99.8 | - | - |
| 2. | Netarsudil dimesylate | - | 99.9 | - |
| 3. | Bimatoprost | - | - | 99.4 |

According to Embodiments 261 to 265, the preparation including netarsudil dimesylate, metipranolol mesylate and bimatoprost has good stability after being stored under a storage condition of 5°Cfor 24 months, which meets a requirement for a storage condition of the commercially available eye drops (Optipranolol^{®}, Rhopress^{®} and Lumigan^{®}) before opening.

**Table 423. Contents of various main medicine components in Embodiments 261 to 265 after being stored at 25°Cfor 6 weeks**

| No. | Component | Embodiment 261 | Embodiment 262 | Embodiment 263 | Embodiment 264 | Embodiment 265 |
|---|---|---|---|---|---|---|
| | | Content % | | | | |
| 1. | Netarsudil dimesylate | 100.0 | 100.1 | 99.2 | 99.5 | 99.8 |
| 2. | Metipranolol mesylate | 99.9 | 100.1 | 99.9 | 99.3 | 100.3 |
| 3. | Bimatoprost | 99.8 | 99.8 | 100.3 | 100.9 | 100.2 |

**Table 424. Contents of various main medicine components in commercially available eye drops (Optipranolol^{®}, Rhopress^{®} and Lumigan^{®}) after being stored at 25°Cfor 6 weeks**

| No. | Component | Optipranolol^{®} | Rhopress^{®} | Lumigan^{®} |
|---|---|---|---|---|
| | | Content % | | |
| 1. | Metipranolol hydrochloride | 99.7 | - | - |
| 2. | Netarsudil dimesylate | - | 99.8 | - |
| 3. | Bimatoprost | - | - | 99.5 |

According to Embodiments 261 to 265, the preparation including netarsudil dimesylate, metipranolol mesylate and bimatoprost has good stability after being stored under a storage condition of 25°Cfor 6 weeks, which meets a requirement for a storage condition of the commercially available eye drops (Optipranolol^{®}, Rhopress^{®} and Lumigan^{®}) after opening.

**Table 425. Contents of various main medicine components in Embodiments 261 to 265 after being stored at 40°Cfor 14 days**

| No. | Component | Embodiment 261 | Embodiment 262 | Embodiment 263 | Embodiment 264 | Embodiment 265 |
|---|---|---|---|---|---|---|
| | | Content % | | | | |
| 1. | Netarsudil dimesylate | 99.7 | 99.9 | 99.1 | 99.2 | 98.8 |
| 2. | Metipranolol mesylate | 99.1 | 100.2 | 99.8 | 100.8 | 99.1 |
| 3. | Bimatoprost | 99.2 | 98.9 | 100.5 | 99.5 | 99.2 |

**Table 426. Contents of various main medicine components in commercially available eye drops (Optipranolol^{®}, Rhopress^{®} and Lumigan^{®}) after being stored at 40°Cfor 14 days**

| No. | Component | Optipranolol^{®} | Rhopress^{®} | Lumigan^{®} |
|---|---|---|---|---|
| | | Content % | | |
| 1. | Metipranolol hydrochloride | 100.1 | - | - |
| 2. | Netarsudil dimesylate | - | 99.5 | - |
| 3. | Bimatoprost | - | - | 99.2 |

According to Embodiments 261 to 265, the preparation including netarsudil dimesylate, metipranolol mesylate and bimatoprost has good stability after being stored under a storage condition of 40°Cfor 14 days, which meets a requirement for departing from a storage condition of the commercially available eye drops (Optipranolol^{®}, Rhopress^{®} and Lumigan^{®}) for a short time.

### Embodiments 266 to 270: a preparation including netarsudil dihydrochloride, metipranolol hydrochloride and bimatoprost

**Table 427. Preparation of a local ophthalmic medicine composition solution for reducing an intra-ocular pressure through a conventional method as follows:**

| No. | Component | Embodiment 266 | Embodiment 267 | Embodiment 268 | Embodiment 269 | Embodiment 270 |
|---|---|---|---|---|---|---|
| 1. | Netarsudil dihydrochloride | 11.6 mg | 11.6 mg | 23.2 mg | 23.2 mg | 46.4 mg |
| 2. | Metipranolol hydrochloride | 111.8 mg | 335.4 mg | 111.8 mg | 335.4 mg | 670.8 mg |
| 3. | Bimatoprost | 30 mg | 30 mg | 30 mg | 30 mg | 30 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 5 mg | 5 mg | 5 mg | 5 mg | 5 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 428. Contents of various main medicine components in Embodiments 266 to 270 after being stored at 5°Cfor 24 months**

| No. | Component | Embodiment 266 | Embodiment 267 | Embodiment 268 | Embodiment 269 | Embodiment 270 |
|---|---|---|---|---|---|---|
| | | Content % | | | | |
| 1. | Netarsudil dihydrochloride | 99.4 | 99.9 | 99.6 | 99.1 | 99.3 |
| 2. | Metipranolol hydrochloride | 101.6 | 100.3 | 100.2 | 100.6 | 100.1 |
| 3. | Bimatoprost | 101.1 | 99.4 | 100.9 | 100.5 | 100.5 |

According to Embodiments 266 to 270, the preparation including netarsudil dihydrochloride, metipranolol hydrochloride and bimatoprost has good stability after being stored under a storage condition of 5°Cfor 24 months, which meets a requirement for a storage condition of the commercially available eye drops (Optipranolol^{®}, Rhopress^{®} and Lumigan^{®}) before opening.

**Table 429. Contents of various main medicine components in Embodiments 266 to 270 after being stored at 25°Cfor 6 weeks**

| No. | Component | Embodiment 266 | Embodiment 267 | Embodiment 268 | Embodiment 269 | Embodiment 270 |
|---|---|---|---|---|---|---|
| | | Content % | | | | |
| 1. | Netarsudil dihydrochloride | 100.1 | 100.3 | 100.7 | 100.3 | 98.7 |
| 2. | Metipranolol hydrochloride | 100.6 | 100.5 | 100.4 | 99.2 | 100.3 |
| 3. | Bimatoprost | 99.3 | 100.1 | 99.7 | 100.9 | 99.7 |

According to Embodiments 266 to 270, the preparation including netarsudil dihydrochloride, metipranolol hydrochloride and bimatoprost has good stability after being stored under a storage condition of 25°Cfor 6 weeks, which meets a requirement for a storage condition of the commercially available eye drops (Optipranolol^{®}, Rhopress^{®} and Lumigan^{®}) after opening.

**Table 430. Contents of various main medicine components in Embodiments 266 to 270 after being stored at 40°Cfor 14 days**

| No. | Component | Embodiment 266 | Embodiment 267 | Embodiment 268 | Embodiment 269 | Embodiment 270 |
|---|---|---|---|---|---|---|
| | | Content % | | | | |
| 1. | Netarsudil dihydrochloride | 101.2 | 100.7 | 100.2 | 100.7 | 101.2 |
| 2. | Metipranolol hydrochloride | 99.9 | 100.6 | 99.3 | 100.1 | 100.3 |
| 3. | Bimatoprost | 100.2 | 100.4 | 100.2 | 100.0 | 100.1 |

According to Embodiments 266 to 270, the preparation including netarsudil dihydrochloride, metipranolol hydrochloride and bimatoprost has good stability after being stored under a storage condition of 40°Cfor 14 days, which meets a requirement for departing from a storage condition of the commercially available eye drops (Optipranolol^{®}, Rhopress^{®} and Lumigan^{®}) for a short time.

### Embodiments 271 to 275: a preparation including netarsudil dimesylate, metipranolol mesylate and travoprost

**Table 431. Preparation of a local ophthalmic medicine composition solution for reducing an intra-ocular pressure through a conventional method as follows:**

| No. | Component | Embodiment 271 | Embodiment 272 | Embodiment 273 | Embodiment 274 | Embodiment 275 |
|---|---|---|---|---|---|---|
| 1. | Netarsudil dimesylate | 14.2 mg | 14.2 mg | 28.5 mg | 28.5 mg | 57.0 mg |
| 2. | Metipranolol mesylate | 131.1 mg | 393.2 mg | 131.1 mg | 393.2 mg | 786.4 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 432. Contents of various main medicine components in Embodiments 271 to 275 after being stored at 5°Cfor 24 months**

| No. | Component | Embodiment 271 | Embodiment 272 | Embodiment 273 | Embodiment 274 | Embodiment 275 |
|---|---|---|---|---|---|---|
| | | Content % | | | | |
| 1. | Netarsudil dimesylate | 99.7 | 99.9 | 100.5 | 100.4 | 100.5 |
| 2. | Metipranolol mesylate | 100.4 | 100.8 | 100.7 | 100.3 | 100.4 |
| 3. | Travoprost | 100.4 | 99.5 | 99.3 | 100.8 | 99.4 |

**Table 433. Contents of various main medicine components in commercially available eye drops (Optipranolol^{®}, Rhopress^{®} and Travatan^{®}) after being stored at 5°Cfor 24 months**

| No. | Component | Optipranolol^{®} | Rhopress^{®} | Travatan^{®} |
|---|---|---|---|---|
| | | Content % | | |
| 1. | Metipranolol hydrochloride | 99.8 | - | - |
| 2. | Netarsudil dimesylate | - | 99.9 | - |
| 3. | Travoprost | - | - | 99.8 |

According to Embodiments 271 to 275, the preparation including netarsudil dimesylate, metipranolol mesylate and travoprost has good stability after being stored under a storage condition of 5°Cfor 24 months, which meets a requirement for a storage condition of the commercially available eye drops (Optipranolol^{®}, Rhopress^{®} and Travatan^{®}) before opening.

**Table 434. Contents of various main medicine components in Embodiments 271 to 275 after being stored at 25°Cfor 6 weeks**

| No. | Component | Embodiment 271 | Embodiment 272 | Embodiment 273 | Embodiment 274 | Embodiment 275 |
|---|---|---|---|---|---|---|
| | | Content % | | | | |
| 1. | Netarsudil dimesylate | 100.5 | 99.4 | 100.5 | 100.4 | 100.3 |
| 2. | Metipranolol mesylate | 101.5 | 99.7 | 99.4 | 100.7 | 100.5 |
| 3. | Travoprost | 100.4 | 98.9 | 100.6 | 100.2 | 100.7 |

**Table 435. Contents of various main medicine components in commercially available eye drops (Optipranolol^{®}, Rhopress^{®} and Travatan^{®}) after being stored at 25°Cfor 6 weeks**

| No. | Component | Optipranolol^{®} | Rhopress^{®} | Travatan^{®} |
|---|---|---|---|---|
| | | Content % | | |
| 1. | Metipranolol hydrochloride | 99.7 | - | - |
| 2. | Netarsudil dimesylate | - | 99.8 | - |
| 3. | Travoprost | - | - | 99.5 |

According to Embodiments 271 to 275, the preparation including netarsudil dimesylate, metipranolol mesylate and travoprost has good stability after being stored under a storage condition of 25°Cfor 6 weeks, which meets a requirement for a storage condition of the commercially available eye drops (Optipranolol^{®}, Rhopress^{®} and Travatan^{®}) after opening.

**Table 436. Contents of various main medicine components in Embodiments 271 to 275 after being stored at 40°Cfor 14 days**

| No. | Component | Embodiment 271 | Embodiment 272 | Embodiment 273 | Embodiment 274 | Embodiment 275 |
|---|---|---|---|---|---|---|
| | | Content % | | | | |
| 1. | Netarsudil dimesylate | 100.7 | 99.3 | 100.4 | 100.5 | 99.5 |
| 2. | Metipranolol mesylate | 99.4 | 100.2 | 99.4 | 98.9 | 99.3 |
| 3. | Travoprost | 99.3 | 100.4 | 100.3 | 100.4 | 99.1 |

**Table 437. Contents of various main medicine components in commercially available eye drops (Optipranolol^{®}, Rhopress^{®} and Travatan^{®}) after being stored at 40°Cfor 14 days**

| No. | Component | Optipranolol^{®} | Rhopress^{®} | Travatan^{®} |
|---|---|---|---|---|
| | | Content % | | |
| 1. | Metipranolol hydrochloride | 99.6 | - | - |
| 2. | Netarsudil dimesylate | - | 99.5 | - |
| 3. | Travoprost | - | - | 99.7 |

According to Embodiments 271 to 275, the preparation including netarsudil dimesylate, metipranolol mesylate and travoprost has good stability after being stored under a storage condition of 40°Cfor 14 days, which meets a requirement for departing from a storage condition of the commercially available eye drops (Optipranolol^{®}, Rhopress^{®} and Travatan^{®}) for a short time.

### Embodiments 276 to 280: a preparation including netarsudil dihydrochloride, metipranolol hydrochloride and travoprost

**Table 438. Preparation of a local ophthalmic medicine composition solution for reducing an intra-ocular pressure through a conventional method as follows:**

| No. | Component | Embodiment 276 | Embodiment 277 | Embodiment 278 | Embodiment 279 | Embodiment 280 |
|---|---|---|---|---|---|---|
| 1. | Netarsudil dihydrochloride | 11.6 mg | 11.6 mg | 23.2 mg | 23.2 mg | 46.4 mg |
| 2. | Metipranolol hydrochloride | 111.8 mg | 335.4 mg | 111.8 mg | 335.4 mg | 670.8 mg |
| 3. | Travoprost | 4 mg | 4 mg | 4 mg | 4 mg | 4 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 15 mg | 15 mg | 15 mg | 15 mg | 15 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 439. Contents of various main medicine components in Embodiments 276 to 280 after being stored at 5°Cfor 24 months**

| No. | Component | Embodiment 276 | Embodiment 277 | Embodiment 278 | Embodiment 279 | Embodiment 280 |
|---|---|---|---|---|---|---|
| | | Content % | | | | |
| 1. | Netarsudil dihydrochloride | 99.4 | 99.2 | 100.1 | 99.6 | 100.1 |
| 2. | Metipranolol hydrochloride | 101.6 | 99.4 | 99.0 | 99.2 | 101.4 |
| 3. | Travoprost | 99.5 | 99.8 | 100.3 | 100.6 | 100.7 |

According to Embodiments 276 to 280, the preparation including netarsudil dihydrochloride, metipranolol hydrochloride and travoprost has good stability after being stored under a storage condition of 5°Cfor 24 months, which meets a requirement for a storage condition of the commercially available eye drops (Optipranolol^{®}, Rhopress^{®} and Travatan^{®}) before opening.

**Table 440. Contents of various main medicine components in Embodiments 276 to 280 after being stored at 25°Cfor 6 weeks**

| No. | Component | Embodiment 276 | Embodiment 277 | Embodiment 278 | Embodiment 279 | Embodiment 280 |
|---|---|---|---|---|---|---|
| | | Content % | | | | |
| 1. | Netarsudil dihydrochloride | 100.2 | 99.5 | 100.1 | 99.6 | 100.6 |
| 2. | Metipranolol hydrochloride | 99.4 | 101.9 | 100.5 | 100.3 | 100.2 |
| 3. | Travoprost | 99.1 | 99.3 | 100.9 | 99.7 | 99.6 |

According to Embodiments 276 to 280, the preparation including netarsudil dihydrochloride, metipranolol hydrochloride and travoprost has good stability after being stored under a storage condition of 25°Cfor 6 weeks, which meets a requirement for a storage condition of the commercially available eye drops (Optipranolol^{®}, Rhopress^{®} and Travatan^{®}) after opening.

**Table 441. Contents of various main medicine components in Embodiments 276 to 280 after being stored at 40°Cfor 14 days**

| No. | Component | Embodiment 276 | Embodiment 277 | Embodiment 278 | Embodiment 279 | Embodiment 280 |
|---|---|---|---|---|---|---|
| | | Content % | | | | |
| 1. | Netarsudil dihydrochloride | 100.6 | 99.5 | 99.9 | 100.6 | 99.4 |
| 2. | Metipranolol hydrochloride | 100.2 | 100.1 | 100.2 | 99.4 | 99.2 |
| 3. | Travoprost | 100.1 | 98.7 | 100.6 | 101.3 | 101.6 |

According to Embodiments 276 to 280, the preparation including netarsudil dihydrochloride, metipranolol hydrochloride and travoprost has good stability after being stored under a storage condition of 40°Cfor 14 days, which meets a requirement for departing from a storage condition of the commercially available eye drops (Optipranolol^{®}, Rhopress^{®} and Travatan^{®}) for a short time.

### Embodiments 281 to 285: a preparation including netarsudil dimesylate, metipranolol mesylate and tafluprost

**Table 442. Preparation of a local ophthalmic medicine composition solution for reducing an intra-ocular pressure through a conventional method as follows:**

| No. | Component | Embodiment 281 | Embodiment 282 | Embodiment 283 | Embodiment 284 | Embodiment 285 |
|---|---|---|---|---|---|---|
| 1. | Netarsudil dimesylate | 14.2 mg | 14.2 mg | 28.5 mg | 28.5 mg | 57.0 mg |
| 2. | Metipranolol mesylate | 131.1 mg | 393.2 mg | 131.1 mg | 393.2 mg | 786.4 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 |

A preparation process may refer to the preparation process in Embodiments 83 to 87.

**Table 443. Contents of various main medicine components in Embodiments 281 to 285 after being stored at 5°Cfor 24 months**

| No. | Component | Embodiment 281 | Embodiment 282 | Embodiment 283 | Embodiment 284 | Embodiment 285 |
|---|---|---|---|---|---|---|
| | | Content % | | | | |
| 1. | Netarsudil dimesylate | 100.1 | 99.4 | 99.9 | 99.1 | 101.3 |
| 2. | Metipranolol mesylate | 100.5 | 99.4 | 100.1 | 99.4 | 99.4 |
| 3. | Tafluprost | 99.2 | 100.9 | 100.3 | 100.6 | 100.2 |

**Table 444. Contents of various main medicine components in commercially available eye drops (Optipranolol^{®}, Rhopress^{®} and Tapros^{®}) after being stored at 5°Cfor 24 months**

| No. | Component | Optipranolol^{®} | Rhopress^{®} | Tapros^{®} |
|---|---|---|---|---|
| | | Content % | | |
| 1. | Metipranolol hydrochloride | 99.8 | - | - |
| 2. | Netarsudil dimesylate | - | 99.9 | - |
| 3. | Tafluprost | - | - | 99.4 |

According to Embodiments 281 to 285, the preparation including netarsudil dimesylate, metipranolol mesylate and tafluprost has good stability after being stored under a storage condition of 5°Cfor 24 months, which meets a requirement for a storage condition of the commercially available eye drops (Optipranolol^{®}, Rhopress^{®} and Tapros^{®}) before opening.

**Table 445. Contents of various main medicine components in Embodiments 281 to 285 after being stored at 25°Cfor 6 weeks**

| No. | Component | Embodiment 281 | Embodiment 282 | Embodiment 283 | Embodiment 284 | Embodiment 285 |
|---|---|---|---|---|---|---|
| | | Content % | | | | |
| 1. | Netarsudil dimesylate | 100.4 | 101.5 | 101.3 | 100.1 | 99.1 |
| 2. | Metipranolol mesylate | 100.6 | 100.5 | 99.3 | 99.5 | 101.4 |
| 3. | Tafluprost | 99.3 | 99.1 | 99.6 | 99.3 | 100.3 |

**Table 446. Contents of various main medicine components in commercially available eye drops (Optipranolol^{®}, Rhopress^{®} and Tapros^{®}) after being stored at 25°Cfor 6 weeks**

| No. | Component | Optipranolol^{®} | Rhopress^{®} | Tapros^{®} |
|---|---|---|---|---|
| | | Content % | | |
| 1. | Metipranolol hydrochloride | 99.7 | - | - |
| 2. | Netarsudil dimesylate | - | 99.8 | - |
| 3. | Tafluprost | - | - | 99.3 |

According to Embodiments 281 to 285, the preparation including netarsudil dimesylate, metipranolol mesylate and tafluprost has good stability after being stored under a storage condition of 25°Cfor 6 weeks, which meets a requirement for a storage condition of the commercially available eye drops (Optipranolol^{®}, Rhopress^{®} and Tapros^{®}) after opening.

**Table 447. Contents of various main medicine components in Embodiments 281 to 285 after being stored at 40°Cfor 14 days**

| No. | Component | Embodiment 281 | Embodiment 282 | Embodiment 283 | Embodiment 284 | Embodiment 285 |
|---|---|---|---|---|---|---|
| | | Content % | | | | |
| 1. | Netarsudil dimesylate | 99.2 | 99.1 | 99.4 | 99.5 | 100.2 |
| 2. | Metipranolol mesylate | 99.4 | 100.2 | 100.5 | 100.1 | 99.0 |
| 3. | Tafluprost | 99.6 | 99.2 | 100.2 | 100.6 | 100.2 |

**Table 448. Contents of various main medicine components in commercially available eye drops (Optipranolol^{®}, Rhopress^{®} and Tapros^{®}) after being stored at 40°Cfor 14 days**

| No. | Component | Optipranolol^{®} | Rhopress^{®} | Tapros^{®} |
|---|---|---|---|---|
| | | Content % | | |
| 1. | Metipranolol hydrochloride | 99.6 | - | - |
| 2. | Netarsudil dimesylate | - | 99.5 | - |
| 3. | Tafluprost | - | - | 99.8 |

According to Embodiments 281 to 285, the preparation including netarsudil dimesylate, metipranolol mesylate and tafluprost has good stability after being stored under a storage condition of 40°Cfor 14 days, which meets a requirement for departing from a storage condition of the commercially available eye drops (Optipranolol^{®}, Rhopress^{®} and Tapros^{®}) for a short time.

### Embodiments 286 to 290: a preparation including netarsudil dihydrochloride, metipranolol hydrochloride and tafluprost

**Table 449. Preparation of a local ophthalmic medicine composition solution for reducing an intra-ocular pressure through a conventional method as follows:**

| No. | Component | Embodiment 286 | Embodiment 287 | Embodiment 288 | Embodiment 289 | Embodiment 290 |
|---|---|---|---|---|---|---|
| 1. | Netarsudil dihydrochloride | 11.6 mg | 11.6 mg | 23.2 mg | 23.2 mg | 46.4 mg |
| 2. | Metipranolol hydrochloride | 111.8 mg | 335.4 mg | 111.8 mg | 335.4 mg | 670.8 mg |
| 3. | Tafluprost | 1.5 mg | 1.5 mg | 1.5 mg | 1.5 mg | 1.5 mg |
| 4. | Boric acid | 50 mg | 50 mg | 50 mg | 50 mg | 50 mg |
| 5. | Mannitol | 4500 mg | 4500 mg | 4500 mg | 4500 mg | 4500 mg |
| 6. | Benzalkonium chloride | 20 mg | 20 mg | 20 mg | 20 mg | 20 mg |
| 7. | Sodium hydroxide | q.s. | q.s. | q.s. | q.s. | q.s. |
| 8. | Water for injection added by | 100 mL | 100 mL | 100 mL | 100 mL | 100 mL |

A preparation process may refer to the preparation process in Embodiments 83 to 87.

**Table 450. Contents of various main medicine components in Embodiments 286 to 290 after being stored at 5°Cfor 24 months**

| No. | Component | Embodiment 286 | Embodiment 287 | Embodiment 288 | Embodiment 289 | Embodiment 290 |
|---|---|---|---|---|---|---|
| | | Content % | | | | |
| 1. | Netarsudil dihydrochloride | 99.4 | 100.1 | 100.1 | 100.3 | 99.2 |
| 2. | Metipranolol hydrochloride | 100.6 | 100.2 | 100.6 | 100.3 | 100.1 |
| 3. | Tafluprost | 99.7 | 99.1 | 100.5 | 100.3 | 100.2 |

According to Embodiments 286 to 290, the preparation including netarsudil dihydrochloride, metipranolol hydrochloride and tafluprost has good stability after being stored under a storage condition of 5°Cfor 24 months, which meets a requirement for a storage condition of the commercially available eye drops (Optipranolol^{®}, Rhopress^{®} and Tapros^{®}) before opening.

**Table 451. Contents of various main medicine components in Embodiments 286 to 290 after being stored at 25°Cfor 6 weeks**

| No. | Component | Embodiment 286 | Embodiment 287 | Embodiment 288 | Embodiment 289 | Embodiment 290 |
|---|---|---|---|---|---|---|
| | | Content % | | | | |
| 1. | Netarsudil dihydrochloride | 99.2 | 100.1 | 99.2 | 99.2 | 100.5 |
| 2. | Metipranolol hydrochloride | 100.2 | 99.4 | 100.3 | 100.2 | 100.5 |
| 3. | Tafluprost | 100.5 | 100.1 | 99.1 | 100.9 | 99.6 |

According to Embodiments 286 to 290, the preparation including netarsudil dihydrochloride, metipranolol hydrochloride and tafluprost has good stability after being stored under a storage condition of 25°Cfor 6 weeks, which meets a requirement for a storage condition of the commercially available eye drops (Optipranolol^{®}, Rhopress^{®} and Tapros^{®}) after opening.

**Table 452. Contents of various main medicine components in Embodiments 286 to 290 after being stored at 40°Cfor 14 days**

| No. | Component | Embodiment 286 | Embodiment 287 | Embodiment 288 | Embodiment 289 | Embodiment 290 |
|---|---|---|---|---|---|---|
| | | Content % | | | | |
| 1. | Netarsudil dihydrochloride | 100.1 | 100.3 | 99.5 | 99.1 | 100.3 |
| 2. | Metipranolol hydrochloride | 100.2 | 100.1 | 100.7 | 99.7 | 100.5 |
| 3. | Tafluprost | 100.3 | 100.4 | 100.4 | 100.5 | 99.6 |

According to Embodiments 286 to 290, the preparation including netarsudil dihydrochloride, metipranolol hydrochloride and tafluprost has good stability after being stored under a storage condition of 40°Cfor 14 days, which meets a requirement for departing from a storage condition of the commercially available eye drops (Optipranolol^{®}, Rhopress^{®} and Tapros^{®}) for a short time.

### Comparative examples 105 to 108: a preparation including netarsudil dimesylate, metipranolol hydrochloride and latanoprost

**Table 453. Preparation of a local ophthalmic medicine composition solution for reducing an intra-ocular pressure through a conventional method as follows:**

| No. | Component | Comparative example 105 | Comparative example 106 | Comparative example 107 | Comparative example 108 |
|---|---|---|---|---|---|
| 1. | Netarsudil dimesylate | 14.2 mg | 14.2 mg | 28.5 mg | 28.5 mg |
| 2. | Metipranolol hydrochloride | 111.8 mg | 335.4 mg | 111.8 mg | 335.4 mg |
| 3. | Latanoprost | 5 mg | 5 mg | 5 mg | 5 mg |
| 4. | Boric acid | 50 mg | 50 mg | 50 mg | 50 mg |
| 5. | Mannitol | 4500 mg | 4500 mg | 4500 mg | 4500 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 mL |

A preparation process may refer to the preparation process in Embodiments 83 to 87.

**Table 454. Contents of various main medicine components in Comparative examples 105 to 108 after being stored at 5°Cfor 7 days**

| No. | Component | Comparative example 105 | Comparative example 106 | Comparative example 107 | Comparative example 108 |
|---|---|---|---|---|---|
| | | Content % | | | |
| 1. | Netarsudil dimesylate | <30 | <30 | <30 | <30 |
| 2. | Metipranolol hydrochloride | 100.3 | 101.1 | 100.2 | 99.6 |
| 3. | Latanoprost | 100.9 | 99.6 | 99.2 | 99.3 |

According to Comparative examples 105 to 108: the preparation including netarsudil dimesylate, metipranolol hydrochloride and latanoprost has incompatibility under the storage condition of 5°C, and netarsudil dimesylate is precipitated.

### Comparative examples 109 to 112: a preparation including netarsudil dimesylate, metipranolol hydrochloride and bimatoprost

**Table 455. Preparation of a local ophthalmic medicine composition solution for reducing an intra-ocular pressure as follows:**

| No. | Component | Comparative example 109 | Comparative example 110 | Comparative example 111 | Comparative example 112 |
|---|---|---|---|---|---|
| 1. | Netarsudil dimesylate | 14.2 mg | 14.2 mg | 28.5 mg | 28.5 mg |
| 2. | Metipranolol hydrochloride | 111.8 mg | 335.4 mg | 111.8 mg | 335.4 mg |
| 3. | Bimatoprost | 30 mg | 30 mg | 30 mg | 30 mg |
| 4. | Boric acid | 50 mg | 50 mg | 50 mg | 50 mg |
| 5. | Mannitol | 4500 mg | 4500 mg | 4500 mg | 4500 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection | 100 mL | 100 mL | 100 mL | 100 mL |
| | added by | | | | |

A preparation process may refer to the preparation process in Embodiments 83 to 87.

**Table 456. Contents of various main medicine components in Comparative examples 109 to 112 after being stored at 5°Cfor 7 days**

| No. | Component | Comparative example 109 | Comparative example 110 | Comparative example 111 | Comparative example 112 |
|---|---|---|---|---|---|
| | | Content % | | | |
| 1. | Netarsudil dimesylate | <30 | <30 | <30 | <30 |
| 2. | Metipranolol hydrochloride | 99.5 | 100.3 | 101.5 | 100.2 |
| 3. | Bimatoprost | 99.6 | 100.5 | 100.1 | 99.5 |

According to Comparative examples 109 to 112: the preparation including netarsudil dimesylate, metipranolol hydrochloride and bimatoprost has incompatibility under the storage condition of 5°C, and netarsudil dimesylate is precipitated.

### Comparative examples 113 to 116: a preparation including netarsudil dimesylate, metipranolol hydrochloride and travoprost

**Table 457. Preparation of a local ophthalmic medicine composition solution for reducing an intra-ocular pressure as follows:**

| No. | Component | Comparative example 113 | Comparative example 114 | Comparative example 115 | Comparative example 116 |
|---|---|---|---|---|---|
| 1. | Netarsudil dimesylate | 14.2 mg | 14.2 mg | 28.5 mg | 28.5 mg |
| 2. | Metipranolol hydrochloride | 111.8 mg | 335.4 mg | 111.8 mg | 335.4 mg |
| 3. | Travoprost | 4 mg | 4 mg | 4 mg | 4 mg |
| 4. | Boric acid | 50 mg | 50 mg | 50 mg | 50 mg |
| 5. | Mannitol | 4500 mg | 4500 mg | 4500 mg | 4500 mg |
| 6. | Benzalkonium chloride | 5 mg | 5 mg | 5 mg | 5 mg |
| 7. | Sodium hydroxide | q.s. | q.s. | q.s. | q.s. |
| 8. | Water for injection added by | 100 mL | 100 mL | 100 mL | 100 mL |

A preparation process may refer to the preparation process in Embodiments 83 to 87.

**Table 458. Contents of various main medicine components in Comparative examples 113 to 116 after being stored at 5°Cfor 7 days**

| No. | Component | Comparative example 113 | Comparative example 114 | Comparative example 115 | Comparative example 116 |
|---|---|---|---|---|---|
| | | Content % | | | |
| 1. | Netarsudil dimesylate | <30 | <30 | <30 | <30 |
| 2. | Metipranolol hydrochloride | 100.1 | 101.2 | 100.5 | 100.2 |
| 3. | Travoprost | 99.0 | 99.3 | 99.1 | 100.8 |

According to Comparative examples 113 to 116, the preparation including netarsudil dimesylate, metipranolol hydrochloride and travoprost has incompatibility under the storage condition of 5°C, and netarsudil dimesylate is precipitated.

### Comparative examples 117 to 120: a preparation including netarsudil dimesylate, metipranolol hydrochloride and tafluprost

**Table 459. Preparation of a local ophthalmic medicine composition solution for reducing an intra-ocular pressure as follows:**

| No. | Component | Comparative example 117 | Comparative example 118 | Comparative example 119 | Comparative example 120 |
|---|---|---|---|---|---|
| 1. | Netarsudil dimesylate | 14.2 mg | 14.2 mg | 28.5 mg | 28.5 mg |
| 2. | Metipranolol hydrochloride | 111.8 mg | 335.4 mg | 111.8 mg | 335.4 mg |
| 3. | Tafluprost | 1.5 mg | 1.5 mg | 1.5 mg | 1.5 mg |
| 4. | Boric acid | 50 mg | 50 mg | 50 mg | 50 mg |
| 5. | Mannitol | 4500 mg | 4500 mg | 4500 mg | 4500 mg |
| 6. | Benzalkonium chloride | 20 mg | 20 mg | 20 mg | 20 mg |
| 7. | Sodium hydroxide | q.s. | q.s. | q.s. | q.s. |
| 8. | Water for injection added by | 100 mL | 100 mL | 100 mL | 100 mL |

A preparation process may refer to the preparation process in Embodiments 83 to 87.

**Table 460. Contents of various main medicine components in Comparative examples 117 to 120 after being stored at 5°Cfor 7 days**

| No. | Component | Comparative example 117 | Comparative example 118 | Comparative example 119 | Comparative example 120 |
|---|---|---|---|---|---|
| | | Content % | | | |
| 1. | Netarsudil dimesylate | <30 | <30 | <30 | <30 |
| 2. | Metipranolol hydrochloride | 100.3 | 100.3 | 99.7 | 100.2 |
| 3. | Tafluprost | 99.1 | 99.4 | 99.1 | 99.3 |

According to Comparative examples 117 to 120, the preparation including netarsudil dimesylate, metipranolol hydrochloride and tafluprost has incompatibility under the storage condition of 5°C, and netarsudil dimesylate is precipitated.

## Claims

1. An ophthalmic dual compound medicine composition, **characterized in that** the medicine composition comprises:
(1) a β-adrenergic receptor blocker, selected from **(S)-1-(tert-butylamino)-3-[(4-morpholin-1,2,5-thiadiazol-3-yl)oxy]-2-propanol** (timolol) or pharmaceutically acceptable salts thereof, 5-[3-[(1,1-dimethylethyl)amino]-2-hydroxypropoxy]-3,4-dihydro-2(1H)-quinolone (carteolol) or pharmaceutically acceptable salts thereof, (RS)-1-[4-[2-(cyclopropylmethoxy)ethyl]phenoxy]3-[(1-methyl ethyl)-ammonia]-2-propanol (betaxolol) or pharmaceutically acceptable salts thereof, or 4-[2-hydroxy-3-[(1-methyl ethyl)amino]propoxy]-2,3,6-trimethyl-phenoll-acetate (metipranolol) or pharmaceutically acceptable salts thereof;
(2) (S)-2,4-dimethylbenzoic acid4-(3-amino-1-(isoquinoline-6-ylamino)-1-oxoprop-2-yl)benzyl ester (netarsudil) or pharmaceutically acceptable salts thereof;
(3) a preservative, selected from benzalkonium chloride, thimerosal, chlorbutanol, methyl p-hydroxybenzoate, propyl p-hydroxybenzoate, phenethyl alcohol, edetate disodium, boric acid, sorbic acid or any combination thereof;
(4) a buffering agent, selected from boric acid or salts thereof, sodium dihydrogen phosphate dihydrate, sodium dihydrogen phosphate monohydrate, sodium phosphate anhydrate, citric acid or salts thereof, gluconic acid or salts thereof, acetic acid or salts thereof, phosphoric acid or salts thereof, various amino acids such as glutamic acid and ε-aminocaproic acid, a tris(hydroxymethyl) aminomethane buffer, or any combination thereof;
(5) a tonicity agent, selected from glycerol, sorbitol, mannitol, propylene glycol, erythritol, arabitol, xylitol, ribitol, galactitol, polyethylene glycol, lactitol and other sugar alcohol, sodium chloride, potassium chloride and calcium chloride, or any combination thereof;
(6) a pH regulator, selected from sodium hydroxide, potassium hydroxide, sodium carbonate, sodium hydrogen carbonate, hydrochloric acid, citric acid or salts thereof, phosphoric acid or salts thereof, acetic acid or salts thereof, and tartaric acid and/or hydrochloric acid or salts thereof; and
(7) water for injection.

2. An ophthalmic triple compound medicine composition, **characterized in that** the medicine composition comprises:
(1) a β-adrenergic receptor blocker, selected from (S)-1-(tert-butylamino)-3-[(4-morpholin-1,2,5-thiadiazol-3-yl)oxy]-2-propanol (timolol) or pharmaceutically acceptable salts thereof, 5-[3-[(1,1-dimethylethyl)amino]-2-hydroxypropoxy]-3,4-dihydro-2(1H)-quinolone (carteolol) or pharmaceutically acceptable salts thereof, (RS)-1-[4-[2-(cyclopropylmethoxy)ethyl]phenoxy]3-[(1-methyl ethyl)-ammonia]-2-propanol (betaxolol) or pharmaceutically acceptable salts thereof, or 4-[2-hydroxy-3-[(1-methyl ethyl)amino]propoxy]-2,3,6-trimethyl-phenoll-acetate (metipranolol) or pharmaceutically acceptable salts thereof;
(2) (S)-2,4-dimethylbenzoic acid4-(3-amino-1-(isoquinoline-6-ylamino)-1-oxoprop-2-yl)benzyl ester (netarsudil) or pharmaceutically acceptable salts thereof;
(3) a prostaglandin analog, selected from latanoprost, bimatoprost, travoprost, tafluprost, AR-102, cloprostenol isopropyl ester, 13,14-dihydrocloprostenol isopropyl ester, latanoprostene bunod, unoprostone, PGF1α isopropyl ester, PGF2α isopropyl ester, PGF3α isopropyl ester or fluprostenol isopropyl ester;
(4) a preservative, selected from benzalkonium chloride, thimerosal, chlorbutanol, methyl p-hydroxybenzoate, propyl p-hydroxybenzoate, phenethyl alcohol, edetate disodium, boric acid, sorbic acid or any combination thereof;
(5) a buffering agent, selected from boric acid or salts thereof, sodium dihydrogen phosphate dihydrate, sodium dihydrogen phosphate monohydrate, sodium phosphate anhydrate, citric acid or salts thereof, gluconic acid or salts thereof, acetic acid or salts thereof, phosphoric acid or salts thereof, various amino acids such as glutamic acid and ε-aminocaproic acid, tris(hydroxymethyl) aminomethane buffer, or any combination thereof;
(6) a tonicity agent, selected from glycerol, sorbitol, mannitol, propylene glycol, erythritol, arabitol, xylitol, ribitol, galactitol, polyethylene glycol, lactitol and other sugar alcohol, sodium chloride, potassium chloride and calcium chloride, or any combination thereof;
(7) a pH regulator, selected from sodium hydroxide, potassium hydroxide, sodium carbonate, sodium hydrogen carbonate, sodium hydrogen carbonate, hydrochloric acid, citric acid or salts thereof, phosphoric acid or salts thereof, acetic acid or salts thereof, and tartaric acid and/or hydrochloric acid or salts thereof; and
(8) water for injection.

3. The compound medicine composition according to claim 1 or 2, **characterized in that** the timolol is in a form of free alkali or any pharmaceutically acceptable salt (except maleate) thereof in a case that the netarsudil is dimesylate, preferably, timolol mesylate, timolol sulfate, timolol hydrobromide, timolol phosphate, timolol nitrate, timolol citrate, timolol tartrate, timolol salicylate, timolol malate, timolol lactate, timolol phenylacetate, timolol succinate, timolol hydriodate, timolol formate, timolol acetate, timolol benzoate, timolol esilate, timolol oxalate or timolol propionate; the carteolol is in a form of free alkali or any pharmaceutically acceptable salt (except hydrochloride) thereof, preferably, carteolol mesylate, carteolol hydrobromide, carteolol sulfate, carteolol esilate, carteolol nitrate, carteolol citrate, carteolol tartrate, carteolol salicylate, carteolol malate, carteolol lactate, carteolol phenylacetate, carteolol succinate, carteolol hydriodate, carteolol formate, carteolol acetate, carteolol benzoate, carteolol esilate, carteolol oxalate or carteolol propionate; the betaxolol is in a form of free alkali or any pharmaceutically acceptable salt (except hydrochloride) thereof, preferably, betaxolol mesylate, betaxolol hydrobromide, betaxolol sulfate, betaxolol esilate, betaxolol nitrate, betaxolol citrate, betaxolol tartrate, betaxolol salicylate, betaxolol malate, betaxolol lactate, betaxolol phenylacetate, betaxolol succinate, betaxolol hydriodate, betaxolol formate, betaxolol acetate, betaxolol benzoate, betaxolol esilate, betaxolol oxalate or betaxolol propionate; and the metipranolol is in a form of free alkali or any pharmaceutically acceptable salt (except hydrochloride) thereof, preferably, metipranolol mesylate, metipranolol hydrobromide, metipranolol sulfate, metipranolol esilate, metipranolol nitrate, metipranolol citrate, metipranolol tartrate, metipranolol salicylate, metipranolol malate, metipranolol lactate, metipranolol phenylacetate, metipranolol succinate, metipranolol hydriodate, metipranolol formate, metipranolol acetate, metipranolol benzoate, metipranolol esilate, metipranolol oxalate or metipranolol propionate.

4. The compound medicine composition according to claim 1 or 2, **characterized in that** the netarsudil is in a form of free alkali or any pharmaceutically acceptable salt (except mesylate) thereof in a case that the β-adrenergic receptor blocker is timolol maleate, or carteolol hydrochloride, or betaxolol hydrochloride, or metipranolol hydrochloride, preferably, netarsudil maleate, netarsudil sulfate, netarsudil dihydrobromide, netarsudil dihydrochloride, netarsudil diformate, netarsudil dinitrate, netarsudil diacetate, netarsudil dibenzoate, netarsudil diphenylacetate, netarsudil succinate, netarsudil oxalate, netarsudil dihydriodate, or netarsudil dipropionate.

5. The compound medicine composition according to any one of claim 1, 3 or 4, **characterized in that** the medicine composition comprises:
(1) 0.02%w/v to 4.0%w/v β-adrenergic receptor blocker, preferably, timolol free alkali or salts thereof, carteolol free alkali or salts thereof, betaxolol free alkali or salts thereof, or metipranolol free alkali or salts thereof;
(2) 0.005 %w/v to 0.1%w/v netarsudil free alkali or salts thereof;
(3) 0.01%w/v to 10.0%w/v tonicity agent;
(4) 0.01 %w/v to 1.0 %w/v buffering agent; and
(5) 0.001%w/v to 0.02 %w/v preservative;
(6) wherein pH is in a range from 4.5 to 5.4; and
(7) an osmotic pressure is in a range from 280 mOsmol/kg to 320 mOsmol/kg.

6. The compound medicine composition according to any one of claims 2 to 4, **characterized in that** the medicine composition comprises:
(1) 0.02%w/v to 4.0%w/v β-adrenergic receptor blocker, preferably, timolol free alkali or salts thereof, carteolol free alkali or salts thereof, betaxolol free alkali or salts thereof, or metipranolol free alkali or salts thereof;
(2) 0.005 %w/v to 0.1%w/v netarsudil free alkali or salts thereof;
(3) 0.0005 %w/v to 0.05 %w/v prostaglandin analog;
(4) 0.01%w/v to 10.0%w/v tonicity agent;
(5) 0.01%w/v to 1.0%w/v buffering agent;
(6) 0.001 %w/v to 0.02 %w/v preservative;
(7) wherein pH is in a range from 4.5 to 5.4; and
(8) an osmotic pressure is in a range from 280 mOsmol/kg to 320 mOsmol/kg.

7. The compound medicine composition according to any one of claims 1 to 6, **characterized in that** the medicine composition does not comprise precipitate after being stored at 5°Cfor 24 months, and a content of each main medicine component has no significant change compared with zero day; the medicine composition does not comprise precipitate after being stored at 25°Cfor 6 weeks, and a content of each main medicine component has no significant change compared with zero day; and the medicine composition does not comprise precipitate after being stored at 40°Cfor 14 days, and a content of each main medicine component has no significant change compared with zero day.

8. Use of the compound medicine composition according to any one of claims 1 to 7 in preparation of medicine for preventing or treating an eye disease.

9. The use according to claim 8, **characterized in that** the eye disease is glaucoma or symptoms related thereto.

10. Use of the compound medicine composition according to any one of claims 1 to 7 in preparation of medicine for reducing an intra-ocular pressure.
